(19)

Europäisches
Patentamt
European
Patent Office
Office européen
des brevets

(11) **EP 2 181 966 A1**

(12) **EUROPEAN PATENT APPLICATION**
published in accordance with Art. 153(4) EPC

(43) Date of publication:
**05.05.2010 Bulletin 2010/18**

(51) Int Cl.:
**C02F 3/34** *(2006.01)*     **C02F 3/12** *(2006.01)*
**G01N 33/18** *(2006.01)*

(21) Application number: **08765759.9**

(22) Date of filing: **19.06.2008**

(86) International application number:
**PCT/JP2008/061264**

(87) International publication number:
**WO 2008/156151 (24.12.2008 Gazette 2008/52)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **19.06.2007   JP 2007161550
05.11.2007   JP 2007287694
05.11.2007   JP 2007287698**

(71) Applicant: **KOBELCO ECO-SOLUTIONS CO., LTD.
Kobe-shi
Hyogo 651-0072 (JP)**

(72) Inventors:
  • **YAMASHITA, Tetsuo
    Kobe-shi
    Hyogo 651-2241 (JP)**
  • **TAKEDA, Naohiro
    Kobe-shi
    Hyogo 651-2241 (JP)**

  • **MINAKAWA, Mie
    Kobe-shi
    Hyogo 651-2241 (JP)**
  • **TAKEZAKI, Jun
    Kobe-shi
    Hyogo 651-2241 (JP)**
  • **FUJITA, Masafumi
    Nakakoma-gun
    Yamanashi 409-3862 (JP)**
  • **TSUJI, Koji
    Kofu-shi
    Yamanashi 400-0017 (JP)**
  • **AKASHI, Akira
    Kobe-shi
    Hyogo 651-2241 (JP)**

(74) Representative: **Wablat, Wolfgang
    Potsdamer Chaussee 48
    14129 Berlin (DE)**

(54) **SIMULATION METHOD, SIMULATION APPARATUS, BIOLOGICAL TREATMENT METHOD AND BIOLOGICAL TREATMENT APPARATUS**

(57)    It is intended to provide a simulation method, whereby the calibration operation load can be reduced while minimizing a lowering in prediction accuracy, and a simulation apparatus. It is also intended to provide a biological treatment method, whereby the required operation load can be reduced, and a biological treatment apparatus. These problems can be solved by employing as parameters the maximum reaction speed in the reaction of decomposing a material to be treated with a bacterium and the amount of the above-described material to be treated that is loaded per bacterial cell in a unit time during the biological treatment process or the amount of the above-described material that has been treated per bacterial cell in a unit time in a state where these parameters are in a definite functional relation.

Fig. 1

Discharge water → 1 → Nitrification tank (2) → 3 → Denitrification tank (4) → 5 → Reaeration tank (6) → 7 → Sedimentation tank (8) → 9 → Separated liquid

Return sluge

Excess sludge

**Description**

TECHNICAL FIELD

The present invention relates to a simulation method, a simulation apparatus, a biological treatment method, and a biological treatment apparatus.

BACKGROUND ART

Presently, efficiency improvement of treatment, advancement of treatment capacity, power saving of energy used for water treatment, cost reduction of water treatment, and the like, are in progress in water treatment such as sewage treatment and industrial wastewater treatment.

For example, an approach for predicting the behavior of process under various conditions on the basis of experiences and setting the operating conditions of a water treatment plant has disadvantages of inefficiency in water treatment, waste of energy, and an increase in cost since it is difficult to quantitatively predict the behavior of process under various conditions. Hence, improvement of this disadvantage is demanded.

Therefore, simulations for calculating the reactions of growth, extinction, and the like, of bacterial groups have been introduced in place of the prediction of the behavior of process under various conditions on the basis of experiences and more quantitative predictions have been attempted (See, for example, Patent Documents 1 to 4).

Among them, Patent Document 2 discloses a sewage treatment process simulator system of modeling units making up of sewage treatment process as parts.

This system has IAWQ (present "IWA") activated sludge model No. 2 that is generally called "ASM2" or the like and includes a simulator that calculates model component output values for every part on the basis of model component input values by the IAWQ (present "IWA") activated sludge model No. 2 and a measurement means for online measuring water quality of influent water that flows into a sewage treatment process.

In addition, this system has a conversion means that uses a correlation equation including measurements and model component input values and provides for a computing means for converting measurements from the measurement means into model component input values using the correlation equation of the conversion means.

Additionally, the invention described in Patent Document 2 calibrates reaction rate constants on the basis of water quality of influent water and treated water quality.

However, the water quality of treated water in biological treatment is influenced by various factors, and even if calibration based on treated water quality is executed to once obtain simulation results close to actual treated water quality, the deviation between the actual treated water quality and the simulation results is enlarged with time passage. Thus, the prediction accuracy of treated water quality is decreased unless the calibration is implemented frequently.

In other words, in conventional simulation methods and simulation apparatus, there is a problem that it is difficult to reduce calibration operation load while suppressing a lowering in prediction accuracy.

Moreover, in the biological treatment method and the biological treatment apparatus for carrying out the biological treatment process while predicting treated water quality by such a simulation, the prevention of the lowering in prediction accuracy is difficult, and therefore it is difficult to reduce load such as an operation of ascertaining actual treated water quality.

Patent Document 1: Japanese Patent Laid-Open No. 8-323393
Patent Document 2: Japanese Patent Laid-Open No. 2000-107796
Patent Document 3: Japanese Examined Patent Application Publication No. 7-106357
Patent Document 4: Japanese Patent Laid-Open No. 9-47785

DISCLOSURE OF THE INVENTION

PROBLEMS TO BE SOLVED BY THE INVENTION

The present invention is directed to providing a simulation method and a simulation apparatus capable of reducing the load of a calibration operation while maintaining high prediction accuracy.

In addition, the invention is directed to providing a biological treatment method and a biological treatment apparatus capable of reducing the load of the required operation.

MEANS FOR SOLVING THE PROBLEMS

The present inventors have diligently studied to solve the above-described problems, and as a result, found that the maximum reaction rate of a substance to be treated with bacteria that has been conventionally regarded as a constant varies depending on the amount of the above-described substance to be treated that is loaded per unit number of bacteria per unit time and the amount of the above-described substance that as been treated per unit number of bacteria per unit time in a biological treatment process.
Additionally, the inventors have found that use of the value of the maximum reaction rate as a parameter in a state where the value has functional relations with the above-described amounts is allowed to obtain simulation results which are more excellent in prediction accuracy than conventional results, to complete the present invention.

That is to say, the simulation method according to the present invention is a simulation method of using the value of the maximum reaction rate of a substance to be treated with bacteria as a parameter in order to predict the quality of treated water after a biological treatment process of biologically treating water to be treated containing a substance to be treated with bacteria that decompose the substance to be treated, wherein the value of the maximum reaction rate is used as a parameter in a state where the value of the maximum reaction rate and the amount of the substance to be treated that is loaded per bacterial cell per unit time or the amount of the substance to be treated that has been treated per bacterial cell per unit time in the biological treatment process are in a functional relation, and the above-described function is an increasing function of V with increasing L, wherein V represents the value of the maximum reaction rate and L represents the amount of the substance to be treated that is loaded per bacterial cell per unit time or the amount of the substance to be treated that has been treated per bacterial cell per unit time in the biological treatment process.

In addition, in this simulation method, it is preferable that the value of the maximum reaction rate is used as a parameter in a state where the functional relation of equation (1):

$$[\text{Eq. 1}]$$

$$V = f\ (L)\quad \cdots\ (1)$$

is satisfied, wherein V (fg·copy$^{-1}$·h$^{-1}$) represents the value of the maximum reaction rate and L (fg·copy$^{-1}$·day$^{-1}$) represents the amount of the substance to be treated that is loaded per bacterial cell per unit time or the amount of the substance to be treated that has been treated per bacterial cell per unit time in the biological treatment process, and the function $f(x)$ of a variable x (wherein x > 0) is a function between a function $g(x)$ that increases y1 in equation (2):

$$[\text{Eq. 2}]$$

$$y_1 = g\ (x)\quad \cdots\ (2)$$

with increasing x and a function $h(x)$ that increases $y_2$ in equation (3):

$$[\text{Eq. 3}]$$

$$y_2 = h\ (x)\quad \cdots\ (3)$$

with increasing x and satisfies $y_2 > y_1$.

Moreover, the simulation apparatus according to the present invention is a simulation apparatus of using the value of the maximum reaction rate of a substance to be treated with bacteria as a parameter and executing a simulation in order to predict the quality of treated water after a biological treatment process of biologically treating water to be treated containing a substance to be treated with bacteria that decompose the substance to be treated, wherein the value of the maximum reaction rate is used as a parameter in a state where the value of the maximum reaction rate and the amount of the substance to be treated that is loaded per bacterial cell per unit time or the amount of the substance to be treated that has been treated per bacterial cell per unit time in the biological treatment process are in a functional relation, and the above-described function is an increasing function of V with increasing L, wherein V represents the value of the maximum reaction rate and L represents the amount of the substance to be treated that is loaded per bacterial cell per unit time or the amount of the substance to be treated that has been treated per bacterial cell per unit time in the biological treatment process.

In addition, in this simulation apparatus, it is preferable that the value of the maximum reaction rate is used as a parameter in a state where the functional relation of equation (1):

$$[\text{Eq. 4}]$$

$$V = f\ (L)\quad \cdot\cdot\cdot\ (1)$$

is satisfied, wherein V ($fg\cdot copy^{-1}\cdot h^{-1}$) represents the value of the maximum reaction rate and L ($fg\cdot copy^{-1}\cdot day^{-1}$) represents the amount of the substance to be treated that is loaded per bacterial cell per unit time or the amount of the substance to be treated that has been treated per bacterial cell per unit time in the biological treatment process, and the function f(x) of a variable x (wherein x>0) is a function between a function g(x) that increases $y_1$ in equation (2):

$$[\text{Eq. 5}]$$

$$y_1 = g\ (x)\quad \cdot\cdot\cdot\ (2)$$

with increasing x and a function h(x) that increases $y_2$ in equation (3):

$$[\text{Eq. 6}]$$

$$y_2 = h\ (x)\quad \cdot\cdot\cdot\ (3)$$

with increasing x and satisfies $y_2 > y_1$.

Additionally, the biological treatment method according to the present invention is a biological treatment method in which the biological treatment process is performed while predicting the quality of treated water after a biological treatment process of biologically treating water to be treated containing a substance to be treated with bacteria that decompose the substance to be treated, by means of a simulation that uses as a parameter the value of the maximum reaction rate of the substance to be treated with the bacteria, wherein the value of the maximum reaction rate is used as a parameter in a state where the value of the maximum reaction rate and the amount of the substance to be treated that is loaded per bacterial cell per unit time or the amount of the substance to be treated that has been treated per bacterial cell per unit time in the biological treatment process are in a functional relation, and the above-described function is an increasing function of V with increasing L, wherein V represents the value of the maximum reaction rate and L represents the amount of the substance to be treated that is loaded per bacterial cell per unit time or the amount of the substance to be treated that has been treated per bacterial cell per unit time in the biological treatment process.

In addition, in this biological treatment method, it is preferable that the value of the maximum reaction rate is used as a parameter in a state where the functional relation of equation (1):

$$[\text{Eq. 7}]$$

$$V = f\ (L)\quad \cdot\cdot\cdot\ (1)$$

is satisfied, wherein V ($fg\cdot copy^{-1}\cdot h^{-1}$) represents the value of the maximum reaction rate and L ($fg\cdot copy^{-1}\cdot day^{-1}$) represents

the amount of the substance to be treated that is loaded per bacterial cell per unit time or the amount of the substance to be treated that has been treated per bacterial cell per unit time in the biological treatment process, and the function f(x) of a variable x (wherein x>0) is a function between a function g(x) that increases $y_1$ in equation (2):

[Eq. 8]

$$y_1 = g \ (x) \quad \cdot \cdot \cdot \ (2)$$

with increasing x and a function h(x) that increases $y_2$ in equation (3):

[Eq. 9]

$$y_2 = h \ (x) \quad \cdot \cdot \cdot \ (3)$$

with increasing x and satisfies $y_2 > y_1$.

Moreover, the biological treatment apparatus according to the present invention is a biological treatment apparatus in which the biological treatment process is performed while predicting the quality of treated water after a biological treatment process of biologically treating water to be treated containing a substance to be treated with bacteria that decompose the substance to be treated, by means of a simulation that uses as a parameter the value of the maximum reaction rate of the substance to be treated with the bacteria, wherein the value of the maximum reaction rate is used as the parameter in a state where the value of the maximum reaction rate and the amount of the substance to be treated that is loaded per bacterial cell per unit time or the amount of the substance to be treated that has been treated per bacterial cell per unit time in the biological treatment process are in a functional relation, and the above-described function is an increasing function of V with increasing L, wherein V represents the value of the maximum reaction rate and L represents the amount of the substance to be treated that is loaded per bacterial cell per unit time or the amount of the substance to be treated that has been treated per bacterial cell per unit time in the biological treatment process.

In addition, in this biological treatment apparatus, it is preferable that the value of the maximum reaction rate is used as a parameter in a state where the functional relation of equation (1):

[Eq. 10]
$$V = f \ (L) \quad \cdot \cdot \cdot \ (1)$$

is satisfied, wherein V (fg·copy$^{-1}$·h$^{-1}$) represents the value of the maximum reaction rate and L (fg·copy$^{-1}$·day$^{-1}$) represents the amount of the substance to be treated that is loaded per bacterial cell per unit time or the amount of the substance to be treated that has been treated per bacterial cell per unit time in a biological treatment process, and the above-described function f(x) of a variable x (wherein x>0) is a function between a function g(x) that increases $y_1$ in equation (2):

[Eq. 11]

$$y_1 = g \ (x) \quad \cdot \cdot \cdot \ (2)$$

with increasing x and a function h(x) that increases $y_2$ in equation (3):

[Eq. 12]

$$y_2 = h \ (x) \quad \cdot \cdot \cdot \ (3)$$

with increasing x and satisfies $y_2 > y_1$.

Additionally, a "increasing function of V with increasing L, wherein V represents the value of the maximum reaction rate and L represents the amount of a substance to be treated that is loaded per bacterial cell per unit time or the amount of the substance to be treated that has been treated per bacterial cell per unit time in the biological treatment process" intends to be a state where the whole function has a positive slope when the values of L are plotted in the abscissa and the values of V are plotted in the ordinate to create a graph of this function, and also intends to include the case where the value of V locally decreases with increasing L.

Moreover, this "amount of a substance to be treated that is loaded per bacterial cell per unit time" refers to the "amount of substances to be treated" obtained by totaling the "amount of a substance to be treated that is introduced from the outside into the biological treatment process" and the "amount of a substance to be treated that is produced by other bacteria in the biological treatment process" and other amounts.

In addition, this "amount of a substance to be treated that is loaded per bacterial cell per unit time" refers to the "amount of substances to be treated that have decomposed" obtained by totaling the "amount of a substance to be treated that has decomposed, among the amount of substances to be treated that are introduced from the outside into the biological treatment process" and the "amount of a substance to be treated that has decomposed, among the amount of substances to be treated that are produced by the other bacteria in the biological treatment process" and other amounts. This "treated amount of substance to be treated that has decomposed" can be calculated by subtracting the "amount of a substance to be treated that remains in the treated liquid" from the total amount of the "amount of a substance to be treated that is introduced from the outside into the biological treatment process" and the "amount of a substance to be treated that is produced by the other bacteria and the like in the biological treatment process."

Additionally, unless otherwise indicated herein, the term "maximum reaction rate" refers to the maximum mass of a substance to be treated which is decomposed by one bacterial cell per unit time, and for example, this "maximum reaction rate" can be calculated using equation (4) from the amount of the concentration change of a substance to be treated per unit time in the biological treatment process: $\Delta S$ (fg/m$^3$/h) and the number of bacterial cells per unit volume: n (copies/m$^3$) used in the biological treatment process.

[Eq. 13]

$$\text{Maximum reaction rate} \ (fg/copy/h) = \Delta S \ (fg/m^3/h) \div n \ (copies/m^3) \qquad \cdots (4)$$

Moreover, the variation in concentration of a substance to be treated that is decomposed per unit time can be calculated, for example, by preparing a sample obtained by mixing a solution containing a substance to be treated and sludge containing bacteria that decomposes the substance to be treated and determining the variation of the concentration of the substance to be treated in the sample with time.

The "amount of a substance to be treated that is loaded per bacterial cell per unit time in the biological treatment process" can also be calculated, for example, by calculating the total amount Q (fg/m$^3$/day) of the amount of the substance to be treated that is introduced from the outside into the biological treatment process per unit time per unit volume and the amount of the substance to be treated that is produced by other bacteria and the like in the biological treatment process per unit time per unit volume and the number of bacteria n (copies/m$^3$) used per unit volume in the biological process and then calculating equation (5) below.

[Eq. 14]

Amount of substance to be treated that is loaded per bacterial cell per unit time in the biological treatment process:

$$\mathrm{(fg/copy/day)} = Q \;\mathrm{(fg/m^3/day)} \div n \;\mathrm{(copies/m^3)} \qquad : L$$

$$\cdots (5)$$

Further, the "amount of a substance to be treated that is treated per bacterial cell per unit time in the biological treatment process" can also be calculated by replacing "Q(fg/m³/day)" in equation (5) above by the amount obtained by subtracting the amount of the substance to be treated that remains in the treat liquid from the total amount of the amount of the substance to be treated that is introduced from the outside in the biological treatment process and the amount of the substance to be treated that is produced by the other bacteria and the like and calculating "L (fg/copy/day)" in equation (5) above.

In addition, "the function f(x) indicating the relationship between the maximum reaction rate and the amount of a substance to be treated that is loaded per bacterial cell per unit time or the amount of the substance to be treated that has been treated per bacterial cell per unit time, in the biological treatment process, is a function between g(x) and h(x)" means that "the relation h(x)>f(x)>g(x) is satisfied," wherein x>0.

Additionally, the relation h(x)>f(x)>g(x) may be simply satisfied in the range of the values that are practically taken by the amount of a substance to be treated that is loaded per bacterial cell per unit time or the amount (L: $\mathrm{fg \cdot copy^{-1} \cdot day^{-1}}$) of the substance to be treated that has been treated per bacterial cell per unit time, in the biological treatment process, and for example, the relation h(x)>f(x)>g(x) may not be satisfied in the case where x becomes infinite. The range of the values that can be substantially taken by the amount of a substance to be treated that is loaded per bacterial cell per unit time or the amount of the above-described substance to be treated (L) that has been treated per bacterial cell per unit time in this biological treatment process is typically from 100 to 4,000 ($\mathrm{fg \cdot copy^{-1} \cdot day^{-1}}$) in the process of ammonia oxidation by ammonia-oxidizing bacteria for treating nitrogen-containing discharge water that mainly contains nitrogen compounds such as ammonia or nitric acid as the substances to be treated and rarely contains other organic compounds, or the like.

Moreover, for example, in the process of nitrite oxidation by nitrite-oxidizing bacteria, the range is typically from 1,000 to 60,000 ($\mathrm{fg \cdot copy^{-1} \cdot day^{-1}}$).

In addition, the range is typically from 5 to 70 ($\mathrm{fg \cdot copy^{-1} \cdot day^{-1}}$) in the process of nitrate reduction by nitrate-reducing bacteria.

Furthermore, the range is typically from 5 to 120 ($\mathrm{fg \cdot copy^{-1} \cdot day^{-1}}$) in the process of nitrite reduction by nitrite-reducing bacteria.

Additionally, considering the case where other organic compounds are also included in addition to nitrogen compounds such as ammonia, such as sewage, the range of the values that can be substantially taken by the amount of a substance to be treated that is loaded per bacterial cell per unit time or the amount of the substance to be treated (L) that has been treated per bacterial cell per unit time, in the biological treatment process, is usually from 100 to 35,000 ($\mathrm{fg \cdot copy^{-1} \cdot day^{-1}}$) in the process of the ammonia oxidation by ammonia-oxidizing bacteria.

Moreover, in the process of nitrite oxidation by nitrite-oxidizing bacteria, the range is typically from 1,000 to 12,000,000 ($\mathrm{fg \cdot copy^{-1} \cdot day^{-1}}$).

In addition, the range is typically from 5 to 2,500 ($\mathrm{fg \cdot copy^{-1} \cdot day^{-1}}$) in the process of nitrate reduction by nitrate-reducing bacteria.

Furthermore, the range is typically from 5 to 3,500 ($\mathrm{fg \cdot copy^{-1} \cdot day^{-1}}$) in the process of nitrite reduction by nitrite-reducing bacteria.

Additionally, the amount of a substance to be treated that is treated per bacterial cell per unit time cannot exceed the amount of the substance to be treated that is loaded per bacterial cell per unit time, in the biological treatment process in a usual biological treatment process.

In addition, in the cases where the amount of a substance to be treated (nitrogen component) that is loaded per bacterial cell per unit time in the biological treatment process such as nitrification and denitrification is from 100 to 35,000 ($\mathrm{fg \cdot copy^{-1} \cdot day^{-1}}$) in the process of ammonia oxidation by ammonia-oxidizing bacteria, where the amount is from 1,000 to 120,000,000 ($\mathrm{fg \cdot copy^{-1} \cdot day^{-1}}$) in the process of nitrite oxidation by nitrite-oxidizing bacteria, where the amount is from 5 to 2,500 ($\mathrm{fg \cdot copy^{-1} \cdot day^{-1}}$) in the process of the nitrate reduction by nitrate-reducing bacteria, and where the amount is from 5 to 3,500 ($\mathrm{fg \cdot copy^{-1} \cdot day^{-1}}$) in the process of nitrite reduction by nitrite-reducing bacteria, the simulation results on the basis of the amounts of the substance to be treated that has been treated per bacterial cell per unit time have a small possibility of having large differences between the simulation results and the actual values.

EFFECTS OF THE INVENTION

According to the present invention, also in the case where the amount or the like of the substance to be treated that is loaded per unit number of bacteria per unit time in the biological treatment process is changed, the maximum reaction rate of the substance to be treated with bacteria can be defined more precisely, thereby being capable of improving the prediction accuracy of the simulation.

Therefore, the load of the calibration operation may be reduced while suppressing a decrease in prediction accuracy in the simulation method and the simulation apparatus.

The inspection frequency of actual treated water quality can also be decreased, so that the load required for the biological treatment method or operation of the biological treatment apparatus may be reduced.

BRIEF DESCRIPTION OF THE DRAWINGS

Fig. 1 is a schematic block diagram indicating a biological treatment apparatus.

Fig. 2 shows a graph indicating a correlation between the maximum reaction rate in an ammonia oxidation reaction by ammonia-oxidizing bacteria and the amount of a substance to be treated that is loaded per bacterial cell per day in Experimental Example 1.

Fig. 3 shows a graph indicating a correlation between the maximum reaction rate in a nitrite oxidation reaction by nitrite-oxidizing bacteria and the amount of a substance to be treated that is loaded per bacterial cell per day in Experimental Example 1.

Fig. 4 shows a graph indicating a correlation between the maximum reaction rate in a nitrate reduction reaction by nitrate-reducing bacteria and the amount of a substance to be treated that is loaded per bacterial cell per day in Experimental Example 1.

Fig. 5 shows a graph indicating a correlation between the maximum reaction rate in a nitrite reduction reaction by nitrite-reducing bacteria and the amount of a substance to be treated that is loaded per bacterial cell per day in Experimental Example 1.

Fig. 6 is a schematic diagram indicating experimental facilities that models a biological treatment apparatus.

Fig. 7 shows a graph indicating a load fluctuation of nitrogen.

Fig. 8 shows a graph indicating treated water quality in a biological treatment process (after experiment).

Fig. 9 shows a graph indicating a change in the number of bacteria in a reaction tank during the experimental period.

Fig. 10 shows a graph indicating a change in the number of bacteria in the reaction tank during the experimental period.

Fig. 11 shows a graph indicating a change in MLSS in the reaction tank during the experimental period.

Fig. 12 shows a graph indicating a correlation between the maximum reaction rate in the ammonia oxidation reaction by ammonia-oxidizing bacteria and the amount of a substance to be treated that is loaded per bacterial cell per day in Experimental Example 2.

Fig. 13 shows a graph indicating a correlation between the maximum reaction rate in the nitrite oxidation reaction by nitrite-oxidizing bacteria and the amount of a substance to be treated that is loaded per bacterial cell per day in Experimental Example 2.

Fig. 14 shows a graph indicating a correlation between the maximum reaction rate in the nitrate reduction reaction by nitrate-reducing bacteria and the amount of a substance to be treated that is loaded per bacterial cell per day in Experimental Example 2.

Fig. 15 shows a graph indicating a correlation between the maximum reaction rate in the nitrite reduction reaction by nitrite-reducing bacteria and the amount of a substance to be treated that is loaded per bacterial cell per day in Experimental Example 2.

Fig. 16 shows a graph indicating a correlation between the maximum reaction rate in the ammonia oxidation reaction by ammonia-oxidizing bacteria and the amount of a substance to be treated that is loaded per bacterial cell per day, covering Experimental Examples 1 to 3.

Fig. 17 shows a graph indicating a correlation between the maximum reaction rate in the nitrite oxidation reaction by nitrite-oxidizing bacteria and the amount of a substance to be treated that is loaded per bacterial cell per day, covering Experimental Examples 1 to 3.

Fig. 18 shows a graph indicating a correlation between the maximum reaction rate in the nitrate reduction reaction by nitrate-reducing bacteria and the amount of a substance to be treated that is loaded per bacterial cell per day, covering Experimental Examples 1 to 3.

Fig. 19 shows a graph indicating a correlation between the maximum reaction rate in the nitrite reduction reaction by nitrite-reducing bacteria and the amount of a substance to be treated that is loaded per bacterial cell per day, covering Experimental Examples 1 to 3.

Fig. 20 shows a comparison table indicating the difference between the simulation of the present invention and a conventional simulation.

Fig. 21 shows simulation-result comparison graphs in the simulation of the present invention and the conventional simulation.

Fig. 22 shows simulation-result comparison graphs in the simulation of the present invention and the conventional simulation.

Fig. 23 shows a graph indicating a correlation between the maximum reaction rate in the ammonia oxidation reaction by ammonia-oxidizing bacteria and the amount of a substance to be treated that is loaded per bacterial cell per day (results in Experimental Example 4).

Fig. 24 shows a graph indicating a correlation between the maximum reaction rate in the nitrite oxidation reaction by nitrite-oxidizing bacteria and the amount of the substance to be treated that is loaded per bacterial cell per day (results in Experimental Example 4).

Fig. 25 shows a graph indicating a correlation between the maximum reaction rate in the nitrate reduction reaction by nitrate-reducing bacteria and the amount of the substance to be treated that is loaded per bacterial cell per day (results in Experimental Example 4).

Fig. 26 shows a graph indicating a correlation between the maximum reaction rate in the nitrite reduction reaction of nitrite-reducing bacteria and the amount of the substance to be treated that is loaded per bacterial cell per day (results in Experimental Example 4).

Fig. 27 shows a graph indicating changes in the maximum reaction rate by ammonia-oxidizing bacteria for chloride ion concentrations (results of Experimental Example 5).

Fig. 28 shows a graph indicating changes in the maximum reaction rate by nitrite-oxidizing bacteria for chloride ion concentrations (results of Experimental Example 5).

Fig. 29 shows a graph indicating changes in the maximum reaction rate by nitrate-reducing bacteria for chloride ion concentrations (results of Experimental Example 5).

Fig. 30 shows a graph indicating changes in the maximum reaction rate by nitrite-reducing bacteria for chloride ion concentrations (results of Experimental Example 5).

DESCRIPTIONS OF THE REFERENCE NUMERALS

2: Nitrification tank, 4: Denitrification tank, 6: Reaeration tank, and 8: Sedimentation tank

BEST MODE FOR CARRYING OUT THE INVENTION

A simulation method of biological discharge water treatment of this embodiment will be described by way of an example in which a biological treatment process such as nitrification/denitrification is carried out using, as water to be treated, discharge water containing a nitrogen component as a substance to be treated.

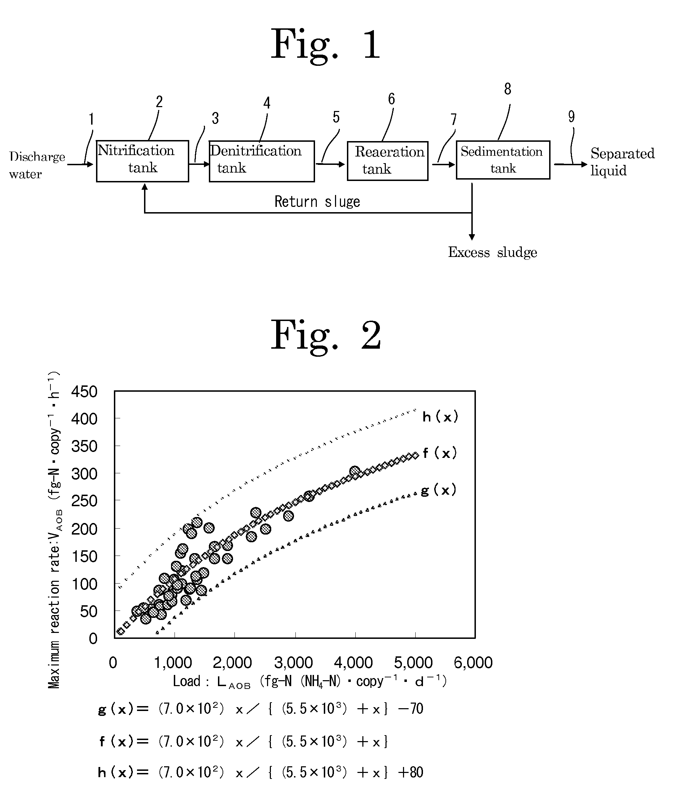

Fig. 1 is a schematic block diagram indicating a biological treatment apparatus for performing a biological treatment of discharge water while predicting the quality of treated water after each biological treatment process by the simulation method of this embodiment.

In the drawing, reference numeral 1 represents a first connecting duct for introducing discharge water into a series of treatment processes; and reference numeral 2 represents a nitrification tank into which discharge water (water to be treated) is introduced through the first connecting duct 1.

In the drawing, reference numeral 3 is a second connecting duct in which a treated liquid discharged from a nitrification tank 2 flows; and reference numeral 4 represents a denitrification tank into which a treated liquid of the nitrification tank 2 flows as water to be treated through this second connecting duct 3.

In the drawing, reference numeral 5 is a third connecting duct in which a treated liquid discharged from a denitrification tank 4 flows; and reference numeral 6 represents a reaeration tank into which treated liquid of the denitrification tank 4 flows as water to be treated through this third connecting duct 5.

In the drawing, reference numeral 7 is a fourth connecting duct in which a treated liquid discharged from a reaeration tank 6 flows; and reference numeral 8 represents a sedimentation tank into which treated liquid of the reaeration tank 6 flows as water to be treated through this fourth connecting duct 7.

In addition, in the drawing, reference numeral 9 represents a fifth connecting duct through which a supernatant liquid settled and separated from the sedimentation tank 8 is discharged as a separated liquid outside the system. Additionally, though not described in detail here, the biological treatment apparatus shown in this Fig. 1 includes a connecting duct (hereinafter, may be called "sludge withdrawing piping") for discharging as excess sludge a part of sludge settled and separated in the sedimentation tank 8 outside the system and a connecting duct (hereinafter, may also be called "sludge return piping") for sending back a part of the sludge settled and separated in the sedimentation tank 8 to the nitrification tank 2.

Water to be treated (discharge water) introduced through the first connecting duct 1 into the nitrification tank 2 typically includes ammonia nitrogen.

In addition, activated sludge including ammonia-oxidizing bacteria, nitrite-oxidizing bacteria, and the like, is housed in the nitrification tank 2, and ammonia nitrogen, which is a substance to be treated, introduced into the nitrification tank 2 by the introduction of the above-described water to be treated is decomposed (oxidized) by bacteria in a mixed phase formed in the nitrification tank 2 with the activated sludge and the water to be treated.

Examples of the ammonia-oxidizing bacteria include, for example, Nitrosomonas, Nitrosococcus and the like; examples of the nitrite-oxidizing bacteria include, for example, Nitrobacter, Nitrospira, and the like.

Additionally, bacteria showing oxidation capacity in the decomposition of organic substances in water to be treated may further be contained in the activated sludge as bacteria showing oxidation capacity and examples of such bacteria include, for example, Bacillus, Zoogloea, Micrococcus species, and other species.

Various measurements are determined for mixed phases formed in this nitrification tank 2 by water to be treated and activated sludge that are flowed therein.

In this nitrification tank 2, a simulation apparatus is disposed that predicts the quality of treated water flowed downward toward the denitrification tank 4 using the measurements as parameters.

This simulation apparatus uses as parameters the values of the maximum reaction rates in nitrification reactions caused by bacteria contained in the sludge and also the values of the maximum reaction rates are used in a state where the maximum reaction rates have functional relations with the amounts of ammonia nitrogen that is loaded by ammonia-oxidizing bacteria in this nitrification tank 2.

A simulation method by this simulation apparatus will be set forth in detail in a latter part.

Activated sludge including nitrate-reducing bacteria, nitrite-reducing bacteria, nitrous oxide reducing bacteria, and the like is housed in the denitrification tank 4.

In addition, nitrate nitrogen, nitrite nitrogen, nitrous oxide, and the like, which are substances to be treated, are introduced into this denitrification tank 4 by the introduction of treated water of the nitrification tank 2 (water to be treated in the denitrification tank 4).

The introduced substances to be treated are decomposed (reduced) by the bacteria in a mixed phase formed in the denitrification tank 4 from the activated sludge and the water to be treated.

Then, nitrite nitrogen, nitrate nitrogen and the like are reduced into nitrogen gas by the bacteria, which is released to the atmosphere, whereby the nitrogen components are removed from the water to be treated.

For example, bacteria involved in denitrification activity can be contained in the activated sludge of the denitrification tank 4 as bacteria that show reducing ability to the substance to be treated and the examples include Alcaligenes, Azoarcus, Paracoccus, Pseudomonas species and other species.

Various measurements are carried out on the mixed phase formed in this denitrification tank 4 from water to be treated and activated sludge that are flowed therein from the nitrification tank.

Then, a simulation apparatus is disposed that predicts the quality of treated water flowed downward toward the reaeration tank 6, using the measurements as parameters.

This simulation apparatus uses as parameters the values of the maximum reaction rates in denitrification caused by bacteria contained in the sludge and also the values of the maximum reaction rates are used in a state where the maximum reaction rates have functional relations with the amounts of the substances to be treated that are loaded to the bacteria in the denitrification tank 4.

A simulation method by this simulation apparatus will be set forth in detail in a latter part.

The reaeration tank 6 is provided with an aeration means (not shown) for decomposing and removing organic substances that remain in the treated water (water to be treated that is introduced from the denitrification tank 4) of the denitrification tank 4 under an aerobic condition.

The sedimentation tank 8 is formed so as to have a volume sufficient for the inflow of water to be treated to secure an average retention time necessary to settle and separate solid components such as activated sludge contained in water to be treated that is introduced from the reaeration tank 6 from liquid components.

Next, the simulation apparatus and the simulation method will be described.

The simulation model of water quality used for this simulation apparatus can use a model that incorporates a functional relation between the value of the maximum reaction rate and the amount of a substance to be treated that is loaded per bacterial cell per unit time (e.g., one day) in the biological treatment process into ASM1, ASM2, ASM2d, ASM3, or the like that is made by IWA (International Water Association) and modifies it.

In particular, ASM3 is easy in expansion of its model, so that a simulation model on the basis of ASM3 is preferably used.

For instance, a function is incorporated into a simulation apparatus such that a specified relationship between the value of the maximum reaction rate and the amount of the substance to be treated that is loaded per bacterial cell per day may be maintained for a model based on the ASM3 and simulation is implemented.

Here, the definition of a function constructed between the value of the maximum reaction rate and the amount of the substance to be treated that is loaded per bacterial cell per day in the biological treatment process will be described. This function can be provided, for example, by collecting a plurality of data to obtain the correlation between the value of the maximum reaction rate and the amount of the substance to be processed that is loaded per bacterial cell per day in the biological treatment process and executing regression analysis using the data constellation.

This maximum reaction rate: V (fg/copy/h) can be calculated by calculating the amount of the concentration change of a substance to be treated per unit time in the biological treatment process: $\Delta S$ (fg/m³/h) and the number of bacteria per unit volume used in the biological treatment process: n (copies/m³) and calculating equation (4):

[Eq. 15]

Maximum reaction rate

$$ (fg/copy/h) = \Delta S (fg/m^3/h) \div n (copies/m^3) \quad \cdots (4) $$

In addition, the unit of the number of bacteria (copy) can be converted into the unit of biomass (g-COD$_{Cr}$) and adopted for simulation, and for example, the number of bacteria (copy) can be converted into the unit of biomass (g-COD$_{Cr}$) with a conversion factor of the number of bacteria: 1 copy = $3.965 \times 10^{-10}$ mg - COD$_{Cr}$ = $3.965 \times 10^{-13}$g - COD$_{Cr}$ and adopted.

The amount of a substance to be treated that is loaded per bacterial cell per day in the biological treatment process: L (fg/copy/day) can be calculated by calculating the amount of a substance to be treated that is loaded per unit time per unit volume in the biological treatment process: Q (fg/m³/day) and the number of bacteria per unit volume used in the biological treatment process: n (copies/m³) and calculating equation (5):

[Eq. 16]

Amount of substance to be treated that is loaded per bacterial cell per day in the

biological treatment

$$ :L \quad (fg/copy/day) = Q (fg/m^3/day) \div n (copies/m^3) \quad \cdots (5) $$

process                                                                                          .

Additionally, the amount (value of Q) of a substance to be treated that is loaded per unit time per unit volume does not need measurement for one day, and for example, the value of a load for half a day is measured and its value is doubled to obtain a value per day, and inversely measurements for two days or more are divided by the number of measurement days to obtain a value per day.

Then, when the value of a substance to be measured that is loaded per bacterial cell per day in the biological treatment process: L (fg·copy$^{-1}$·day$^{-1}$) is changed, the change of the value of the maximum reaction rate: V (fg·copy$^{-1}$·day$^{-1}$) is analyzed based on data by collecting the data of several points to tens of points.

In addition, the number of points for data collection is preferably at least 4 points.

These data typically form a point group that rises to its right side when the amount of a substance to be treated that is loaded per bacterial cell per day in the biological treatment process is taken as the x axis (abscissa) and the value of the maximum reaction rate is taken as the y axis (ordinate) and then the data are plotted on this coordinate.

Additionally, the value of the maximum reaction rate can be determined by the measurement method that is described in the latter part.

That is to say, these point groups can substantially be made to be between the function g(x) that increases $y_1$ in equation (2):

[Eq. 17]

$$y_1 = g(x) \quad \cdots \quad (2)$$

with increasing x and a function h(x) that increases $y_2$ in equation (3):

[Eq. 18]

$$y_2 = h(x) \quad \cdots \quad (3)$$

with increasing x and satisfies $y_2 > y_1$.

Then, the function f(x) that is between g(x) and h(x) is properly set, so that the value of the maximum reaction rate: V ($fg \cdot copy^{-1} \cdot day^{-1}$) is used as a parameter for a simulation apparatus in a state where the value of the maximum reaction rate has the functional relation of equation (1):

[Eq. 19]

$$V = f(L) \quad \cdots \quad (1)$$

to the value of a substance to be treated that is loaded per bacterial cell per day in the biological treatment process: (L value), whereby the prediction accuracy of the quality of the treated water can be improved as compared with the conventional simulation and thus the calibration operation load can be reduced.

Therefore, this function f(x) usually increases the value of the maximum reaction rate (V) as the value (L) of the amount of a substance to be treated that is loaded per bacterial cell per day in the biological treatment process is increased.

In addition, the function f(x) is preferably selected from the functions defined by, for example, general equation (6):

[Eq. 20]

$$f(x) = a \cdot x / (b + x) + c \quad \cdots \quad (6)$$

(wherein a, b and c are constants) from the viewpoint of being capable of approximating the simulation result by actual treated water quality.

Next, the simulation apparatus and the simulation method in each biological treatment process for nitrification and denitrification will be described in more detail.

(Nitrification process)

In the nitrification tank 2, since two processes of ammonia oxidation in which ammonia nitrogen is decomposed to nitrite nitrogen by ammonia-oxidizing bacteria and nitrite oxidation in which a part or the whole of the nitrite nitrogen formed by the ammonia oxidation is oxidized to nitrate nitrogen by nitrite-oxidizing bacteria are carried out, parameters are set for each process.

(Ammonia oxidation)

The oxidation reaction of ammonia nitrogen in the nitrification tank 2 has been given by equation (7) below, for example, in the conventional model.
[Eq. 21]

$$\frac{dS_{NH4}}{dt} = \frac{\mu_{NH4}}{Y_{NH4}} \frac{S_{NH4}}{K_{NH4} + S_{NH4}} \frac{S_{O2}}{K_{O2} + S_{O2}} \frac{S_{ALK}}{K_{ALK} + S_{ALK}} X_{NH4} \quad \cdots \quad (7)$$

wherein,

$\mu_{NH4}$: Maximum specific growth rate (1/day)
$Y_{NH4}$: Growth yield (g-$COD_{Cr}$/g$NH_4$-N)
$S_{O2}$: Dissolved oxygen concentration (g$O_2$/m$^3$)
$S_{NH4}$: Soluble ammonia concentration (g $NH_4$-N/m$^3$)
$S_{ALK}$: Alkalinity (mole $HCO_3$/m$^3$)
$K_{O2}$: Saturation coefficient for dissolved oxygen (g$O_2$/m$^3$)
$K_{NH4}$: Saturation coefficient for ammonia (g$NH_4$-N/ m$^3$)
$K_{ALK}$: Saturation coefficient for alkalinity (mole $HCO_3$/ m$^3$)
$X_{NH4}$: Ammonia-oxidizing bacterial concentration in nitrification tank (g-$COD_{Cr}$/m$^3$)

In addition, since these $\mu_{NH4}$ and $Y_{NH4}$ are constants, the value of ($\mu_{NH4}$/$Y_{NH4}$), which is the maximum mass of a substance to be treated that is decomposed by unit ammonia-oxidizing bacterial amount per day (maximum reaction rate), has been given a constant in the conventional model.
On the other hand, in the simulation apparatus according to the present embodiment, equation (8) below is incorporated and used in place of the model of equation (7) above.
[Eq. 22]

$$\frac{dS_{NH4}}{dt} = f_{AOB}(L_{AOB}) \frac{S_{NH4}}{K_{NH4} + S_{NH4}} \frac{S_{O2}}{K_{O2} + S_{O2}} \frac{S_{ALK}}{K_{ALK} + S_{ALK}} X_{AOB} \quad \cdots \quad (8)$$

wherein

$L_{AOB}$: Amount of ammonia nitrogen that is loaded to one ammonia-oxidizing bacterial cell per day (fg$NH_4$-N/copy/day)
$S_{O2}$: Dissolved oxygen concentration (g$O_2$/m$^3$)
$S_{NH4}$: Soluble ammonia concentration (g$NH_4$-N/m$^3$)
$S_{ALK}$: Alkalinity (mole $HCO_3$/m$^3$)
$K_{O2}$: Saturation coefficient for dissolved oxygen (g$O_2$/m$^3$)
$K_{NH4}$: Saturation coefficient for ammonia (g $NH_4$-N/m$^3$)
$K_{ALK}$: Saturation coefficient for alkalinity (mole $HCO_3$/m$^3$)
$X_{AOB}$: Ammonia-oxidizing bacterial concentration in nitrification tank copies/m$^3$)

In addition, it is described previously that biomass (g-$COD_{Cr}$) can be adopted for the simulation in place of the number of bacteria (copy).
Additionally, the maximum mass of a substance to be treated that is decomposed by unit ammonia-oxidizing bacterial amount per day (i.e., the maximum reaction rate ($\mu NH_4$/$Y_{NH4}$) in equation (7)) is set to be the function ($f_{AOB}$ ($L_{AOB}$)) that has as a variable an amount of ammonia nitrogen that is loaded per ammonia-oxidizing bacterial cell per day, whereby the prediction accuracy of the quality of treated water can be improved as compared with the conventional simulation and the calibration operation load can be reduced.
Moreover, the function used in this equation (8) having x (wherein x>0) as a variable is made a function that is between a function $g_{AOB}(x)$ that increases $y_{AOB1}$ in equation (9):

[Eq. 23]

$$y_{AOB1} = g_{AOB}(x) \quad \cdots \quad (9)$$

with increasing x and a function $h_{AOB}(x)$ that increases $y_{AOB2}$ in equation (10):

[Eq. 24]

$$y_{AOB2} = h_{AOB}(x) \quad \cdots \quad (10)$$

with increasing x and also satisfies $y_{AOB2} > y_{AOB1}$, whereby the prediction accuracy of the simulation may be improved further.

Typically, in the reaction of ammonia oxidation by ammonia-oxidizing bacteria used in the biological treatment of water to be treated (discharge water in which an object to be treated is mainly a nitrogen component) that rarely contains organic compounds and the like except nitrogen compounds such as ammonia and nitric acid, in the case where the amount of ammonia nitrogen that is loaded per its ammonia-oxidizing bacterial cell per day: $L_{AOB}$ (fgNH$_4$-N/copy/day) is in the range of $100 \leq L_{AOB} \leq 4,000$, the function $g_{AOB}(x)$ in equation (9) is set to be equation (11):

[Eq. 25]

$$g_{AOB}(x) = \{a_1 \cdot x / (b_1 + x)\} + c_1 \quad \cdots \quad (11)$$

(wherein, $a_1 = 7.0 \times 10^2$, $b_1 = 5.5 \times 10^3$, and $c_1 = -7.0 \times 10$) and the function $h_{AOB}(x)$ in equation (10) is given by equation (12):

[Eq. 26]

$$h_{AOB}(x) = \{a_2 \cdot x / (b_2 + x)\} + c_2 \quad \cdots \quad (12)$$

(wherein, $a_2 = 7.0 \times 10^2$, $b_2 = 5.5 \times 10^3$, and $c_2 = 8.0 \times 10$), whereby the prediction accuracy of the simulation may be further improved.

In other words, for example, the maximum reaction rate by the ammonia-oxidizing bacteria in the biological treatment process is defined by equation (13):

[Eq. 27]

$$V_{AOB} = (7.0 \times 10^2) \times L_{AOB} / \{(5.5 \times 10^3) + L_{AOB}\} \quad \cdots \quad (13)$$

wherein $V_{AOB}$: Maximum reaction rate (fg·copy$^{-1}$·day$^{-1}$) and $L_{AOB}$: Amount of ammonia nitrogen that is loaded per ammonia-oxidizing bacterial cell per day (fg NH$_4$-N·copy$^{-1}$·day$^{-1}$) and can be used as a parameter for the simulation apparatus.

In addition, in the case where ammonia oxidation reaction is predicted in ammonia-oxidizing bacteria as a whole used in the biological treatment of water to be treated that generally contains nitrogen components as substance to be treated, including sewage, the values of $a_1$, $b_1$ and $c_1$ in equation (11) are set to $4.0 \times 10^3$, $1.0 \times 10^4$ and $-2.5 \times 10^3$, respectively, and the values of $a^2$, $b^2$ and $c^2$ in equation (12) are set to $4.0 \times 10^3$, $1.0 \times 10^4$ and $2.5 \times 10^3$, respectively, so that predicted values with high precision can be obtained as compared with those in the conventional simulation.

Therefore, the function that relates $V_{AOB}$ and $L_{AOB}$ is preferably set such that the maximum reaction rate: $V_{AOB}$ (fg·copy$^{-1}$·day$^{-1}$) satisfies the relation:

$$\{4.0 \times 10^3 \cdot L_{AOB}/(1.0 \times 10^4 + L_{AOB}) - 2.5 \times 10^3\} \leq V_{AOB} \leq \{4.0 \times 10^3 \cdot L_{AOB}/(1.0 \times 10^4 + L_{AOB}) + 2.5 \times 10^3\}$$

in the case where the amount of ammonia nitrogen: $L_{AOB}$ (fg·copy$^{-1}$·day$^{-1}$) that is loaded per ammonia-oxidizing bacterial cell per day meets the relation of $1.0 \times 10^2 \leq L_{AOB} \leq 3.5 \times 10^4$.

According to this preferred aspect, the function is generally applicable to biological treatment of water to be treated in which nitrogen components are contained therein as the substance to be treated, including sewage as well.

Moreover, when chloride ions together with nitrogen components are contained in water to be treated, the chloride ion concentration also affects the maximum reaction rate.

Moreover, since ammonia-oxidizing bacteria tend to increase the maximum reaction rate of the ammonia oxidation reaction with increasing the chloride ion concentration, the value of the maximum reaction rate is used as a parameter in a state where the maximum reaction rate has a specified functional relation to the amount of ammonia nitrogen and also to the amount of chloride ions, in water to be treated, whereby the precision of the simulation can be further improved.

Therefore, the precision of the simulation can be further improved by incorporating equation (14) below in place of the model of equation (8) and using it.

[Eq. 28]

$$\frac{dS_{NH4}}{dt} = f_{AOB}(L_{AOB}) k_{AOB}(D_{CL}) \frac{S_{NH4}}{K_{NH4} + S_{NH4}} \frac{S_{O2}}{K_{O2} + S_{O2}} \frac{S_{ALK}}{K_{ALK} + S_{ALK}} X_{AOB} \quad \cdots \quad (14)$$

wherein,

$L_{AOB}$: Amount of ammonia nitrogen that is loaded per ammonia-oxidizing bacterial cell per day (fg NH4-N/copy/day)
$D_{CL}$: Chloride ion concentration in water to be treated that flows into nitrification process (mg/l)
$S_{O2}$: Dissolved oxygen concentration (g $O_2/m^3$)
$S_{NH4}$: Soluble ammonia concentration (g NH4-N/m$^3$)
$S_{ALK}$: Alkalinity (mole HCO3/m$^3$)
$K_{O2}$: Saturation coefficient for dissolved oxygen (g $O_2/m^3$)
$K_{NH4}$: Saturation coefficient for ammonia (g NH4-N/m$^3$)
$K_{ALK}$: Saturation coefficient for alkalinity (mole HCO3/m$^3$)
$X_{AOB}$: Ammonia-oxidizing bacterial concentration in nitrification tank (copies/m$^3$)

In addition, it is described previously that biomass (g-COD$_{Cr}$) can be adopted for the simulation in place of the number of bacteria (copy).

Moreover, the maximum mass of a substance to be treated that is decomposed per ammonia-oxidizing bacterial cell per day (i.e., maximum reaction rate: $V_{AOB}$) is set to be a product ($V_{AOB} = f_{AOB}(L_{AOB}) \cdot k_{AOB}(D_{CL})$) of a function ($f_{AOB}(L_{AOB})$) that has a variable of the amount of ammonia nitrogen that is loaded per ammonia-oxidizing bacterial cell per day and a function ($k_{AOB}(D_{CL})$) that has a variable of the chloride ion concentration, whereby the prediction accuracy of the quality of treated water can be improved as compared with the conventional simulation and the calibration operation load can be reduced.

This function ($k_{AOB}(D_{CL})$) having the variable of the chloride ion concentration is preferably set such that the chloride ion concentration in the water to be treated that is flowed into the nitrification process: $D_{CL}$(mg/l) satisfies $(1 + 4.4 \times 10^{-5} \cdot D_{CL}) \leq k_{AOB}(D_{CL}) \leq (1 + 1.64 \times 10^{-4} \cdot D_{CL})$ within the range of $D_{CL} \leq 4.0 \times 10^3$.

(Nitrite oxidation)

The oxidation reaction of nitrite nitrogen in the nitrification tank 2 has been given by equation (15) below, for example, in the conventional model.

[Eq. 29]

$$\frac{dS_{NO2}}{dt} = \frac{\mu_{NO2}}{Y_{NO2}} \frac{S_{NO2}}{K_{NO2} + S_{NO2}} \frac{S_{O2}}{K_{O2} + S_{O2}} \frac{S_{ALK}}{K_{ALK} + S_{ALK}} X_{NO2} \quad \cdots \quad (15)$$

wherein,

$\mu_{NO2}$: Maximum specific growth rate (1/day)
$Y_{NO2}$: Growth yield (g-$COD_{Cr}$/g$NO_2$-N)
$S_{O2}$: Dissolved oxygen concentration (g$O_2$/m$^3$)
$S_{NO2}$: Soluble nitrite concentration (g$NO_2$-N/m$^3$)
$S_{ALK}$: Alkalinity (mole $HCO_3$/m$^3$)
$K_{O2}$: Saturation coefficient for dissolved oxygen (g $O_2$/m$^3$)
$K_{O2}$: Saturation coefficient for nitrite (g$NO_2$_N/m$^3$)
$K_{ALK}$: Saturation coefficient for alkalinity (mole $HCO_3$/m$^3$)
$X_{NO2}$: Nitrite-oxidizing bacterial concentration in nitrification tank (g-$COD_{Cr}$/m$^3$)

In addition, since these $\mu_{NO2}$ and $Y_{NO2}$ are constants, the value of ($\mu_{NO2}$/$Y_{NO2}$), which is the maximum mass of a substance to be treated that is decomposed by unit nitrite-oxidizing bacterial amount per day (maximum reaction rate), has been given a constant in the conventional model.

On the other hand, in the simulation apparatus according to the present embodiment, equation (16) below is incorporated and used in place of the model of equation (15) above.

[Eq. 30]

$$\frac{dS_{NO2}}{dt} = f_{NOB}(L_{NOB}) \frac{S_{NO2}}{K_{NO2} + S_{NO2}} \frac{S_{O2}}{K_{O2} + S_{O2}} \frac{S_{ALK}}{K_{ALK} + S_{ALK}} X_{NOB} \quad \cdots \quad (16)$$

wherein,

$L_{NOB}$: Amount of nitrite nitrogen that is loaded to one nitrite-oxidizing bacterial cell per day (fg$NO_2$-N/copy/day)
$S_{O2}$: Dissolved oxygen concentration (g$O_2$/m$^3$)
$S_{NO2}$: Soluble nitrite concentration (g$NO_2$-N/m$^3$)
$S_{ALK}$: Alkalinity (mole $HCO_3$/m$^3$)
$K_{O2}$: Saturation coefficient for dissolved oxygen (g$O_2$/m$^3$)
$K_{O2}$: Saturation coefficient for nitrite (g$NO_2$-N/m$^3$)
$K_{ALK}$: Saturation coefficient for alkalinity (mole $HCO_3$/m$^3$)
$X_{NOB}$: Nitrite-oxidizing bacterial concentration in nitrification tank (copies/m$^3$)

In addition, it is described previously that biomass (g-$COD_{Cr}$) can be adopted for the simulation in place of the number of bacteria (copy).

Additionally, the maximum mass of a substance to be treated that is decomposed by unit nitrite-oxidizing bacterial amount per day (i.e., the maximum reaction rate ($\mu_{NO2}$/$Y_{NO2}$) in equation (15)) is set to be the function ($f_{NOB}$($L_{NOB}$)) that has as a variable an amount of nitrite nitrogen that is loaded per nitrite-oxidizing bacterial cell per day, whereby the prediction accuracy of the quality of treated water can be improved as compared with the conventional simulation and the calibration operation load can be reduced.

Moreover, the function used in this equation (16) having x as a variable (wherein x>0) is made a function that is between a function $g_{NOB}(x)$ that increases the value of $y_{NOB1}$ in equation (17):

[Eq. 31]

$$y_{NOB1} = g_{NOB}(x) \quad \cdots \quad (17)$$

with increasing x and a function $h_{NOB}(x)$ that increases the value of $y_{NOB2}$ in equation (18):

[Eq. 32]

$$y_{NOB2} = h_{NOB}(x) \quad \cdots \quad (18)$$

with increasing x and also satisfies $y_{NOB2} > y_{NOB1}$, whereby the prediction accuracy of the simulation may be improved further.

Typically, in the case where nitrite nitrogen is oxidized when the amount of nitrite nitrogen that is loaded per nitrite-oxidizing bacterial cell per day: $L_{NOB}$ (fgNO$_2$-N/copy/day) is in the range of $1000 \leq L_{NOB} \leq 60{,}000$ by nitrite-oxidizing bacteria used in nitrite oxidation of treated water after water to be treated that rarely contains organic compounds and the like except nitrogen compounds such as ammonia and nitric acid (discharge water in which a substance to be treated is mainly a nitrogen component) is biologically treated with ammonia oxidizing bacteria, the function $g_{NOB}(x)$ in equation (17) is set to be equation (19):

[Eq. 33]

$$g_{NOB}(x) = \{a_3 \cdot x / (b_3 + x)\} + c_3 \quad \cdots \quad (19)$$

(wherein, $a_3 = 2.5 \times 10^4$, $b_3 = 9.5 \times 10^4$ and $C_3 = 1.7 \times 10^3$) and the function $h_{NOB}(x)$ in equation (18) is set to be equation (20):

[Eq. 34]

$$h_{NOB}(x) = \{a_4 \cdot x / (b_4 + x)\} + c_4 \quad \cdots \quad (20)$$

(wherein, $a_4 = 2.5 \times 10^4$, $b_4 = 9.5 \times 10^4$ and $c_4 = 2.2 \times 10^3$), and the maximum reaction rate of the oxidation reaction of nitrite nitrogen by the nitrite-oxidizing bacteria is defined by a function that is within these $g_{NOB}(x)$ and $h_{NOB}(x)$ and has as a variable the amount of nitrite nitrogen that is loaded per nitrite-oxidizing bacterial cell per day, whereby the prediction accuracy of the simulation may be improved further.

In other words, for example, the maximum reaction rate by the nitrite-oxidizing bacteria in the biological treatment process is defined by equation (21):

[Eq. 35]

$$V_{NOB} = (2.5 \times 10^4) \times L_{NOB} / \{(9.5 \times 10^4) + L_{NOB}\} \quad \cdots \quad (21)$$

wherein $V_{NOB}$: Maximum reaction rate (fg·copy$^{-1}$·day$^{-1}$) and $L_{NOB}$: Amount of nitrite nitrogen that is loaded per nitrite-oxidizing bacterial cell per day (fgNO$_2$-N·copy$^{-1}$·day$^{-1}$) and can be used as a parameter for the simulation.

In addition, in the case where nitrite oxidation reaction is predicted in nitrite-oxidizing bacteria as a whole used in the biological treatment of water to be treated that generally contains nitrogen components as substance to be treated, including sewage, the values of $a_3$, $b_3$ and $c_3$ in equation (19) are set to $2.5 \times 10^5$, $4.0 \times 10^5$ and $-1.0 \times 10^5$, respectively, and the values of $a_4$, $b_4$ and $c_4$ in equation (20) are set to $2.5 \times 10^5$, $4.0 \times 10^5$ and $1.0 \times 10^5$, respectively, so that predicted values with high precision can be obtained as compared with those in the conventional simulation.

Therefore, the function that relates $V_{NOB}$ and $L_{NOB}$ is preferably set such that the maximum reaction rate: $V_{NOB}$ (fg·copy$^{-1}$·day$^{-1}$) satisfies the relation:

$$\{2.5\times10^5\cdot L_{NOB}/(4.0\times10^5+L_{NOB})-1.0\times10^5\}\leq V_{NOB}\leq\{2.5\times10^5\cdot L_{NOB}/(4.0\times10^5+L_{NOB})+1.0\times10^5\}$$

in the case where the amount of nitrite nitrogen: $L_{NOB}$ (fg·copy$^{-1}$·day$^{-1}$) that is loaded per nitrite-oxidizing bacterial cell per day meets the relation of $1.0\times10^3\leq L_{NOB}\leq1.2\times10^6$.

According to this preferred aspect, the function is generally applicable to biological treatment of water to be treated in which nitrogen components are contained therein as the substance to be treated, including sewage as well.

Moreover, when chloride ions together with nitrogen components are contained in water to be treated, the chloride ion concentration also affects the maximum reaction rate.

Moreover, since nitrite-oxidizing bacteria tend to increase the maximum reaction rate of the nitrite oxidation reaction with increasing the chloride ion concentration, the value of the maximum reaction rate is used as a parameter in a state where the maximum reaction rate has a specified functional relation to the amount of nitrite nitrogen and also to the amount of chloride ions, in water to be treated, whereby the precision of the simulation can be further improved.

Therefore, the precision of the simulation can be further improved by incorporating equation (22) below in place of the model of equation (16) and using it.

[Eq. 36]

$$\frac{dS_{NO2}}{dt} = f_{NOB}(L_{NOB})k_{NOB}(D_{CL})\frac{S_{NO2}}{K_{NO2}+S_{NO2}}\frac{S_{O2}}{K_{O2}+S_{O2}}\frac{S_{ALK}}{K_{ALK}+S_{ALK}}X_{NOB} \quad \cdots \quad (22)$$

wherein,

$L_{NOB}$: Amount of nitrite nitrogen that is loaded to one nitrite-oxidizing bacterial cell per day (fgNO$_2$-N/copy/day)
$D_{CL}$: Chloride ion concentration in water to be treated that flows into nitrification process (mg/l)
$S_{O2}$: Dissolved oxygen concentration (gO$_2$/m$^3$)
$S_{NO2}$: Soluble nitrite concentration (gNO$_2$-N/m$^3$)
$S_{ALK}$: Alkalinity (mole HCO$_3$/m$^3$)
$K_{O2}$: Saturation coefficient for dissolved oxygen (g O$_2$/m$^3$)
$K_{O2}$: Saturation coefficient for nitrite (gNO$_2$-N/m$^3$)
$K_{ALK}$: Saturation coefficient for alkalinity (mole HCO$_3$/m$^3$)
$X_{NOB}$: Nitrite-oxidizing bacterial concentration in nitrification tank (copies/m$^3$)

In addition, it is described previously that biomass (g-COD$_{Cr}$) can be adopted for the simulation in place of the number of bacteria (copy).

Moreover, the maximum mass of a substance to be treated that is decomposed per nitrite-oxidizing bacterial cell per day (i.e., maximum reaction rate: $V_{NOB}$) is set to be a product ($V_{NOB}=f_{NOB}(L_{NOB})\cdot k_{NOB}(D_{CL})$) of a function ($f_{NOB}(L_{NOB})$) that has a variable of the amount of nitrite nitrogen that is loaded per nitrite-oxidizing bacterial cell per day and a function ($k_{NOB}(D_{CL})$) that has a variable of the chloride ion concentration, whereby the prediction accuracy of the quality of treated water can be improved as compared with the conventional simulation and the calibration operation load can be reduced.

This function ($k_{NOB}(D_{CL})$) having the variable of the chloride ion concentration is preferably set such that the chloride ion concentration in the water to be treated that flows into the nitrification process: $D_{CL}$(mg/l) satisfies $(1-8.7\times10^{-5}\cdot D_{CL})\leq k_{NOB}(DcL)\leq(1-4.0\times10^{-5}\cdot D_{CL})$ within the range of $D_{CL}\leq4.0\times10^3$.

(Denitrification process)

In the denitrification tank 4, since two processes of nitrite reduction in which nitrate nitrogen is reduced to nitrite nitrogen by nitrate-reducing bacteria and nitrite reduction in which the nitrite nitrogen formed by the nitrate-reducing bacteria is reduced to nitrogen by nitrite-reducing bacteria are carried out, parameters are set for each process.

(Nitrate reduction)

For the reduction reaction of nitrate nitrogen by nitrate-reducing bacteria in the denitrification tank 4, the maximum reaction rate of the reduction reaction of nitrate nitrogen by nitrate-reducing bacteria is defined by a function that is between the function $g_{NARB}(x)$ that increases $y_{NARB1}$ in equation (23):

$$[Eq. 37]$$
$$y_{NARB1} = g_{NARB}(x) \quad \cdots (23)$$

with increasing x and the function $h_{NARB}(x)$ that increases the value of $y_{NARB2}$ in equation (3):

$$[Eq. 38]$$
$$y_{NARB2} = h_{NARB}(x) \quad \cdots (24)$$

with increasing x and also satisfies $y_{NARB2} > y_{NARB1}$ when x is a variable (wherein x>0), whereby the prediction accuracy of the simulation may be improved further.

Typically, in the case where nitrate nitrogen is reduced when the amount of nitrate nitrogen that is loaded per nitrate-reducing bacterial cell per day: $L_{NARB}$(fgNO$_3$-N/copy/day) is in the range of $5.0 \leq L_{NAPB} \leq 70$ by nitrate-reducing bacteria used in nitrate reduction of treated water after water to be treated that rarely contains organic compounds and the like except nitrogen compounds such as ammonia and nitric acid (discharge water in which a substance to be treated is mainly a nitrogen component) is biologically treated (nitrification process) by ammonia oxidizing bacteria and nitrite-oxidizing bacteria, the function $g_{NARB}(x)$ in equation (23) is set to be equation (25):

$$[Eq. 39]$$
$$g_{NARB}(x) = \{a_5 \cdot x / (b_5 + x)\} + c_5 \quad \cdots (25)$$

(wherein, $a_5=1.0 \times 10^2$, $b_5=8.5 \times 10^2$ and $c_5=-2.0$) and the function $h_{NARB}(x)$ in equation (24) is set to be equation (26):

$$[Eq. 40]$$
$$h_{NARB}(x) = \{a_6 \cdot x / (b_6 + x)\} + c_6 \quad \cdots (26)$$

(wherein, $a_6=1.0 \times 10^2$, $b_6=8.5 \times 10^2$ and $C_6=1.8$), and the maximum reaction rate of the reduction reaction of nitrate nitrogen by the nitrate-reducing bacteria is defined by a function that is between these $g_{NAEB}(x)$ and $h_{NARB}(x)$ and has as a variable the amount of nitrate nitrogen that is loaded per nitrate-reducing bacterial cell per day, whereby the prediction accuracy of the simulation may be improved further.

In other words, for example, the maximum reaction rate by the nitrate-reducing bacteria in the biological treatment process is defined by equation (27):

$$[Eq. 41]$$
$$V_{NARB} = (1.0 \times 10^2) \times L_{NARB} / \{(8.5 \times 10^2) + L_{NARB}\} \quad \cdots (27)$$

wherein $V_{NARB}$: Maximum reaction rate (fg·copy$^{-1}$·day$^{-1}$) and $L_{NARB}$: Amount of nitrate nitrogen that is loaded per nitrate-reducing bacterial cell per day (fgNO$_3$-N·copy$^{-1}$·day$^{-1}$) and can be used as a parameter for the simulation.

In addition, in the case where nitrate reduction reaction is predicted in nitrate-reducing bacteria as a whole used in the biological treatment of water to be treated that generally contains nitrogen components as substance to be treated, including sewage, the values of $a_5$, $b_5$ and $c_5$ in equation (25) are set to $2.2\times10^2$, $7.0\times10^2$ and $-1.7\times10^2$, respectively, and the values of $a_6$, $b_6$ and $c_6$ in equation (26) are set to $2.2\times10^2$, $7.0\times10^2$ and 70, respectively, so that predicted values with high precision can be obtained as compared with those in the conventional simulation.

Therefore, the function that relates $V_{NARB}$ and $L_{NARB}$ is preferably set such that the maximum reaction rate: $V_{NARB}$ (fg·copy$^{-1}$·day$^{-1}$) satisfies the relation:

$$\{2.2\times10^2\cdot L_{NARB}/(7.0\times10^2+L_{NARB})-1.7\times10^2\}\leq V_{NARB}\leq\{2.2\times10^2\cdot L_{NARB}/(7.0\times10^2+L_{NARB})+70\}$$

in the case where the amount of nitrite nitrogen: $L_{NARB}$(fg·copy$^{-1}$,day$^{-1}$) that is loaded per nitrate-reducing bacterial cell per day meets the relation of $5.0\leq L_{NARB}\leq2,500$.

According to this preferred aspect, the function is generally applicable to biological treatment of water to be treated in which nitrogen components are contained therein as substance to be treated, including sewage as well.

Moreover, when chloride ions together with nitrogen components are contained in water to be treated, the chloride ion concentration also affects the maximum reaction rate.

Moreover, since nitrate-reducing bacteria tend to decrease the maximum reaction rate of the nitrate reduction reaction with increasing the chloride ion concentration, the value of the maximum reaction rate is used as a parameter in a state where the maximum reaction rate has a specified functional relation to the amount of nitrate nitrogen and also to the amount of chloride ions, in water to be treated, whereby the precision of the simulation can be further improved.

Thus, the maximum mass of a substance to be treated that is decomposed per nitrate-reducing bacterial cell per day (i.e., maximum reaction rate: $V_{NARB}$) is set to be a product ($V_{NARB}=f_{NARB}(L_{NARB})\cdot k_{NARB}(D_{CL})$) of a function ($f_{NARB}(L_{NARB})$) that has a variable of the amount of nitrite nitrogen that is loaded per nitrate-reducing bacterial cell per day and a function ($k_{NARB}(D_{CL})$) that has a variable of the chloride ion concentration, whereby the prediction accuracy of the quality of treated water can be improved as compared with the conventional simulation and the calibration operation load can be reduced.

This function ($k_{NARB}(D_{CL})$) having the variable of chloride ion concentration is preferably set such that the chloride ion concentration in the water to be treated that is flowed into the nitrification process: $D_{CL}$(mg/L) satisfies $(1-7.9\times10^{-5}\cdot D_{CL})\leq k_{NARB}(D_{CL})\leq(1-1.0\times10^{-5}\cdot D_{CL})$ within the range of $D_{CL}\leq4.0\times10^3$.

(Nitrite reduction)

For the reduction reaction of nitrite nitrogen by nitrite reducing bacteria in the denitrification tank 4, the maximum reaction rate of the reduction reaction of nitrite nitrogen or the like by nitrite reducing bacteria is defined by a function that is between the function $g_{NIRB}(x)$ that increases $y_{NIRB1}$ in equation (28):

[Eq. 42]

$$y_{NIRB1}=g_{NIRB}(x) \quad \cdot\cdot\cdot (28)$$

with increasing x and the function $h_{NIRB}(x)$ that increases the value of $y_{NIRB2}$ in equation (29):

[Eq. 43]

$$y_{NIRB2}=h_{NIRB}(x) \quad \cdot\cdot\cdot (29)$$

with increasing x and also satisfies $y_{NIRB2}>y_{NIRB1}$ when x is a variable (wherein x>0), whereby the prediction accuracy of the simulation may be improved further.

Typically, in the case where nitrite nitrogen is reduced when the amount of nitrite nitrogen that is loaded per nitrite-reducing bacterial cell per day: $L_{NIRB}$(fgNO$_2$-N/copy/day) is in the range of $5.0\leq L_{NIRB}\leq120$ by nitrite-reducing bacteria used in reduction of treated water after water to be treated that rarely contains organic compounds and the like except nitrogen compounds such as ammonia and nitric acid (discharge water in which a substance to be treated is mainly a nitrogen component) is biologically treated (nitrification process) by ammonia oxidizing bacteria and nitrite-oxidizing bacteria, the function $g_{NIRB}(x)$ in equation (28) is set to be equation (30):

[Eq. 44]

$$g_{NIRB}(x) = \{a_7 \cdot x / (b_7 + x)\} + c_7 \quad \cdots \quad (30)$$

(wherein, $a_7=6.0\times10^1$, $b_{7B}=3.5\times10^2$ and $c_7=-7.0$) and the function $h_{NIRB}(x)$ in equation (29) is set to be equation (31):

[Eq. 45]

$$h_{NIRB}(x) = \{a_8 \cdot x / (b_8 + x)\} + c_8 \quad \cdots \quad (31)$$

(wherein, $a_8=6.0\times10^1$, $b_8=3.5x10^2$ and $c_8=3.0$), and the maximum reaction rate of the reduction reaction of nitrite nitrogen by the nitrite-reducing bacteria is defined by a function that is between these $g_{NIRB}(x)$ and $h_{NIRB}(x)$ and has as a variable the nitrite nitrogen that is loaded per nitrite-reducing bacterial cell per day, whereby the prediction accuracy of the simulation may be improved further.

In other words, for example, the maximum reaction rate by the nitrite-reducing bacteria in the biological treatment process is defined by equation (32):

[Eq. 46]

$$V_{NIRB} = (6.0\times10) \times L_{NIRB} / \{(3.5\times10^2) + L_{NIRB}\} \quad \cdots \quad (32)$$

wherein $V_{NIRB}$: Maximum reaction rate ($fg \cdot copy^{-1} \cdot day^{-1}$) and $L_{NIRB}$: Amount of nitrite nitrogen that is loaded per nitrite-reducing bacterial cell per day ($fgNO_2-N \cdot copy^{-1} \cdot day^{-1}$) and can be used as a parameter for the simulation.

In addition, in the case where nitrate reduction reaction is predicted in nitrate-reducing bacteria as a whole used in the biological treatment of water to be treated that generally contains nitrogen components as substance to be treated, including sewage, the values of $a_7$, $b_7$ and $c_7$ in equation (30) are set to $7.0\times10^2$, $2.5\times10^3$ and $-2.5\times10^2$, respectively, and the values of $a_8$, $b_8$ and cs in equation (31) are set to $7.0\times10^2$, $2.5\times10^3$ and $2.5\times10^2$, respectively, so that predicted values with high precision can be obtained as compared with those in the conventional simulation.

Therefore, the function that relates $V_{NIRB}$ and $L_{NIRB}$ is preferably set such that the maximum reaction rate: $V_{NIRB}$ ($fg \cdot copy^{-1} \cdot day^{-1}$) satisfies the relation: $(7.0\times10^2 \cdot L_{NIRB}/(2.5\times10^3+L_{NIRB})-2.5\times10^2) \leq V_{NIRB} \leq \{7.0\times10^2 \cdot L_{NIRB}/(2.5 \cdot 10^3+L_{NIRB})+2.5\times10^2\}$ in the case where the amount of nitrite nitrogen: $L_{NIRB}$ ($fg \cdot copy^{-1} \cdot day^{-1}$) that is loaded per nitrite-reducing bacterial cell per day meets the relation of $5.0 \leq L_{NIRB} \leq 3.5\times10^3$.

According to this preferred aspect, the function is generally applicable to biological treatment of water to be treated in which nitrogen components are contained therein as substance to be treated, including sewage as well.

Moreover, when chloride ions together with nitrogen components are contained in water to be treated, the chloride ion concentration also affects the maximum reaction rate.

Moreover, since nitrite-reducing bacteria tend to decrease the maximum reaction rate of the nitrite reduction reaction with increasing the chloride ion concentration, the value of the maximum reaction rate is used as a parameter in a state where the maximum reaction rate has a specified functional relation to the amount of nitrite nitrogen and also to the amount of chloride ions, in water to be treated, whereby the precision of the simulation can be further improved.

Thus, the maximum mass of a substance to be treated that is decomposed by one nitrite reducing bacterial cell per day (i.e., maximum reaction rate: $V_{NIRB}$) is set to be a product ($V_{NIRB}=f_{NIRB}(L_{NIRB}) \cdot k_{NIRB}(D_{CL})$) of a function ($f_{NIRB}(L_{NIRB})$) that has a variable of the amount of nitrite nitrogen that is loaded per nitrite-reducing bacterial cell per day and a function ($k_{NIRB}(D_{CL})$) that has a variable of the chloride ion concentration, whereby the prediction accuracy of the quality of treated water can be improved as compared with the conventional simulation and the calibration operation load can be reduced.

This function ($k_{NIRB}(D_{CL})$) having the variable of chloride ion concentration is preferably set such that the chloride ion concentration in the water to be treated that is flowed into the nitrification process: $D_{CL}$ (mg/l) satisfies

$(1-6.0 \times 10^{-5} \cdot D_{CL}) \leq k_{NIRB}(D_{CL}) \leq (1-5.0 \times 10^{-6} \cdot D_{CL})$ within the range of $D_{CL} \leq 4.0 \times 10^3$.

In addition, as described above, nitrogen compounds such as ammonia and nitric acid are primarily substances to be treated, and for bacterial reaction in the biological treatment process of water to be treated that does not contains a large amount of other organic compounds, for example, in the case where, in the biological treatment process, the substance to be treated is ammonia nitrogen and the bacteria are ammonia-oxidizing bacteria, the maximum reaction rate is defined by a function that is between between the function $g_{AOB}(x)$ shown in equation (11) and the function $h_{AOB}(x)$ shown in equation (12) above, whereby a simulation with high precision can be usually performed.

Additionally, in the case where the substance to be treated is nitrite nitrogen and the bacteria are nitrite-oxidizing bacteria, the maximum reaction rate is defined by a function, for example, the function indicated in equation (21) above, that is between the function $g_{NOB}(x)$ shown in equation (19) and the function $h_{NOB}(x)$ shown in equation (20) and incorporated in a simulation apparatus, whereby a simulation with high precision can be usually performed.

Additionally, in the case where a substance to be treated is nitrate nitrogen and the bacteria are nitrate-reducing bacteria in the denitrification tank, the maximum reaction rate is defined by a function, for example, the function indicated in equation (27) above, that is between the function $g_{NARB}(x)$ shown in equation (25) and the function $h_{NARB}(x)$ shown in equation (26) above, whereby a simulation with high precision can be usually performed.

Moreover, in the case where a substance to be treated is nitrite nitrogen and the bacteria are nitrite-reducing bacteria in the denitrification tank, the maximum reaction rate is defined by a function, for example, the function indicated in equation (32) above, that is between the function $g_{NIRB}(x)$ shown in equation (30) and the function $h_{NIRB}(x)$ shown in equation (31) above, whereby a simulation with high precision can be usually performed.

Thus, when the biological treatment process has a nitrification process, the maximum reaction rates of ammonia oxidation and nitrite oxidation are defined by the function indicated in equation (13) and the function indicated in equation (21) and incorporated in the simulation apparatus, and when the biological treatment process further includes a denitrification process, the maximum reaction rates of ammonia oxidation, nitrite oxidation, nitrate reduction and nitrite reduction are defined by the function indicated in equation (27) and the function indicated in equation (32) in addition to the function indicated in equation (13) and the function indicated in equation (32) and incorporated in the simulation apparatus, whereby a simulation with higher precision can be carried out.

In addition, optionally, the relationship between the maximum reaction rate and the amount of a substance to be treated that is loaded per bacterial cell per day may be calculated by the method indicated below and incorporated into the simulation apparatus.

Additionally, by means of the method below, the amount of a substance to be treated that is loaded per bacterial cell per day may be calculated and also the amount of the substance to be treated that remains after the treatment may be calculated and then the amount of the substance to be treated that is treated per bacterial cell per day may be calculated to thereby incorporate the function indicating the relationship between the maximum reaction rate and the amount of the substance to be treated that is treated per bacterial cell per day into the simulation apparatus.

(Maximum reaction rate)

(Ammonia oxidation rate)

The rate of ammonia oxidation by bacteria included in activated sludge of the nitrification tank (nitrification sludge) is determined as follows (ammonia oxidation rate test).

In a 500-mL erlenmeyer flask is placed 390 mL of dilution water (a composition per litter: sodium hydrogencarbonate 240 mg, BOD-A solution (buffer solution (pH 7.2) in accordance with JIS K 0102 21) 1mL, BOD-B solution (magnesium sulfate solution in accordance with JIS K 0102 21) 1 mL, BOD-C solution (calcium chloride solution in accordance with JIS K 0102 21) 1 mL, BOD-D solution (ferric chloride solution in accordance with JIS K 0102 21) 1 mL, the balance: water), and then 10 mL of an aqueous ammonium chloride solution (1000 mg-N/L) is added thereto to prepare mixture A. Next, the mixture A in the 500-ml erlenmeyer flask is maintained at 30°C in a thermostat and aerated for 10 minutes or more with stirring to obtain solution A. Here, the pH of the solution A is measured.

500 mL of nitrification sludge is collected and is solid-liquid separated by a centrifuge. The supernatant is removed from the resulting product, and the resulting solution is dispersed in 50 mL of the dilution water with stirring. The total content of resulting product is adjusted to 100 mL with the dilution water, and a nitrification sludge sample is obtained.

Then, 100 ml of the resulting nitrification sludge sample is placed in the 500-mL flask and mixed with the solution A above. Simultaneously with mixing, 5 ml of the sample obtained by the mixing is sampled with a 5-mL syringe and is filtrated with a filter (available from Advantech, brand name: Glass Fiber Filter GF-75, pore size: 0.3 $\mu$m). In addition, until ammonia nitrogen derived from ammonium chloride is consumed (about two hours), sampling is carried out at regular intervals.

Additionally, immediately after mixing of the nitrification sludge sample with the solution A above, 30 mL of a sample is collected besides the sampling and its sludge concentration is determined in accordance with JIS K 0102 14.

After the ammonia nitrogen derived from the ammonium chloride was consumed, the pH of the remaining mixture and also the sludge concentration are determined.

The amount of ammonia nitrogen in a sample at the time of each sampling is measured by ion chromatography analysis (in accordance with JIS K 0102 42.5). The change in the amount of ammonia nitrogen for each unit time is calculated from the analytical results and can be made the maximum reaction rate of the ammonia oxidation.

(Nitrite oxidation rate)

On the other hand, the rate of nitrite oxidation by bacteria contained in nitrification sludge is measured (nitrite oxidation rate test) as follows.

390 mL of the dilution water is placed in a 500-mL erlenmeyer flask and 10 mL of an aqueous sodium nitrite solution (1000mg-N/L) was added thereto to prepare a mixture B. Next, the mixture B in the 500-ml Erlenmeyer flask was maintained at 30°C in a thermostat and aerated for 10 minutes or more with stirring to obtain a solution B. Here, the pH of the solution B was measured.

500 mL of nitrification sludge is collected and is solid-liquid separated by a centrifuge. The supernatant is removed from the resulting product, and the resulting solution is dispersed in 50 mL of the dilution water with stirring. The total content of the resulting product is adjusted to 100 mL with the dilution water, and a nitrification sludge sample is obtained.

Then, 100 ml of the resulting nitrification sludge sample is placed in the 500 m-L flask and mixed with the solution B above. Simultaneously with mixing, 5 ml of the sample obtained by the mixing is sampled with a 5-mL syringe and is filtrated with a filter (available from Advantech, brand name: Glass Fiber Filter GF-75, pore size: 0.3 $\mu$m). In addition, until nitrite nitrogen derived from sodium nitrite is consumed (about two hours), sampling is carried out at regular intervals.

Additionally, immediately after mixing of the nitrification sludge sample with the solution B above, 30 mL of a sample is collected besides the sampling and its sludge concentration is determined.

After nitrite nitrogen derived from sodium nitrite is consumed, the pH of the remaining mixture and also the sludge concentration are determined.

The amount of nitrite nitrogen in a sample at the time of each sampling is measured by ion chromatography analysis (in accordance with JIS K 0102 43.1.2). The change in the amount of nitrite nitrogen for each unit time is calculated from the analytical results and can be made the maximum reaction rate of the nitrite oxidation.

(Nitrate reduction rate)

The measurement of the rate of nitrate reduction by bacteria contained in activated sludge (denitrification sludge) in a denitrification tank (nitrate reduction rate test) is carried out as follows.

380 mL of the dilution water is placed in a 500-mL erlenmeyer flask and the whole is maintained in a thermostat at 30°C and 10 mL of an aqueous sodium nitrate solution (1000 mg-N/L) is added thereto with stirring and then 10 mL of an aqueous methanol solution (5000 mg methanol/L) is added thereto to prepare a mixture C. Then, nitrogen gas is introduced into the mixture C with a spray balloon and the mixture is degassed for 10 minutes. Here, the pH of the mixture C is measured. The 500-mL erlenmeyer flask is closed with a silicone stopper and nitrogen gas is introduced thereinto with stirring to remove the air in the gas phase within the 500-mL erlenmeyer flask. Thereafter, nitrogen gas is introduced into a 1-L Tedlar bag and the bag is put in the silicone stopper of the 500-mL erlenmeyer flask.

500 mL of denitrification sludge is collected and is solid-liquid separated by a centrifuge. The supernatant is removed from the resulting product, and the resulting solution is dispersed in 50 mL of the dilution water with stirring. The total content of the resulting product is adjusted to 100 mL with the dilution water, and a denitrification sludge sample is obtained.

Then, 100 ml of the resulting denitrification sludge sample is placed in the 500 mL flask and mixed with the solution C. Simultaneously with mixing, 5 ml of the sample obtained by the mixing is sampled with a 5-mL syringe and is filtrated with a filter (available from Advantech, brand name: Glass Fiber Filter GF-75, pore size: 0.3 $\mu$m). In addition, until nitrate nitrogen derived from sodium nitrate is consumed (about two hours), sampling is carried out at regular intervals.

Additionally, immediately after mixing of the denitrification sludge sample with the solution C above, 30 mL of a sample is collected besides the sampling and its sludge concentration is determined.

After nitrate nitrogen derived from sodium nitrate is consumed, the pH of the remaining mixture and also the sludge concentration are determined.

The amount of nitrate nitrogen in a sample at the time of each sampling is measured by ion chromatography analysis (in accordance with JIS K 0102 43.2.5). The change in the amount of nitrate nitrogen for each unit time is calculated from the analytical results and can be made the maximum reaction rate of the nitrate oxidation.

(Nitrite reduction rate)

On the other hand, the measurement of the rate of nitrite reduction by bacteria contained in activated sludge (denitrification sludge) in a denitrification tank (nitrite reduction rate test) is carried out as follows.

380 mL of the dilution water is placed in a 500-mL erlenmeyer flask and the whole is maintained in a thermostat at 30°C and 10 mL of an aqueous sodium nitrite solution (1000 mg-N/L) is added thereto with stirring and then 10 mL of an aqueous methanol solution (5000 mg methanol/L) is added thereto to prepare a mixture D. Then, nitrogen gas is introduced into the mixture D with a spray balloon and the mixture is degassed for 10 minutes. Here, the pH of the mixture D is measured. The 500-mL erlenmeyer flask is closed with a silicone stopper and nitrogen gas is introduced thereinto with stirring to remove the air in the gas phase within the 500-mL erlenmeyer flask. Thereafter, nitrogen gas is introduced into a 1-L Tedlar bag and the bag is put in the silicone stopper of the 500-mL erlenmeyer flask.

500 mL of denitrification sludge is collected and is solid-liquid separated by a centrifuge. The supernatant is removed from the resulting product, and the resulting solution is dispersed in 50 mL of the dilution water with stirring. The total content of the resulting product is adjusted to 100 mL with the dilution water, and a denitrification sludge sample is obtained.

Then, 100 ml of the resulting denitrification sludge sample is placed in the 500 mL flask and mixed with the mixture D. Simultaneously with mixing, 5 ml of the sample obtained by the mixing was sampled with a 5-mL syringe and was filtrated with a filter (available from Advantech, brand name: Glass Fiber Filter GF-75, pore size: 0.3 $\mu$m). In addition, until nitrite nitrogen derived from sodium nitrite is consumed (about two hours), sampling is carried out at regular intervals.

Additionally, immediately after mixing of the denitrification sludge sample with the mixture D, 30 mL of a sample is collected besides the sampling and its sludge concentration is determined.

After nitrite nitrogen derived from sodium nitrite is consumed, the pH of the remaining mixture and also the activated sludge concentration are determined.

The amount of nitrite nitrogen in a sample at the time of each sampling is measured by ion chromatography analysis (in accordance with JIS K 0102 43.1.2). The change in the amount of nitrite nitrogen for each unit time is calculated from the analytical results and can be made the maximum reaction rate of the nitrite oxidation.

(Amount of a substance to be treated that is loaded per bacterial cell per day)

The amount of the substance to be treated that is loaded per bacterial cell per day in the biological treatment process can be calculated by calculating the total amount of the amount of the substance to be treated that is introduced from the outside per unit time per unit volume into the biological treatment process and the amount of the substance to be treated that is produced per unit time per unit volume and the number of the bacteria per unit volume that is used in the biological treatment process.

Among these, the amount of the substance to be treated that is introduced per unit time per unit volume from the outside into the biological treatment process can be calculated by, for example, measuring the inflow of water to be treated and the concentration of the substance to be treated in the water to be treated and calculating the amount of the substance to be treated that flows into the nitrification tank, the denitrification tank, and the like per day and then dividing the amount by the volume of the nitrification tank, the denitrification tank, and the like In addition, the amount of the substance to be treated that is produced in the biological treatment process can be typically considered to be equivalent to the amount of the original substance of this substance to be treated that is introduced from the outside into the biological treatment process and the amount of production of nitrite nitrogen in the nitrification tank where ammonia-oxidizing bacteria are housed can be considered to be equivalent to the amount of ammonia nitrogen introduced from the outside into the nitrification tank. Additionally, the number of bacteria per unit volume can be calculated by analyzing the sludge of the nitrification tank, the denitrification tank, or the like to calculate the number of the bacteria and then dividing the number by the volume of the nitrification tank, the denitrification tank or the like.

Among these, the concentration of the substance to be treated in water to be treated can be determined, for example, by indophenol blue absorptiometry, neutralization titrimetry, ionic electrometry, ion chromatography, or the like, according to JIS K 0102, when the substance to be treated is ammonia nitrogen.

Moreover, nitrite nitrogen can be determined by absorptiometry, ion chromatography, and the like, according to JIS K 0102.

In addition, nitrate nitrogen can be determined by reduction distillation-absorptiometry, copper-cadmium column reduction absorptiometry, ion chromatography, and the like, according to JIS K 0102.

Additionally, the methods of determining the total nitrogen include, for example, the method of summation, the Kjeldahl method of nitrogen determination, the reduction-distillation Kjeldahl method, ultraviolet light absorptiometry, etc (See "Sewage Test Method", First Volume, 1997 Edition, JAPAN SEWAGE WORKS ASSOCIATION).

Moreover, the number of various bacteria included in activated sludge (nitrification or denitrification sludge) can be quantified, for example, by extraction of DNA derived from bacteria included in activated sludge (nitrification or denitrification sludge), and a variety of methods of detecting nucleic acids, representative by real time PCR, extracting

DNA derived from bacteria included in activated sludge (nitrification or denitrification sludge) is extracted by an approach used in extraction of DNA from soil, for example, a method of crushing bacteria in activated sludge by physical means (beads, and the like) and extracting DNA. For isolation of DNA, although not particularly limited, for instance, Fast DNA SPIN Kit for Soil (brand name, available from Qbiogene, Inc.), ISOIL for Beads Beating (brand name, available from NIPPON GENE CO., LTD.), and the like can be used. Specifically, for example, when ISOIL for Beads Beating (brand name, available from NIPPON GENE CO., LTD.) is used, DNA can be isolated from activated sludge as follows.

Activated sludge (nitrification or denitrification sludge) is collected from each reaction tank and is placed in a 2-mL microcentrifuge tube. In addition, 2 mL of activated sludge is collected when the solid concentration is from 1,500 to 2,000 mg/L, 1.5 mL of activated sludge is collected when the solid concentration is from 2,000 to 3,000 mg/L, 1 mL of activated sludge is collected when the solid concentration is from 3,000 to 5,000 mg/L, 0.7 mL of activated sludge is collected when the solid concentration is from 5,000 to 7,000 mg/L, and 0.5 mL of activated sludge is collected when the solid concentration is from 7,000 to 10,000 mg/L.

Thereafter, activated sludge placed in the microcentrifuge tube is subjected to centrifugal separation of 20,630xg (1,500 rpm) for two minutes at 4°C and 20,630xg (15,000 rpm) for 30 seconds at 4°C. The resulting activated sludge is then suspended in a 450 μL Lysis Solution BB (brand name: ISOIL for Beads Beating, available from .NIPPON GENE CO., LTD.) that has been heated to 65°C in advance.

Subsequently, the resulting suspension is transferred to a Beads Tube (attached to ISOIL for Beads Beating (brand name, available from NIPPON GENE CO., LTD.)). Additionally, the original tube is cleaned with 450b μL of Lysis Solution BB that has been heated to 65°C in advance, and then the suspension after the cleaning is transferred to the Beads Tube. To the suspension in the Beads Tube is added 50 μL of Lysis Solution 20S (attached to ISOIL for Beads Beating (brand name, NIPPON GENE CO., LTD.)) and mixed.

Thereafter, the resulting mixture is maintained at 65°C for 15 minutes and supplied to a bead crusher (brand name: Beads Beater (available from NIPPON GENE CO., LTD.)) and then subjected to bead beating at 3,000 rpm for 90 seconds. Then, the resulting product is maintained at 65°C for 40 minutes with mild stirring and centrifuged at 12,000xg at 20°C for one minute to recover about 660 μL of the supernatant to a 2-mL tube. To the supernatant is added 440 μL of Purification Solution (attached to ISOIL for Beads Beating (brand name, available from NIPPON GENE CO., LTD.)) and mixed. Subsequently, 600 μL of chloroform is added thereto and mildly stirred and then centrifuged at 12,000xg at 20°C for 15 minutes to recover 900 μL of the water phase to a 2-mL tube. To the resulting product is added an equivalent (900 μL) of Purification Solution (attached to ISOIL for Beads Beating (brand name, available from NIPPON GENE CO., LTD.)) and mixed. The resulting product is centrifuged and the resulting sediment is cleaned with Wash Solution. Then, to the resulting sediment are added 1 mL of 70 volume% ethanol and 2 μL of Ethachinmate (brand name, available from NIPPON GENE CO., LTD.) to carry out ethanol precipitation and obtain a precipitate of DNA. To the resulting precipitate of DNA are added 200 μL of a TE buffer solution (composition: 10 mM Tris-HCl (pH 8.0) and 0.1 mM EDTA) and the DNA is dissolved, thus to obtain a DNA sample for OCR.

Now, the number of the various bacteria included in the activated sludge can be determined by real time PCR. In this case, by using the sample of nucleic acid extracted from the activated sludge, a primer pair suitable to bacteria contained in the activated sludge and a probe, a reaction is carried out under PCR conditions (temperature, time and cycle) suitable to the amplification of the nucleic acid of bacteria that are a target for quantification, to determine the various bacteria and the numbers of the bacteria included in the activated sludge. Examples of the primer pairs for the determination of the number of ammonia-oxidizing bacteria include, for example, CTO 189fA/B, CTO 189fC, RT1r, and the like, and examples thereof for the determination of the number of Nitrospira that is a species of nitrite-oxidizing bacteria include NSR1113f, NSR1264r, and the like. In addition, examples of the probes for the determination of ammonia-oxidizing bacteria include TEMP1 and examples for the determination of the number of Nitrospira that is a species of nitrite-oxidizing bacteria include NSR1143Taq and the like.

Specifically, for example, for the number of bacteria of each of ammonia-oxidizing bacteria, nitrite-oxidizing bacteria and eubacteria, a primer/probe set of a primer pair of forward primers of CTO 189fA/B (GGAGRAAAGCAGGGGATCG (SEQ ID NO: 1)) and CTO 189fC (GGAGGAAAGTAGGGGATCG (SEQ ID NO: 2)) (e.g., a mixture of CTO 189fA/B and CTO 189fC (2:1)) and a reverse primer of RT1r (CGTCCTCTCAGACCARCTACTG (SEQ ID NO: 3)) and a probe of TMP1 (CAACTAGCTAATCAGRCATCRGCCGCTC (SEQ ID NO: 4)) for ammonia-oxidizing bacteria; and a primer/probe set of a primer pair of NSR1113f (CCTGCTTTCAGTTGCTACCG (SEQ ID NO: 5)) and NSR1264r (GTTTGCAGCGCTTTGTACCG (SEQ ID NO: 6)) and a probe of NSR1143Taq (AGCACTCTGAAAGGACTGCCCAGG (SEQ ID NO: 7)) for nitrite-oxidizing bacteria, can be used for determination using TaqMan assays.

Additionally, the respective probes include a base sequence in which 5' end is labeled with FAM (6-carboxyfluorescein) and a base sequence in which 3' end is labeled with TAMRA (6-carboxytetramethylrhodamine).

Moreover, the primer pairs for the determination of the number of the nitrate-reducing bacteria include narH50F, narHr3B, and the like.

Specifically, nitrate-reducing bacteria can be quantitated using narH50F (AARTGYATCGGYTGCCA (SEQ ID NO: 8)) as a forward primer and narHr3B (TCCCARKCCTTGGGRTAG(SEQ ID NO: 9)) as a reverse primer, by the SYBR green, or the like.

In addition, the primer pairs for the determination of the number of nitrite-reducing bacteria include nirK876, nirK1040, cd3aF, R3cd, and the like

Specifically, nitrite-reducing bacteria can be quantitated by the SYBR green method using a primer pair of nirK876 (ATYGGCGGVAYGGCGA (SEQ ID NO: 10)) as a forward primer and nirK1040 (GCCTCGATCAGRTTRTGGTT (SEQ ID NO: 11)) as a reverse primer, or cd3aF (GTSAACGTSAAGGARACSGG (SEQ ID NO: 12)) as a forward primer and R3cd (GASTTCGGRTGSGTCTTGA (SEQ ID NO: 13) as a reverse primer.

The real time PCR uses a calibration curve constructed by using the samples of concentrations of $10^7$, $10^6$, $10^5$, $10^4$, $10^3$ and $10^2$ copies/5 $\mu$L/reaction. Specifically, the calibration curve is constructed by amplifying nucleic acid corresponding to a target gene of real time PCR by PCR and then using a recombinant plasmid that has been cloned to a plasmid vector. For example, for nitrite-oxidizing bacteria, 16S rRNA gene specific to nitrite-oxidizing bacteria is amplified by using a primer pair of NSR1113f and NSR1264r, and the resulting product is cloned to a plasmid vector to obtain a recombinant plasmid. The resulting recombinant plasmid is used as a standard for calibration curve construction. The real time PCR uses a standard sample with a known concentration (number of genes) and DNA (1 ng or 10 ng) purified from the sample as templates. Typically, the real time PCR is carried out on 16S rRNA genes and genes coding enzymes associated with cleaning of water such as the 16S rRNA gene of ammonia-oxidizing bacteria (Appl. Environ. Microbiol., published in 2001, vol. 67, pp. 972 to 976), the 16S rRNA gene of Nitrospira, a species of nitrite-oxidizing bacteria, (Environ. Sci. Technol., published in 2003, vol. 37, pp. 343 to 351), the narH gene of nitrate oxidizing bacteria (System. Appl. Microbiol., published in 2000, vol. 23, pp. 47 to 57), the nirS (cytochrome cd1 type nitrite-reducing bacteria) gene of nitrite-reducing bacteria (FEMS Microbiology Ecology published in 2004, vol. 49, pp. 401 to 417], and the nirK (copper-containing nitrite-reducing enzyme) gene (J. Microbiol. Methods., published in 2004, vol. 59, pp. 327 to 335).

The Ct value (point at which a threshold intersects with an amplification curve of PCR) of each concentration of standard samples is calculated to construct a calibration curve from the relationship between the Ct value and the concentration. On the other hand, the Ct value is also calculated for the sample DNA and the number of copies of the gene per 1 ng or 10 ng of the sample DNA used in the real time PCR is calculated by applying the value to the calibration curve constructed from the standard sample. Finally, the number of bacteria per mL of the activated sludge is obtained from equation (33):

[Eq. 47].

$$\text{Number of bacteria} = [\text{copy number per ng (or 10 ng) of Sample DNA used in real time PCR}] \times [\text{Amount of DNA purified from 1 mL of activated sludge (ng)/Amount of DNA used in real time PCR: 1 ng (or 10 ng)}] ... (33)$$

In addition, the number of genes, which real time PCR targets, present in one bacterial cell varies depending on the kind of bacteria, so that a precise number of bacteria can be calculated by dividing the number of bacteria calculated from equation (33) by the number of genes.

The concentrations of the bacteria (ammonia-oxidizing bacteria, nitrite-oxidizing bacteria, nitrate-reducing bacteria, and nitrite-reducing bacteria) are calculated from the numbers of bacteria obtained by real time PCR on the basis of equation (34):

[Eq. 48]

$$\text{Bacteria concentration (mg-COD}_{cr}\text{/L)} = \text{Dried weight per bacterial cell (mg-dry weight/copy)} \times \text{Number of bacteria (copy/L)} \times \text{Conversion factor (mg-COD}_{cr}\text{/mg-dry weight)} ... (34)$$

Additionally, values such as measurements or literature data [example of literature data: 0.28 pg/cell, Appl. Environ. Microbiol (2002), 68, 245-253) are used for the dry weight per bacterial cell.

Moreover, the conversion factor can be calculated by dividing the amount of enzyme consumption of the bacteria (5x32) by the molecular weight (113) from formula (35).

$$C_5H_7O_2N+5O_2 \rightarrow 5CO_2+2H_2O+NH_3 \qquad (35)$$

In this case, the conversion factor is 1.416.

When this is converted into one cell, one cell (one bacterium)=0.28 pg$\times$1.416mg-$COD_{Cr}$/mg-SS=3.965$\times$10-10mg-$COD_{Cr}$=3.965$\times$10-13g-$COD_{Cr}$.

Typically, nitrogen compounds such as ammonia and nitric acid are a target for substances to be treated, and for the reaction of bacteria in the biological treatment process of water to be treated that does not contain a large amount of other organic compounds, the maximum reaction rate is defined by equations (13), (21), (27), and (32), above, and used as a parameter, thereby being capable of carrying out a simulation with higher precision. However, the method described makes it possible to calculate a function that further agrees with the actual biological treatment process and to incorporate it into a simulation apparatus, whereby a simulation with higher precision can be performed, and thus the calibration operation load can be still further reduced.

In addition, the entries that are incorporated into a simulation apparatus include, in addition to the ones described above, for example, dissolved oxygen of water to be treated (mg-$O_2$/L), inert soluble organic substances (mg-$COD_{Cr}$/L), easily decomposable organic substances (mg-$COD_{Cr}$/L), alkalinity (mole $HCO_3$/L), floating inert organic substances (mg-$COD_{Cr}$/L), slow decomposable organic substances (mg-$COD_{Cr}$/L), heterotrophic bacteria (mg-$COD_{Cr}$/L), intracellular storage organic substances of heterotrophic bacteria (mg-$COD_{Cr}$/L), suspended substances (mg-SS/1), and the like.

Additionally, in this simulation apparatus, the setting is properly changed and simulations in other biological treatments can be performed.

The implementation of biological treatment while carrying out simulation in such simulation apparatus can suppress the problem that it takes time to restore proper conditions because a liquid to be treated that unexpectedly contains a large amount of substance to be treated has flowed to the next step, or that the load related to biological treatment is increased more than required since excessive time has been spent for treatment, thereby enabling the biological treatment method to be well efficient.

Therefore, the load that is required for the operation of a biological treatment apparatus can be reduced, and the operation cost, and the like can also be reduced.

In addition, this simulation method and simulation apparatus, or a biological treatment method and biological treatment equipment, using these method and apparatus, can be expected to have the same effects as in nitrification and denitrification, which have been described in the present embodiment, not only on the processes of nitrification and denitrification, but also on the biological treatment process that uses bacteria having dehalogenation ability such as Dehalococcoides species when the substance to be treated is an organic halogen compound, on the biological treatment processes using sulphate-reducing bacteria, e.g., Desulfotomaculum, Desulfobacter, Desulfobacterium, and Desulfovicrio species, when the substances to be treated are sulfur compounds such as sulfuric acid.

EXAMPLE

(Experimental Example 1)

Ammonia and nitric acid were main substances to be treated, and additionally the correlation between the amount of the maximum reaction rate and the amount of a substance to be treated that is loaded per bacterial cell per day in the biological treatment process was examined for the sludge used in the biological treatment process of nitrification/denitrification for water to be treated that does not contain a large amount of organic compounds.

(Reaction rate of ammonia-oxidizing bacteria)

The reaction rate of ammonia-oxidizing bacteria was determined as follows.

In a 500-mL erlenmeyer flask was placed 390 mL of dilution water (a composition per liter: sodium hydrogencarbonate 240 mg, BOD-A solution (buffer solution (pH 7.2) in accordance with JIS K 0102 21) 1mL, BOD-B solution (magnesium sulfate solution in accordance with JIS K 0102 21) 1 mL, BOD-C solution (calcium chloride solution (in accordance with JIS K 0102 21, calcium chloride solution) 1 mL, BOD-D solution (ferric chloride solution in accordance with JIS K 0102 21) 1 mL, the balance: water), and then 10 mL of an aqueous ammonium chloride solution (1000 mg-N/L) was added thereto to prepare mixture A. Next, the mixture A in the 500-ml erlenmeyer flask was maintained at 30°C in a thermostat and aerated for 10 minutes or more with stirring to obtain solution A. Here, the pH of the solution A was measured.

500 mL of nitrification sludge was collected and was solid-liquid separated by a centrifuge. The supernatant was removed from the resulting product, and the resulting solution was dispersed in 50 mL of the dilution water with stirring. The total content of resulting product was adjusted to 100 mL with the dilution water, and a nitrification sludge sample was obtained.

Then, 100 ml of the resulting nitrification sludge sample was placed in the 500-mL flask and mixed with the solution A above. Simultaneously with mixing, 5 ml of the sample obtained by the mixing was sampled with a 5-mL syringe and was filtrated with a filter (available from Advantech, brand name: Glass Fiber Filter GF-75, pore size: 0.3 $\mu$m). In addition, until the ammonia nitrogen derived from the ammonium chloride was consumed (about two hours), sampling was carried out at regular intervals.

Additionally, immediately after mixing of the nitrification sludge sample with the solution A above, 30 mL of a sample was collected besides the sampling and its sludge concentration is determined in accordance with JIS K 0102 14.

After the ammonia nitrogen derived from the ammonium chloride was consumed, the pH of the remaining mixture and also the sludge concentration were determined.

The amount of ammonia nitrogen in a sample at the time of each sampling was measured by ion chromatography analysis (in accordance with JIS K 0102 42.5). The change in the amount of ammonia nitrogen for each unit time was calculated from the analytical results and this amount of change was made the maximum reaction rate of the ammonia oxidation.

About 50 samples having different sludge concentrations were measured by the measuring method and the correlation between the values of the maximum reaction rates and the amounts of substances to be treated that were loaded per ammonia-oxidizing bacterial cell per day was examined.
The results obtained were plotted in a graph in which the value of the maximum reaction rate was taken in the ordinate and the amount of substance to be treated that was loaded per ammonia-oxidizing bacterial cell per day in the abscissa. The results are shown in FIG. 2.

Fig. 2 also shows that the value of the maximum reaction rate is not constant and as the amount of substance to be treated that is loaded per ammonia-oxidizing bacterial cell per day increases, the value of the maximum reaction rate increases.
It is also shown that when the maximum reaction rate is set to be $V_{AOB}$ (fg·copy$^{-1}$·h$^{-1}$) and the amount of ammonia nitrogen that is loaded per bacterial cell per day is set to be $L_{AOB}$(fg NH4-N·copy$^{-1}$·day$^{-1}$), in the case where the amount of substance to be loaded per ammonia-oxidizing bacterial cell per day: $L_{AOB}$ is in the range of 100 to 4,000 (fg NH4-N·copy$^{-1}$·day$^{-1}$), $V_{AOB}$ and $L_{AOB}$ have relationship that well agrees with equation (36):

[Eq. 49]

$$V_{AOB} = (7.0 \times 10^2) \times L_{AOB} / \{(5.5 \times 10^3) + L_{AOB}\} \quad \cdots (36)$$

(Reaction rate of nitrite-oxidizing bacteria)

The reaction rate of nitrite-oxidizing bacteria is determined as follows.
390 mL of the dilution water was placed in a 500-mL erlenmeyer flask and 10 mL of an aqueous sodium nitrite solution (1000mg-N/L) was added thereto to prepare a mixture B. Next, the mixture B in the 500-ml Erlenmeyer flask is maintained at 30°C in a thermostat and aerated for 10 minutes or more with stirring to obtain the solution B. Here, the pH of the solution B was measured.

500 mL of nitrification sludge was collected and was solid-liquid separated by a centrifuge. The supernatant is removed from the resulting product, and the resulting solution was dispersed in 50 mL of the dilution water with stirring. The total content of resulting product was adjusted to 100 mL with the dilution water, and a nitrification sludge sample was obtained.

Then, 100 ml of the resulting nitrification sludge sample is placed in the 500-mL flask and mixed with the solution B above. Simultaneously with mixing, 5 ml of the sample obtained by the mixing was sampled with a 5-mL syringe and was filtrated with a filter (available from Advantech, brand name: Glass Fiber Filter GF-75, pore size: 0.3 $\mu$m). In addition, until the nitrite nitrogen derived from the sodium nitrite is consumed (about two hours), sampling was carried out at regular intervals.

Additionally, immediately after mixing of the nitrification sludge sample with the solution B above, 30 mL of a sample was collected besides the sampling and its sludge concentration was determined.

After the nitrite nitrogen derived from the sodium nitrite was consumed, the pH of the remaining mixture and also the sludge concentration were determined.

The amount of nitrite nitrogen in a sample at the time of each sampling was measured by ion chromatography analysis (in accordance with JIS K 0102 43.1.2). The change in the amount of nitrite nitrogen per unit time was calculated from the analytical results and this change was made the maximum reaction rate of the nitrite oxidation.

About 50 samples having different sludge concentrations were measured by the measuring method and the correlation between the values of the maximum reaction rates and the amounts of substances to be treated (ammonia nitrogen and nitrite nitrogen) that were loaded per nitrite-oxidizing bacterial cell per day in the biological treatment process was examined.

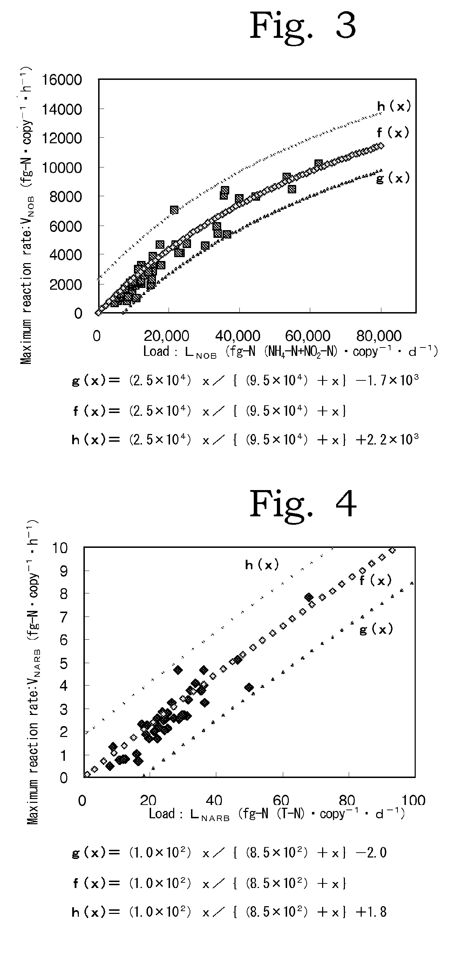

The results obtained were plotted in a graph in which the value of the maximum reaction rate is taken in the ordinate and the amount of substance to be treated that was loaded per nitrite-oxidizing bacterial cell per day in the abscissa. The results are shown in Fig. 3.

Fig. 3 also shows that the value of the maximum reaction rate is not constant and as the amount of substance to be treated that is loaded per nitrite-oxidizing bacterial cell per day increases, the value of the maximum reaction rate increases.

It is additionally shown that when the maximum reaction rate is set to be $V_{NOB}$(fg·copy$^{-1}$·h$^{-1}$) and the total amount of ammonia nitrogen and nitrite nitrogen that are loaded per nitrite-oxidizing bacterial cell per day is set to be $L_{NOB}$(fg-N(NH4-N + NO$_2$-N)·copy$^{-1}$·day$^{-1}$), in the case where the amount of substance to be loaded per nitrite-oxidizing bacterial cell per day: $L_{NOB}$ is in the range of 1,000 to 60,000 (fg·copy$^{-1}$·day$^{-1}$), $V_{NOB}$ and $L_{NOB}$ have relationship that well agrees with equation (37):

$$[\mathrm{Eq.\ 50}]$$

$$V_{NOB} = (2.5 \times 10^4) \times L_{NOB} / \{(9.5 \times 10^4) + L_{NOB}\} \quad \cdots (37)$$

Here, the reason why $L_{NOB}$ is set to be the total amount of ammonia nitrogen and nitrite nitrogen is that ammonia nitrogen is converted into nitrite nitrogen by ammonia-oxidizing bacteria in the nitrification tank and this total amount is typically loaded as nitrite nitrogen.

(Reaction rate of nitrate-reducing bacteria)

The rate of nitrate reduction by bacteria contained in denitrification sludge (nitrate reduction rate test) is determined as follows.

380 mL of the dilution water was placed in a 500-mL erlenmeyer flask and the whole was maintained in a thermostat at 30°C and 10 mL of an aqueous sodium nitrate solution (1000 mg-N/L) was added thereto with stirring and then 10 mL of an aqueous methanol solution (5000 mg methanol/L) was added thereto to prepare a mixture C. Then, nitrogen gas was introduced into the mixture C with a spray balloon and the mixture is degassed for 10 minutes. Here, the pH of the mixture C was measured. The 500-mL erlenmeyer flask is closed with a silicone stopper and nitrogen gas was introduced thereinto with stirring to remove the air in the gas phase within the 500-mL erlenmeyer flask. Thereafter, nitrogen gas was introduced into a 1-L Tedlar bag and the bag was put in the silicone stopper of the 500-mL erlenmever flask.

500 mL of denitrification sludge was collected and is solid-liquid separated by a centrifuge. The supernatant was removed from the resulting product, and the resulting solution was dispersed in 50 mL of the dilution water with stirring. The total content of the resulting product was adjusted to 100 mL with the dilution water, and a denitrification sludge sample was obtained.

Then, 100 ml of the resulting denitrification sludge sample was placed in the 500 mL flask and mixed with the solution C above. Simultaneously with mixing, 5 ml of the sample obtained by the mixing was sampled with a 5-mL syringe and was filtrated with a filter (available from Advantech, brand name: Glass Fiber Filter GF-75, pore size: 0.3 μm). In addition, until the nitrate nitrogen derived from the sodium nitrate was consumed (about two hours), sampling was carried out at regular intervals.

Additionally, immediately after mixing of the denitrification sludge sample with the solution C above, 30 mL of a sample was collected besides the sampling and its sludge concentration was determined.

After nitrate nitrogen derived from sodium nitrate was consumed, the pH of the remaining mixture and also the sludge concentration were determined.

The amount of nitrate nitrogen in a sample at the time of each sampling was measured by ion chromatography analysis (in accordance with JIS K 0102 43.2.5).

The change in the amount of nitrate nitrogen for each unit time is calculated from the analytical results and can be made the maximum reaction rate of the nitrate oxidation.

About 50 samples having different sludge concentrations were measured by the measuring method and the correlation between the values of the maximum reaction rates and the amounts of substances to be treated that were loaded per nitrate-reducing bacterial cell per day in the biological treatment process was examined.

The results obtained were plotted in a graph in which the value of the maximum reaction rate was taken in the ordinate and the amount of nitrate nitrogen that was loaded per nitrate-reducing bacterial cell per day in the abscissa.

The results are shown in Fig. 4.

Fig. 4 also shows that the value of the maximum reaction rate is not constant and as the amount of substance to be treated that is loaded per nitrate-reducing bacterial cell per day increases, the value of the maximum reaction rate increases.

It is further shown that when the maximum reaction rate is set to be $V_{NARB}$ (fg·copy$^{-1}$·h$^{-1}$) and the total amount of nitrogen that is loaded per nitrate-reducing bacterial cell per day is set to be $L_{NARB}$ (fgN·copy$^{-1}$·day$^{-1}$), in the case where the amount of substance to be treated that is loaded per nitrate-reducing bacterial cell per day: $L_{NARB}$ is in the range of 5 to 70 (fg·copy$^{-1}$·day$^{-1}$), $V_{NARB}$ and $L_{NARB}$ have relationship that well agrees with equation (38):

[Eq. 51]

$$V_{NARB} = (1.0 \times 10^2) \times L_{NARB} / \{(8.5 \times 10^2) + L_{NARB}\} \cdots (38)$$

Here, the calculation was executed in Examples, provided that the total nitrogen amount in water to be treated that flows in is substantially equal to the total amount of the amount of ammonia nitrogen, the amount of nitrite nitrogen and the amount of nitrate nitrogen, and that the amount of nitrogen that is loaded per nitrate-reducing bacterial cell is equal to the total nitrogen amount (T-N) in water to be treated since almost all of the ammonia nitrogen and nitrite nitrogen has been converted into nitrate nitrogen in the nitrification tank.

Tentatively, when the amount of the total nitrogen is not equal to the total amount of ammonia nitrogen and nitrite nitrogen and nitrate nitrogen, or ammonia nitrogen and nitrite nitrogen is not completely nitrified to nitrate nitrogen in the nitrification tank, the amount of the nitrate nitrogen that flows into the denitrification tank is desirably $L_{NARB}$.

(Reaction rate of nitrite-reducing bacteria)

The measurement of the rate of nitrite reduction by bacteria contained in denitrification sludge in a denitrification tank (nitrite reduction rate test) was carried out as follows.

380 mL of the dilution water was placed in a 500-mL erlenmeyer flask and the whole was maintained in a thermostat at 30°C and 10 mL of an aqueous sodium nitrite solution (1000 mg-N/L) was added thereto with stirring and then 10 mL of an aqueous methanol solution (5000 mg methanol/L) was added thereto to prepare a mixture D. Then, nitrogen gas is introduced into the mixture D with a spray balloon and the mixture was degassed for 10 minutes. Here, the pH of the mixture D was measured. The 500-mL erlenmeyer flask is closed with a silicone stopper and nitrogen gas was introduced thereinto with stirring to remove the air in the gas phase within the 500-mL erlenmeyer flask. Thereafter, nitrogen gas was introduced into a 1-L Tedlar bag and the bag was put in the silicone stopper of the 500-mL erlenmeyer flask.

500 mL of denitrification sludge was collected and was solid-liquid separated by a centrifuge. The supernatant was removed from the resulting product, and the resulting solution was dispersed in 50 mL of the dilution water with stirring. The total content of the resulting product was adjusted to 100 mL with the dilution water, and a denitrification sludge sample was obtained.

Then, 100 ml of the resulting denitrification sludge sample was placed in the 500 mL flask and mixed with the mixture D above. Simultaneously with mixing, 5 ml of the sample obtained by the mixing was sampled with a 5-mL syringe and was filtrated with a filter (available from Advantech, brand name: Glass Fiber Filter GF-75, pore size: 0.3μm). In addition, until the nitrite nitrogen derived from the sodium nitrite was consumed (about two hours), sampling was carried out at regular intervals.

Additionally, immediately after mixing of the denitrification sludge sample with the mixture D above, 30 mL of a sample was collected besides the sampling and its sludge concentration was determined.

After nitrite nitrogen derived from sodium nitrite was consumed, the pH of the remaining mixture and also the activated sludge concentration were determined.

The amount of nitrite nitrogen in a sample at the time of each sampling is measured by ion chromatography analysis (in accordance with JIS K 0102 43.1.2). The change in the amount of nitrite nitrogen for each unit time was calculated from the analytical results and this change was made the maximum reaction rate of the nitrite reduction.

About 50 samples having different sludge concentrations were measured by the measuring method and the correlation between the values of the maximum reaction rates and the amounts of substances to be treated that were loaded per nitrite-reducing bacterial cell per day in the biological treatment process was examined.

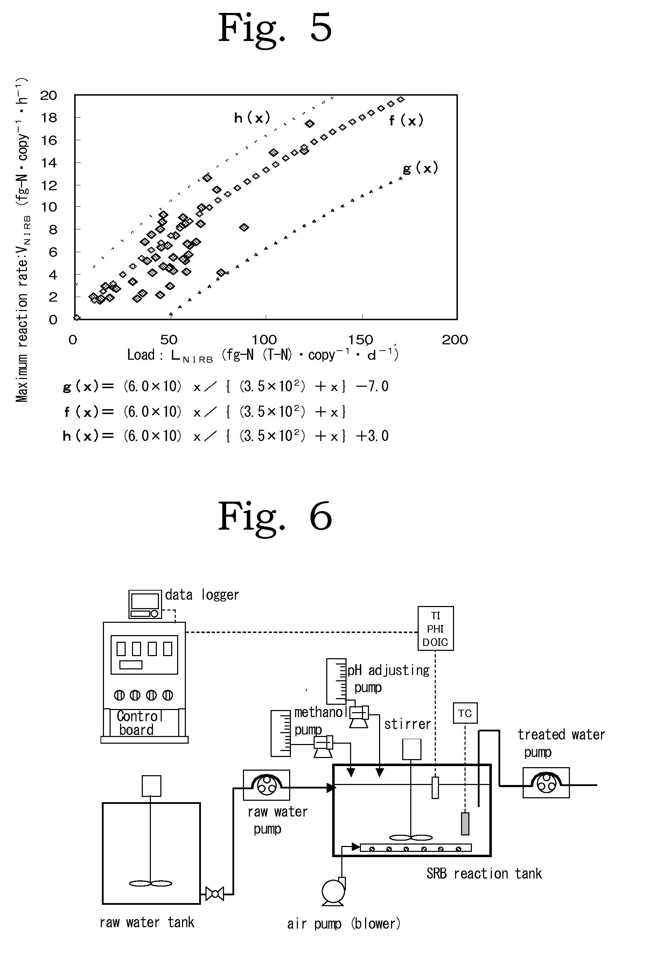

The results obtained were plotted in a graph in which the value of the maximum reaction rate was taken in the ordinate and the amount of substance to be treated that was loaded per nitrite-reducing bacterial cell per day in the abscissa. The results are shown in Fig. 5.

Fig. 5 also shows that the value of the maximum reaction rate is not constant and as the amount of substance to be treated that is loaded per nitrite-reducing bacterial cell per day increases, the value of the maximum reaction rate increases.

It is further shown that when the maximum reaction rate is set to be $V_{NIRB}$ (fg·copy$^{-1}$·h$^{-1}$) and the total amount of nitrogen that is loaded per nitrite-reducing bacterial cell per day is set to be $L_{NIRB}$ (fgN·copy$^{-1}$·day$^{-1}$), in the case where the amount of substance to be treated that is loaded per nitrite-reducing bacterial cell per day: $L_{NIRB}$ is in the range of 5 to 120 (fg·copy$^{-1}$·day$^{-1}$), $V_{NIRB}$ and $L_{NIRB}$ have relationship that well agrees with equation (39):

[Eq. 52]

$$V_{NIRB} = (6.0 \times 10) \times L_{NIRB} / \{(3.5 \times 10^2) + L_{NIRB}\} \cdots (39)$$

Here, the calculation of $L_{NIRB}$ was executed in the example, provided that the total nitrogen amount in water to be treated that flows in is substantially equal to the total amount of the amount of ammonia nitrogen, the amount of nitrite nitrogen and the amount of nitrate nitrogen, and that the amount of nitrogen that is loaded per nitrite reducing bacterial cell is equal to the total nitrogen amount (T-N) in water to be treated since almost, all of the ammonia nitrogen has been converted into nitrate nitrogen or nitrite nitrogen in the nitrification tank.

Tentatively, when the amount of the total nitrogen is not equal to the total amount of ammonia nitrogen and nitrite nitrogen and nitrate nitrogen, or ammonia is not completely nitrified in the nitrification tank, the total amount of the nitrate nitrogen and nitrite nitrogen that flows into the denitrification tank is desirably $L_{NIRB}$.

(Experimental Example 2)

The relationship between the maximum reaction rate and the amount of a substance to be treated that was loaded per bacterial cell per day was examined, with the load higher than that of Experimental Example 1 and the facilities imitating actual facilities.

(Facility)

The outline of an experimental facility used is shown in Fig. 6.

The experimental facility includes a reaction tank with a 35 liter effective volume, a raw water tank for adjusting water to be treated (raw water) that is biologically treated in the reaction tank, a raw water pump for feeding water to be treated from the raw water tank to the reaction tank, a treated water pump for discharging treated water from the reaction tank, an air pump (blower) for diffusion in the reaction tank, a methanol pump for feeding methanol to the reaction tank in a denitrification process, a pH adjusting pump for adjusting the pH of in-tank water in the reaction tank, and the like.

In addition, this experimental facility is configured such that the operation of each equipment is controlled by a timer, set value, and the like on a control board and operating records are recorded in a data logger.

(Setting of biological treatment process)

The cycle operation of a nitrification process, a denitrification process, an oxidation process and a precipitation process was carried out by switching the operating conditions of the reaction tank like (1) to (4) below.

In addition, the amount of introduction of raw water to the reaction tank was set to 14 L/cycle and 54 L/day.

(1) Nitrification process (two hours).

· The introduction of raw water into a reaction tank containing a specified volume of activated sludge-containing liquid is initiated and at the same time diffusion by a blower is conducted and the nitrification process is started.

· The blower is interlocked with the value of a dissolved oxygen analyzer (DO analyzer) placed in the reaction tank and made to turn ON/OFF automatically such that the dissolved oxygen concentration (DO) is within the range of 3.0±0.1 mg/L.

· To avoid a decrease in pH due to nitrification, the pH adjusting pump is interlocked with a pH meter placed in the reaction tank and 2% caustic soda is added by the pH adjusting pump so that the pH can be maintained at 7.7 or higher.

· The water temperature is measured by a temperature sensor in the reaction tank and the water is heated so that the water temperature can be maintained at about 30°C by a heater (the temperature maintenance in the denitrification and oxidation processes is also the same as for this reaction tank).

(2) Denitrification process (two hours)

· Diffusion by the blower is stopped, and the inside of the reaction tank is mixed by a stirrer.
· At the same time, the methanol adjusted to 5% is injected by the pump.

(3) Oxidation process (one hour)

· Diffusion by the blower is restarted at the same time as the initiation of the oxidation process, and the remaining methanol is treated.
· The DO is controlled at 3.0±0.1 mg/L as in the nitrification process.

(4) Precipitation process (one hour).

· The stirrer, blower, heater, as well as the addition of caustic soda are stopped and the inside of the reaction tank is left to stand at the completion of the oxidation process.
· A treated water-discharging pump is operated after 45 minutes and the treated water is discharged till a specified water level.

The whole process of (1) to (4) (6 hours/cycle) was carried out four cycles per day and a set load of nitrogen-containing discharge water was treated.

(Adjustment of water to be treated)

Water to be treated (raw water) that was supplied to the reaction tank was adjusted using the following reagents such that the components of the nitrogen and phosphorus were concentrations imitating the discharge water treatment equipment of a thermal power plant, and was supplied to the reaction tank while varying the nitrogen load and then the amount of nitrogen remaining in the treated water after the treatment was determined.

(Reagents used)

NH4-N: 30 mg/L (ammonium sulfate)
$NO_3$-N: 20 mg/L (sodium nitrate)
$PO_4$-P: 2 mg/L (sodium dihydrogenphosphate)
$NaHCO_3$: 360mg/L (sodium hydrogencarbonate)

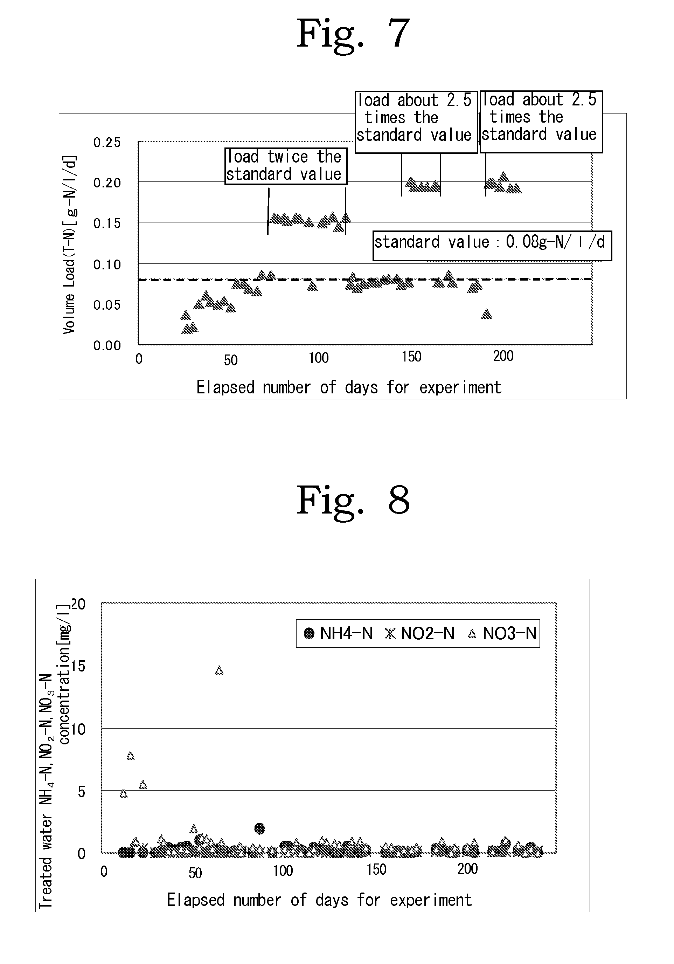

In addition, when the setting of the nitrogen load was increased, a concentrated substrate having a proportion similar to the raw water was prepared and was fed into the reaction tank in parallel to the raw water feeding.
The substrate concentration was adjusted so that the amount of introduction of a concentrated substrate became 1/50 or less of the amount of introduction of raw water, and attention was paid such that the hydraulic retention time in the reaction tank might hardly change even in an increase in load.
How to change the way of imparting the load to this reaction tank is indicated in Fig. 7.
The standard state of the volume load in the reaction tank was made 0.08g-N/L/d, and the load was changed relative to this standard load so as to temporarily become twice and 2.5 times the standard.
How to change the nitrogen component in the treated water discharged in the precipitation process by this load fluctuation was examined.
The results are shown in Fig. 8.
As shown in Fig. 8, the concentration of each form of nitrogen ($NH_4$-N, $NO_2$-N, $NO_3$-N) that remains in the treated water is 1 mg/L or less over the operation period, so that the substantially total amount of nitrogen made to flow into the reaction tank has been shown to be subjected to denitrification treatment.

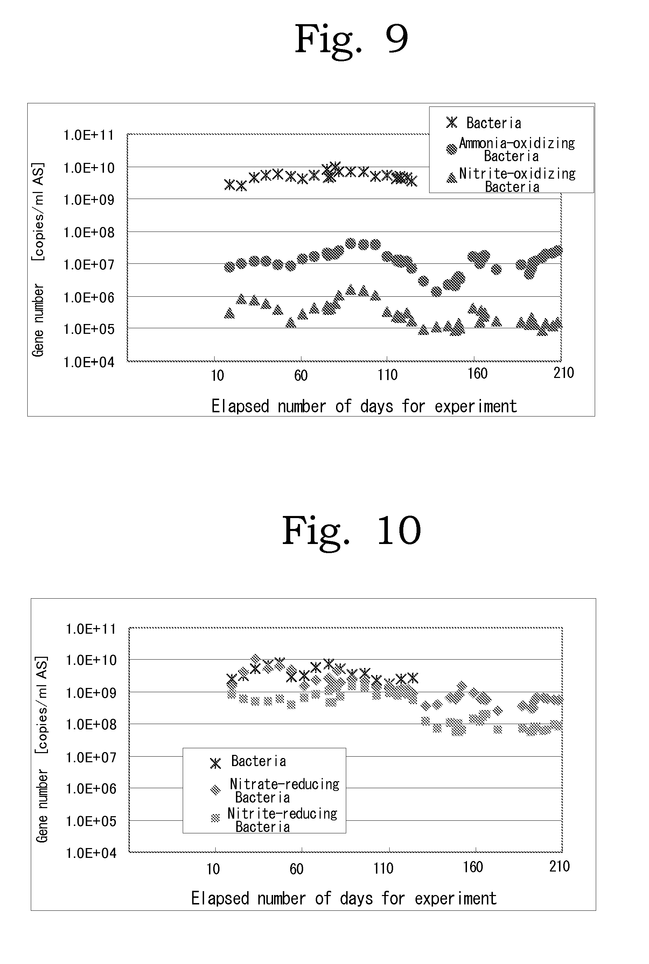

The results of the examination of changes in the number of bacteria in the activated sludge in the reaction tank for this period are shown in Figs. 9 and 10.

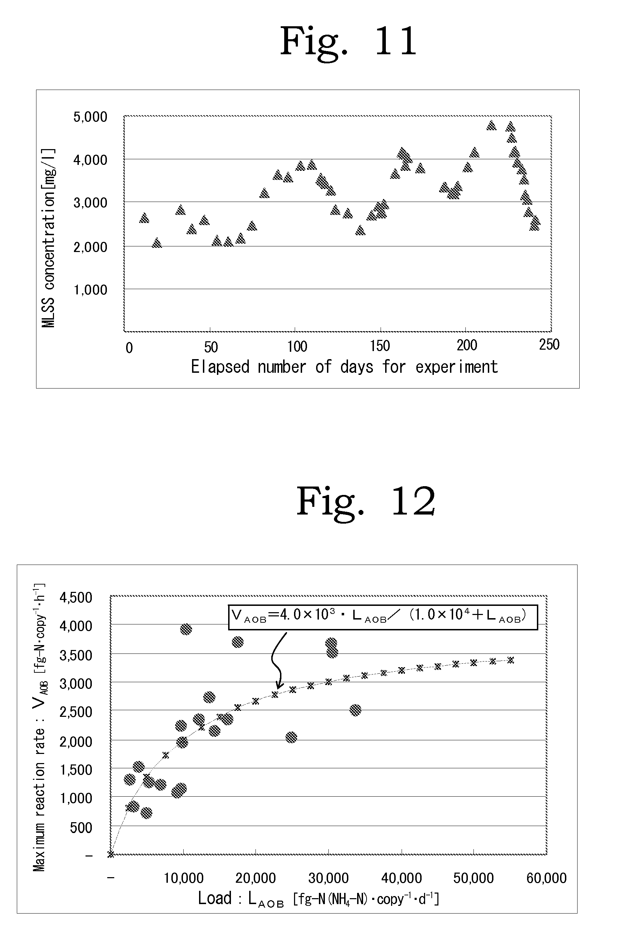

Moreover, the results of examining the variation conditions of the MLSS concentrations in the in-tank water are shown in Fig. 11.

These drawings show that the number of the respective bacteria largely varies in a range by about one order of magnitude during the operation period.

As compared with the fluctuation range of MLSS being in the range of 2,000 to 5,000 mg/L, the number of the bacteria included therein is shown to greatly vary.

The amount of nitrogen that was loaded per bacterial cell per day L (fg·copy$^{-1}$·day$^{-1}$) and the maximum reaction rate V (fg·copy$^{-1}$·h$^{-1}$) were calculated by dividing the number of bacteria by nitrogen load.

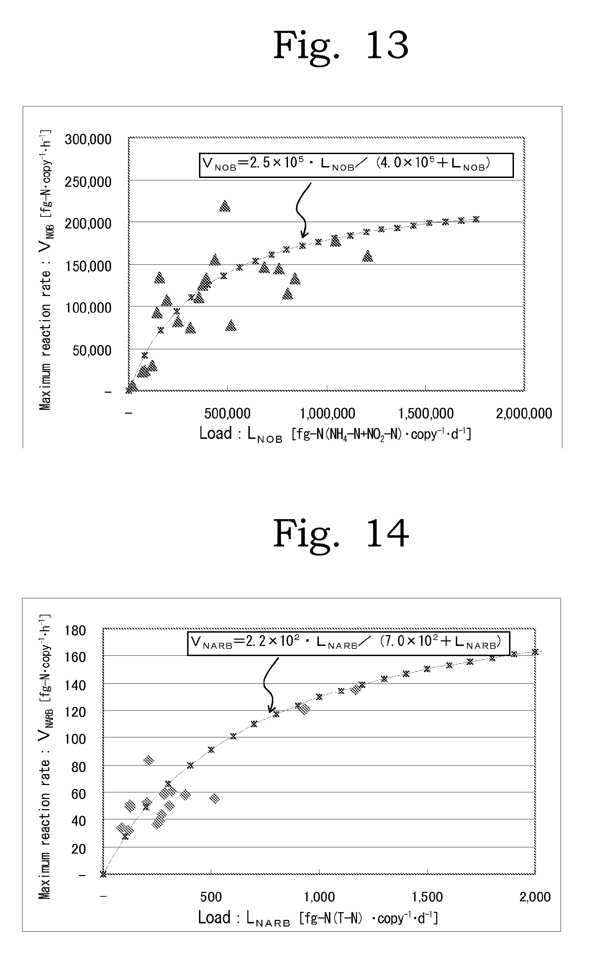

The results are indicated in Figs. 12 to 15. Figs. 12 to 15 show the results of the amounts of nitrogen that are loaded per bacterial cell per day: L(fg·copy$^{-1}$·day$^{-1}$) and the maximum reaction rates: V(fg·copy$^{-1}$·h$^{-1}$) for ammonia-oxidizing bacteria, nitrite-oxidizing bacteria, nitrate-reducing bacteria and nitrite-reducing bacteria, respectively.

Thus, it is shown that in the same manner as in Experimental Example 1, also in a high load, as the amount of a substance to be treated that is loaded per bacterial cell per day is increased, the value of the maximum reaction rate is increased. In addition, the pattern of its increase is shown to be in good agreement with a type of a function expressed by equation (40):

$$\{\text{Eq. 53}\}$$

$$V = a \times L / (b + L) + c \quad \cdots (40)$$

wherein, a, b and c are each independently a constant.

Therefore, the prediction accuracy can be improved by executing a simulation utilizing a function expressed by equation (40).

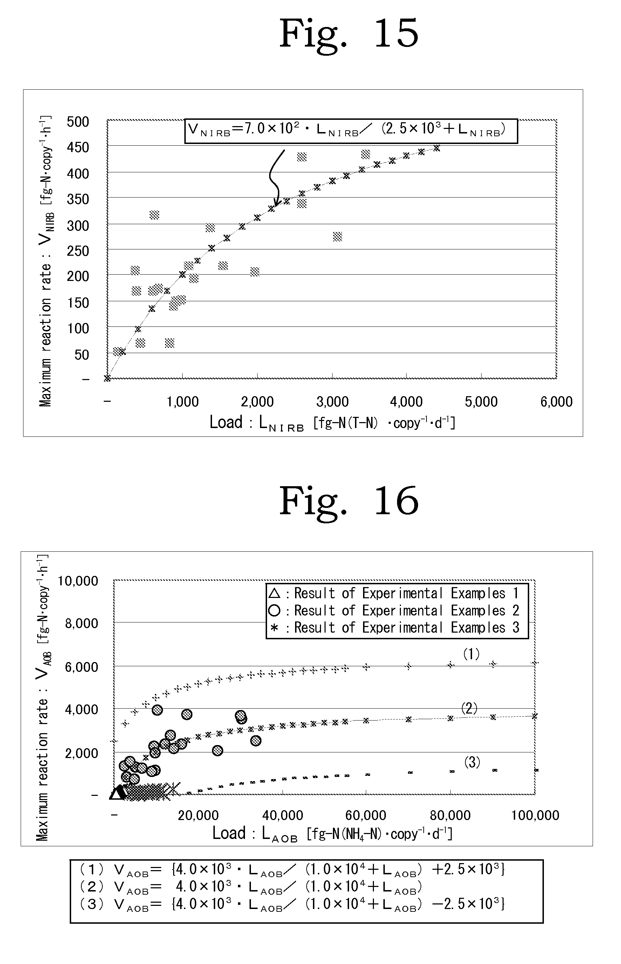

Additionally, the results of Experimental Example 1 (Figs. 2 to 5) and the results shown in Figs. 12 to 15 and additionally the results of a like experiment (Experimental Example 3) in which sludge is collected from a discharge water treatment tank that treats nitrogen-containing discharge water bearing organic compounds are illustrated in Figs. 16 to 19.

Fig. 16 also shows that the function that relates $V_{AOB}$ and $L_{AOB}$ is set such that the maximum reaction rate: $V_{AOB}$ (fg·copy$^{-1}$·h$^{-1}$) satisfies the relation: $\{4.0 \times 10^3 \cdot L_{AOB}/(1.0 \times 10^4 + L_{AOB}) - 2.5 \times 10^3\} V_{AOB} \leq \{4.0 \times 10^3 \cdot L_{AOB}/(1.0 \times 10^4 + L_{AOB}) + 2.5 \times 10^3\}$ in the case where the amount of ammonia nitrogen: $L_{AOB}$(fg·copy$^{-1}$·day$^{-1}$) that is loaded per ammonia-oxidizing bacterial cell per day satisfies the relation of $1.0 \times 10^2 \leq L_{AOB} \leq 3.5 \times 10^4$, thereby being capable of improving the prediction accuracy of the simulation.

It is also shown that the function can be applied to the entire biological treatment of water to be treated in which nitrogen components are contained therein as the substance to be treated, including sewage as well.

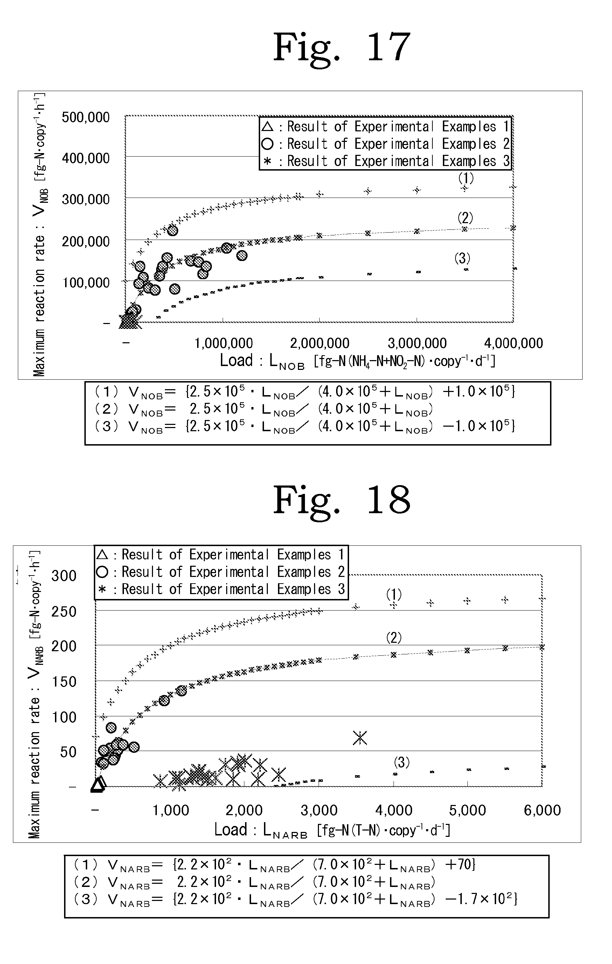

Moreover, Fig. 17 also shows that the function that relates $V_{NOB}$ and $L_{NOB}$ is set such that the maximum reaction rate: $V_{NOB}$(fg·copy$^{-1}$·h$^{-1}$) satisfies the relation: $\{2.5 \times 10^5 \cdot L_{NOB}/(4.0 \times 10^5 + L_{NOB}) - 1.0 \times 10^5\} \leq V_{NOB} \leq \{2.5 \times 10^5 \cdot L_{NOB}/(4.0 \times 10^5 + L_{NOB}) + 1.0 \times 10^5\}$ in the case where the amount of nitrite nitrogen: $L_{NOB}$(fg·copy$^{-1}$·day$^{-1}$) that is loaded per nitrite-oxidizing bacterial cell per day can satisfy the relation of $1.0 \times 10^3 \leq L_{NOB} \leq 1.2 \times 10^6$, thereby improving the prediction accuracy of the simulation.

It is also shown that the function can be applied to the entire biological treatment of water to be treated in which nitrogen components are contained therein as the substance to be treated, including sewage as well.

Fig. 18 also shows that the function that relates $V_{NARB}$ and $L_{NARB}$ is set such that the maximum reaction rate: $V_{NARB}$(fg·copy$^{-1}$·h$^{-1}$) satisfies the relation: $\{2.2 \times 10^2 \cdot L_{NARB}/(7.0 \times 10^2 + L_{NARB}) - 1.7 \times 10^2\} \leq V_{NARB} \leq \{2.2 \times 10^2 \cdot L_{NARB}/(7.0 \times 10^2 + L_{NARB}) + 70\}$ in the case where the amount of nitrite nitrogen: $L_{NARB}$(fg·copy$^{-1}$·day$^{-1}$) that is loaded per nitrate-reducing bacterial cell per day meets the relation of $5.0 \leq L_{NARB} \leq 2,500$, thereby being capable of improving the prediction accuracy of the simulation.

It is also shown that the function can be applied to the entire biological treatment of water to be treated in which nitrogen components are contained therein as the substance to be treated, including sewage as well.

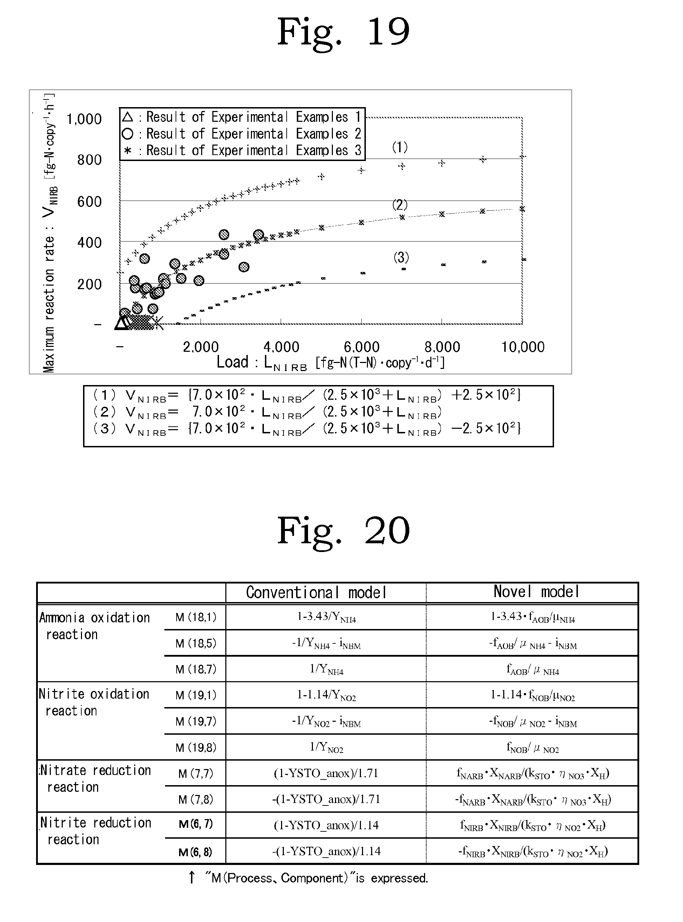

Further, Fig. 19 the function that relates $V_{NIRB}$ and $L_{NIRB}$ is set such that the maximum reaction rate: $V_{NIRB}$ (fg·copy$^{-1}$·h$^{-1}$) satisfies the relation: $\{7.0 \times 10^2 \cdot L_{NIRB}/(2.5 \times 10^3 + L_{NIRB}) - 2.5 \times 10^2\} \leq V_{NIRB} \leq \{7.0 \times 10^2 \cdot L_{NIRB}/(2.5 \times 10^3 + L_{NIRB}) + 2.5 \times 10^2\}$ in the case where the amount of nitrite nitrogen: $L_{NIRB}$ (fg·copy$^{-1}$·day$^{-1}$) that is loaded per nitrite-reducing bacterial cell per day meets the relation of $5.0 \leq L_{NIRB} \leq 3.5 \times 10^3$, thereby being capable of improving the prediction accuracy of the simulation.

It is also shown that the function can be applied to the entire biological treatment of water to be treated in which nitrogen components are contained therein as the substance to be treated, including sewage as well.

That is to say, when a nitrification process is included in the biological treatment process, the maximum reaction rate of ammonia oxidation is defined by a function expressed by equation (41):

[Eq. 54]

$$V_{AOB} = \{4.0 \times 10^3 \cdot L_{AOB} / (1.0 \times 10^4 + L_{AOB})\} \quad \cdots (41)$$

wherein $V_{NOB}$: Maximum reaction rate (fg·copy$^{-1}$·h$^{-1}$), $L_{NOB}$: Amount of nitrite nitrogen that is loaded per nitrite-oxidizing bacterial cell per day (fg NH$_4$-N·copy$^{-1}$·day$^{-1}$), and the maximum reaction rate of nitrite oxidation is defined by a function expressed by equation (42):

[Eq. 55]

$$V_{NOB} = \{2.5 \times 10^5 \cdot L_{NOB} / (4.0 \times 10^5 + L_{NOB})\} \quad \cdots (42)$$

wherein, $V_{AOB}$: Maximum reaction rate (fg·copy$^{-1}$·h$^{-1}$), $L_{AOB}$: Amount of ammonia nitrogen that is loaded per ammonia-oxidizing bacterial cell per day (fg NO$_2$-N·copy$^{-1}$·day$^{-1}$), which are incorporated into a simulation apparatus, and where a denitrification process is further included in the biological treatment process, in addition to functions expressed by equations (41) and (42) above, the maximum reaction rate of nitrate reduction is defined by a function expressed by equation (43):

[Eq. 56]

$$V_{NARB} = \{2.2 \times 10^2 \cdot L_{NARB} / (7.0 \times 10^2 + L_{NARB})\} \quad \cdots (43)$$

wherein, $V_{NARB}$: Maximum reaction rate (fg·copy$^{-1}$·h$^{-1}$), $L_{NARB}$: Amount of nitrate nitrogen that is loaded per nitrate-reducing bacterial cell per day (fg NO$_3$-N·copy$^{-1}$·day$^{-1}$), and the maximum reaction rate of nitrite reduction is defined by a function expressed by equation (44):

[Eq. 57]

$$V_{NIRB} = \{7.0 \times 10^2 \cdot L_{NIRB} / (2.5 \times 10^3 + L_{NIRB})\} \quad \cdots (44)$$

wherein, $V_{NIRB}$: Maximum reaction rate (fg·copy$^{-1}$·h$^{-1}$), $L_{NIRB}$: Amount of nitrite nitrogen that is loaded per nitrite-reducing bacterial cell per day (fg NO$_2$-N·copy$^{-1}$·day$^{-1}$), which are incorporated into the simulation apparatus, thereby being capable of executing a simulation with high precision.

On the basis of these results, the relational expressions of the maximum reaction rate that is varied depending on the amount of nitrogen that is loaded per bacterial cell per day indicated in equations (41) to (44) above were incorporated in place of the maximum reaction rate related to the nitrification and denitrification processes of the ASM3 (Activated Sludge Model No. 3) calculation program of the conventional IWA, and the activated sludge model (novel model) of the present invention was demonstrated.

Peterson's matrix for the ASM calculation based on this novel model is illustrated in Table 1 (only a part).

# Table 1

| Component | SO2 | SI | SA | SF | SNH4 | SN2 | SNO2 | SNO3 | SPO4 | SALK | XI | XS | XH | XSTO | XPAD | XPP | XPHA | XNH4 | XNO2 | XTSS |
|---|---|---|---|---|---|---|---|---|---|---|---|---|---|---|---|---|---|---|---|---|
| Process | 1 | 2 | 3 | 4 | 5 | 6 | 7 | 8 | 9 | 10 | 11 | 12 | 13 | 14 | 15 | 16 | 17 | 18 | 19 | 20 |
| r 1 Hydrolysis of XS | | | | | | | 0 | 0 | | | | | | | | | | 0 | 0 | |
| r 2 Storage of SA by XH under aerobic conditions | | | | | | | 0 | 0 | | | | | | | | | | 0 | 0 | |
| r 3 Storage of SF by XH under aerobic conditions | | | | | | | 0 | 0 | | | | | | | | | | 0 | 0 | |
| r 4 Storage of SA by XH using $NO_2$ under anaerobic conditions | | | | | | -c4no2 | -(1-YSTO_anox)/1.14 | 0 | | | | | | | | | | 0 | 0 | |
| r 5 Storage of SA by XH using $NO_3$ under anaerobic conditions | | | | | | 0 | (1-YSTO_anox)/1.71 | -(1-YSTO_anox)/1.71 | | | | | | | | | | 0 | 0 | |
| r 6 Storage of SF by XH using $NO_2$ under anaerobic conditions | | | | | | -c6no2 | M(6.7) | M(6.8) | | | | | | | | | | 0 | 0 | |
| r 7 Storage of SF by XH using $NO_3$ under anaerobic conditions | | | | | | 0 | M(7.7) | M(7.8) | | | | | | | | | | 0 | 0 | |
| r 8 Proliferation of XH using XSTO under aerobic conditions | | | | | | | 0 | 0 | | | | | | | | | | 0 | 0 | |
| r 9 Proliferation of XH using $NO_2$ and XSTO under anaerobic conditions | | | | | | -c9no2 | -(1-YH_anox)/1.14YH_anox | 0 | | | | | | | | | | 0 | 0 | |
| r 10 Proliferation of XH using $NO_3$ and XSTO under anaerobic conditions | | | | | | 0 | (1-YH_anox)/1.71YH_anox | | | | | | | | | | | 0 | 0 | |
| r 11 XH decomposition by endogenous respiration under aerobic conditions | | | | | | | 0 | 0 | | | | | | | | | | 0 | 0 | |
| r 12 XH decomposition by endogenous respiration using $NO_2$ under anaerobic conditions | | | | | | -c12no2 | (fXI-1)/1.14 | 0 | | | | | | | | | | 0 | 0 | |
| r 13 XH decomposition by endogenous respiration using $NO_3$ under anaerobic conditions | | | | | | 0 | -(fXI-1)/1.71 | (fXI-1)/1.71 | | | | | | | | | | 0 | 0 | |
| r 14 XSTO decomposition by endogenous respiration under aerobic conditions | | | | | | | 0 | 0 | | | | | | | | | | 0 | 0 | |
| r 15 XSTO decomposition by | | | | | | -c15no2 | -1/1.14 | 0 | | | | | | | | | | 0 | 0 | |

EP 2 181 966 A1

| | | | | | | | | | | | | | | | | | | |
|---|---|---|---|---|---|---|---|---|---|---|---|---|---|---|---|---|---|---|
| | | endogenous respiration under anaerobic conditions | | | | | | | | | | | | | | | | |
| r | 16 | XSTO decomposition by endogenous respiration under anaerobic conditions | | | | 0 | 1/1.71 | -1/1.71 | | | | | | | | | 0 | 0 |
| r | 17 | Fermentation reaction (SF→SA) | | | | | 0 | 0 | | | | | | | | | 0 | 0 |
| r | 18 | Proliferation of XNH4 under aerobic conditions (ammonia oxidation) | M(18.1) | 0 | 0 | 0 | M(18.5) | 0 | M(18.7) | 0 | -1*iPBM | 0 | 0 | 0 | 0 | 0 | 0 | 0 | 1 | 0 |
| r | 19 | Proliferation of XNO2 under aerobic conditions (nitrite oxidation) | M(19.1) | 0 | 0 | 0 | -iNBM | 0 | M(19.7) | M(19.8) | -1*iPBM | 0 | 0 | 0 | 0 | 0 | 0 | 0 | 0 | 1 |
| r | 20 | XNH4 decomposition by endogenous respiration under aerobic conditions | fX1-1 | 0 | 0 | 0 | iNBM-iNXI*fXI | 0 | 0 | 0 | iPBM-iPXI*fXI | fXI | 0 | 0 | 0 | 0 | 0 | 0 | -1 | 0 |
| r | 21 | XNO2 decomposition by endogenous respiration under aerobic conditions | fXI-1 | 0 | 0 | 0 | iNBM-iNXI*fXI | 0 | 0 | 0 | iPBM-iPXI*fXI | fXI | 0 | 0 | 0 | 0 | 0 | 0 | 0 | -1 |
| r | 22 | XNH4 decomposition by endogenous respiration using NO₂ under anaerobic conditions | 0 | 0 | 0 | 0 | iNBM-iNXI*fXI | -c22no2_NH4 | (fXI-1)/1.14 | | iPBM-iPXI*fXI | fXI | 0 | 0 | 0 | 0 | 0 | 0 | -1 | 0 |
| r | 23 | XNH4 decomposition by endogenous respiration using NO₃ under anaerobic conditions | 0 | 0 | 0 | 0 | iNBM-iNXI*fXI | 0 | -(fXI-1)/1.71 | (fXI-1)/1.71 | iPBM-iPXI*fXI | fXI | 0 | 0 | 0 | 0 | 0 | 0 | -1 | 0 |
| r | 24 | XNO2 decomposition by endogenous respiration using NO₂ under anaerobic conditions | 0 | 0 | 0 | 0 | iNBM-iNXI*fXI | -c24no2_NO2 | (fXI-1)/1.14 | | iPBM-iPXI*fXI | fXI | 0 | 0 | 0 | 0 | 0 | 0 | 0 | -1 |
| r | 25 | XNO2 decomposition by endogenous respiration using NO₃ under anaerobic conditions | 0 | 0 | 0 | 0 | iNBM-iNXI*fXI | 0 | -(fXI-1)/1.71 | (fXI-1)/1.71 | iPBM-iPXI*fXI | fXI | 0 | 0 | 0 | 0 | 0 | 0 | 0 | -1 |

This matrix differs from the conventional model in which the maximum reaction rate is constant as indicated in Comparative Table in Fig. 20.

The quality of treated water immediately after the nitrogen load was increased by 2.5 times the standard load and the quality of treated water after 48 hours that is considered to be sufficiently affected by the history of the amount of treatment after an increase in load were simulated and compared with actual measurement data.

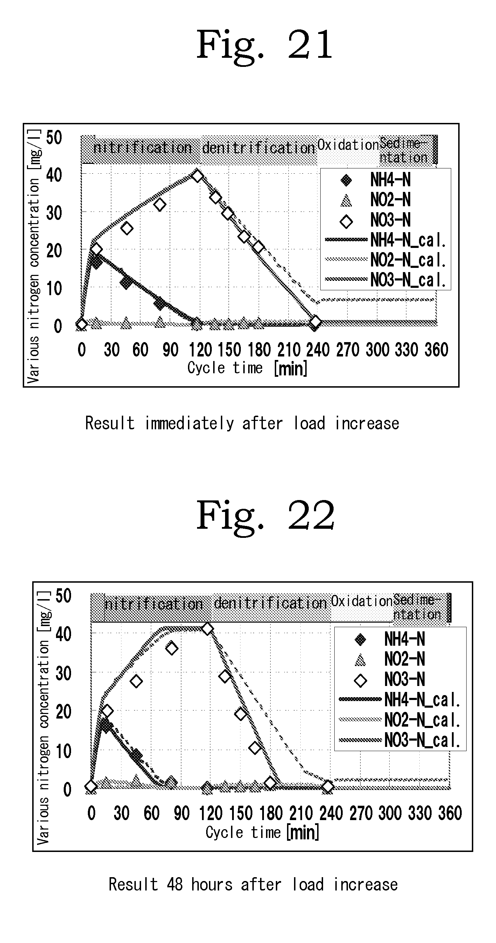

The results are illustrated in Figs. 21 (immediately after load increase) and 22 (48 hours after load increase). The plots in the drawings indicate the analysis values, and the solid lines and broken lines illustrate the calculated results obtained by the simulation (novel model) into which the relational expression of the maximum reaction rate that is varied depending on the amount of nitrogen that is loaded per bacterial cell per day and the results obtained by ASM3 of conventional calculation, respectively.

For the water quality analysis values of the experiments, the consumption of ammonia required about 120 minutes in the nitrification process after load increase and the consumption of nitric acid also required about 120 minutes in the denitrification process; however, the ammonia was consumed in about 80 minutes and the nitric acid in about 60 minutes 48 hours after load increase, whereby the improvement in treatment rate was confirmed.

The generation of the nitrous acid was hardly identified including nitrification and denitrification.

The calculation results by the simulation of a novel model was able to well reproduce the calculation results immediately after load increase and 48 hours after load increase and also the prediction accuracy of water quality achieved the analysis value $\pm 20\%$ that is the target value.

On the other hand, for the conventional calculation results of ASM3, the improvement of the treatment rate for 48 hours in the nitrification process was reproduced due to the growth of the nitrification bacteria; however, the heterotrophic microorganisms are only proliferated in the denitrification process and the improvement in treatment rate was not reproduced, and therefore the change of activity was not considered to be reproduced.

As described above, it is understood that according to the present invention, the prediction accuracy of the simulation can be improved.

(Experimental Example 4)

(Application example of the amount of substance to be treated that was treated per bacterial cell per day)

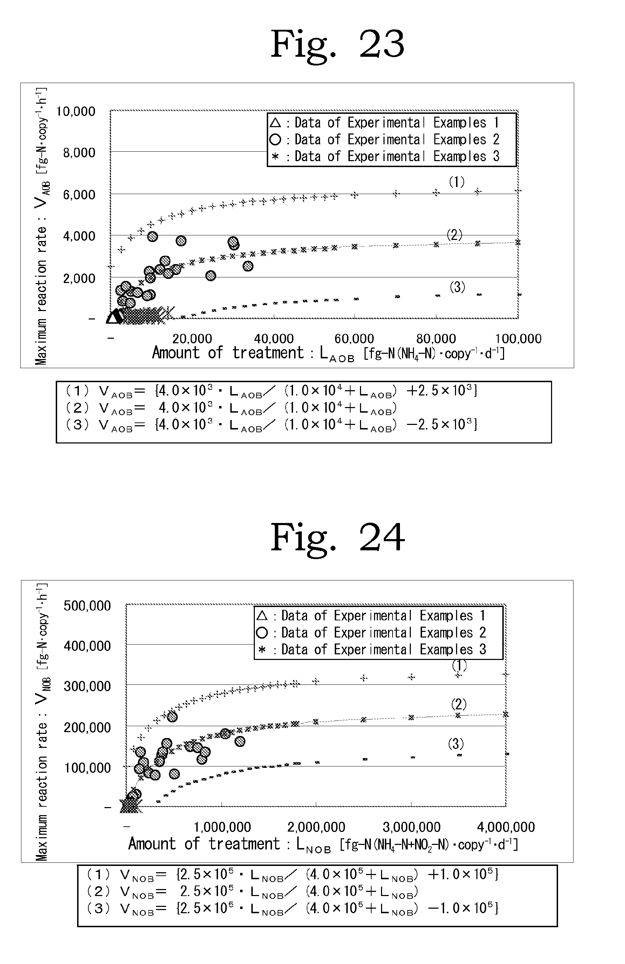

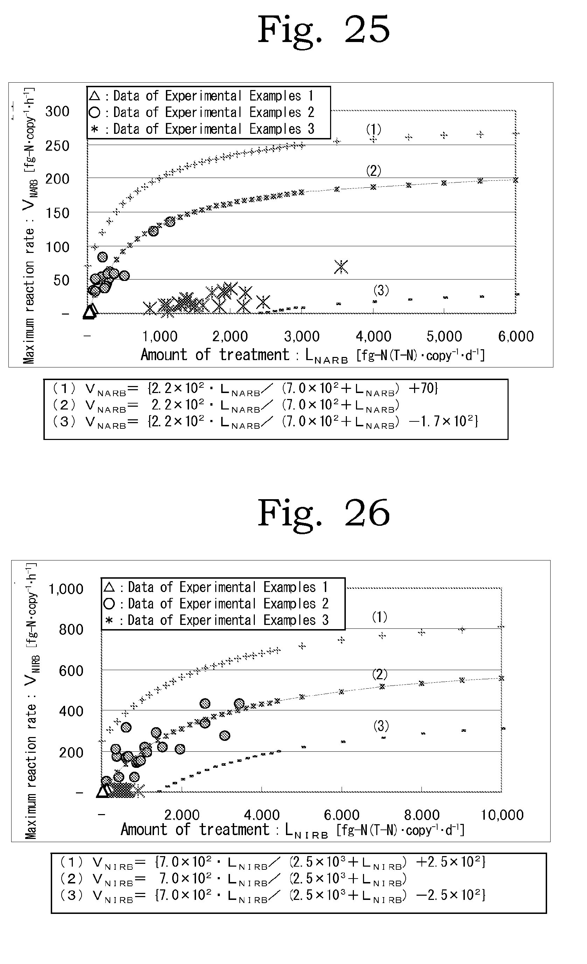

The "amounts of substance to be treated (nitrogen) that were treated per bacterial cell per day" were calculated and the graphed results are illustrated in Figs. 23 to 26 in the same manner as in Figs. 16 to 19, on the basis of the data collected in Experimental Examples 1 to 3.

In addition, if the "amount of a substance to be treated (nitrogen) that is loaded per bacterial cell per day" is used as a factor, in the same manner as in Experimental Examples 1 to 3, the computational speed can be increased when the treated water quality is predicted by calculation since the results of the biological treatment do not need to be reflected.

On the other hand, when the "amount of a substance to be treated (nitrogen) that was treated per bacterial cell per day" is used as a factor, as described in Experimental Examples 4, the quality of the treated water after biological treatment is measured. The "amount of a substance to be treated (nitrogen) that was treated per bacterial cell per day" is calculated based on the measurement, and the result needs to be feedbacked for reflecting the result in the maximum reaction rate, so that labor is required for the prediction of the result.

However, in the case where the difference between the load and the amount of treatment is likely to be generated (a substance to be treated is liable to remain in the treated water), the use of the "amount of a substance to be treated that was treated per bacterial cell per day" as a factor has the advantage of readily obtaining a simulation result with high precision.

Additionally, in the typical biological treatment process, the load per bacterial cell per day is not greatly different from the amount of treatment as illustrated in comparison of Figs. 8 and 16 to 19 with Figs. 23 to 26, the use of the "amount of a substance to be treated that was treated per bacterial cell per day" as a factor is advantageous from the viewpoint of improving the calculation speed.

(Experimental Example 5)

(Investigation of effect of chloride ion)

The maximum reaction rate was measured by the same measurement of the maximum reaction rate as in Experimental Example 1 except that a chloride ion concentration was added to the solutions A, B, C and D prepared in Experimental Example 1 such that each of the solutions had concentrations of 2,000 mg/L and 4,000 mg/L.

Then, provided that the values of the maximum rates of reaction without the influence of chloride ions were expressed by $V_{AOB0}$, $V_{NOB0}$, $V_{NARB0}$, and $V_{NIRB0}$ and were regarded as 100, the values of the maximum rates of reaction ($V_{AOB}$,

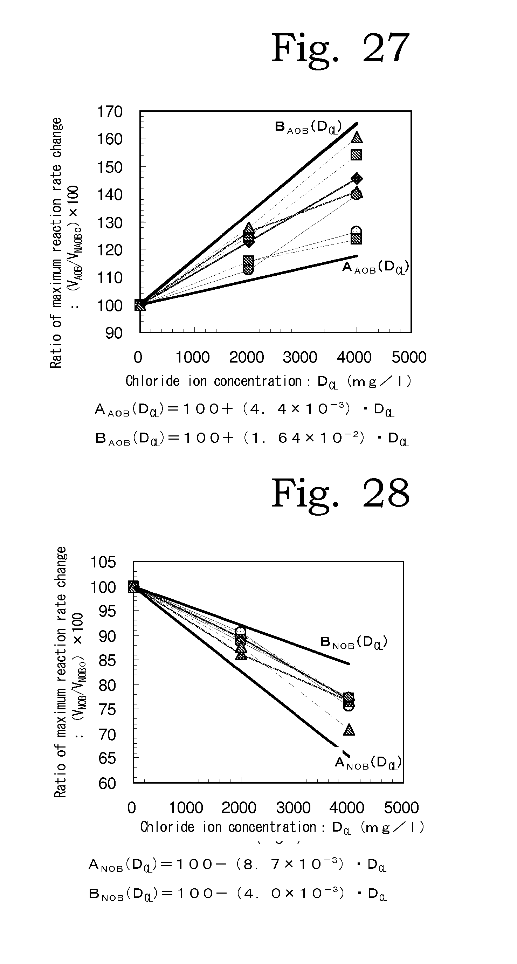

$V_{NOB}$, $V_{NARB0}$, and $V_{NIRB}$) in the case of addition of chloride ions of 2,000 and 4,000 mg/L were evaluated.
The results are shown in Figs. 27 to 30, respectively.

Fig. 27 also shows that in the ammonia oxidation reaction by ammonium oxidizing bacteria, the function: $k_{AOB}(D_{CL})$ that satisfies $(1+4.4\times10^{-5} \cdot D_{CL}) \leq k_{AOB}(D_{CL}) \leq (1+1.64\times10^{-4} \cdot D_{CL})$ (wherein "$D_{CL}$" represents a chloride ion concentration (mg/L) is set and the function of $V_{AOB}=f_{AOB}(L_{AOB}) \cdot k_{AOB}(D_{CL})$ is set between the maximum reaction rate: $V_{AOB}$ and the amount of ammonia nitrogen that is loaded per bacterial cell per unit time: $L_{AOB}$, thereby being capable of improving the prediction accuracy of the quality of the treated water as compared with the case in the conventional simulation.

In addition, Fig. 28 also shows that in the nitrite oxidation reaction by nitrite-oxidizing bacteria, the function: $k_{NOB}(D_{CL})$ that satisfies $(1-8.7\times10^{-5} \cdot D_{CL}) \leq k_{NOB}(D_{CL}) \leq (1-4.0\times10^{-5} \cdot D_{CL})$ (wherein "$D_{CL}$" represents a chloride ion concentration (mg/L) is set and the function of $V_{NOB}=f_{NOB}(L_{NOB}) \cdot k_{NOB}(D_{CL})$ is set between the maximum reaction rate: $L_{NOB}$ and the amount of nitrite nitrogen that is loaded per bacterial cell per unit time: $L_{NOB}$, thereby being capable of improving the prediction accuracy of the quality of the treated water as compared with the case in the conventional simulation.

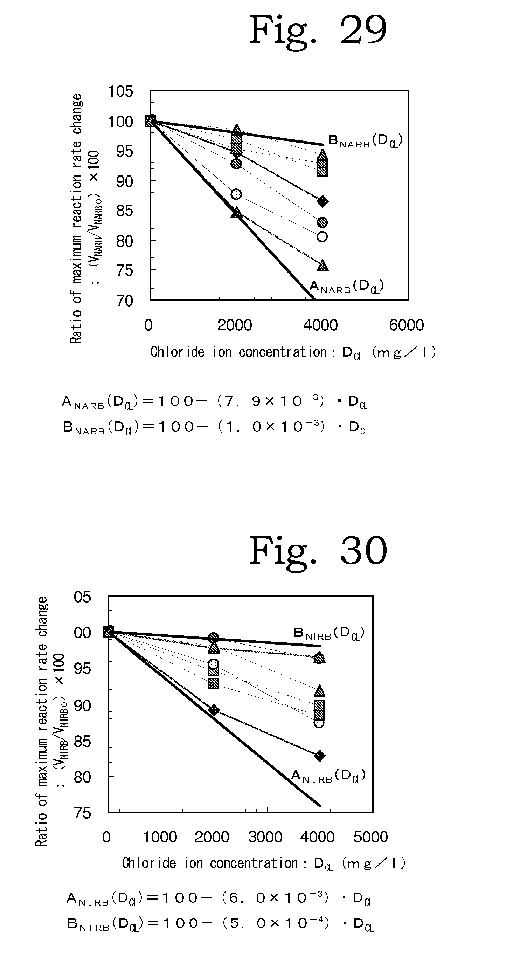

Fig. 29 also shows that in the nitrite oxidation reaction by nitrite-oxidizing bacteria, the function: $k_{NARB}(D_{CL})$ that satisfies $(1-7.9\times10^{-5} \cdot D_{CL}) \leq k_{NARB}(D_{CL}) \leq (1-1.0\times10^{-5} \cdot D_{CL})$ (wherein "$D_{CL}$" represents a chloride ion concentration (mg/L) is set and the function of $V_{NARB}=f_{NARB}(L_{NARB}) \cdot k_{NARB}(D_{CL})$ is set between the maximum reaction rate: $V_{NARB}$ and the amount of nitrite nitrogen that is loaded per bacterial cell per unit time: $L_{NARB}$, thereby being capable of improving the prediction accuracy of the quality of the treated water as compared with the case in the conventional simulation.

Fig. 30 further shows that in the nitrite reduction reaction by nitrite-reducing bacteria, the function: $k_{NIRB}(D_{CL})$ that satisfies $(1-6.0\times10^{-5} \cdot D_{CL}) \leq k_{NABR}(D_{CL}) \leq (1-5.0\times10^{-5} \cdot D_{CL})$ (wherein "$D_{CL}$" represents a chloride ion concentration (mg/L) is set and the function of $V_{NIRB}=f_{NIRB}(L_{NIRB}) \cdot k_{NIRB}(D_{CL})$ is set between the maximum reaction rate: $V_{NIRB}$ and the amount of nitrite nitrogen that is loaded per bacterial cell per unit time: $L_{NIRB}$, thereby being capable of improving the prediction accuracy of the quality of the treated water as compared with the case in the conventional simulation.

In addition, the results in Experimental Example 5 indicated in Figs. 27 to 30 can be reflected not only in the case of making use of, as a factor, the "amount of the substance to be treated that is loaded per bacterial cell per day" as in Experimental Example 1, but in the case of making use of, as a factor, the "amount of the substance to be treated that is treated per bacterial cell per day" as in Experimental Example 4.
In other words, even when the amount of ammonia nitrogen that is treated per ammonium oxidizing bacterial cell per unit time is expressed by "$L_{AOB}$," the amount of nitrite nitrogen that is treated per nitrite-oxidizing bacterial cell per unit time is expressed by "$L_{NOB}$," the amount of nitrate nitrogen that is treated per nitrate-reducing bacterial cell per unit time is expressed by "$L_{NARB}$," and the amount of nitrite nitrogen that is treated per nitrite-reducing bacterial cell per unit time is expressed by "$L_{NIRB}$," the function: $k_{AOB}(D_{CL})$ that satisfies $(1+4.4\times10^{-5} \cdot D_{CL}) \leq k_{AOB}(D_{CL}) \leq (1+1.64\times10^{-4} \cdot D_{CL}$, $k_{NOB}(D_{CL})$ that satisfies $(1-8.7\times10^{-5} \cdot D_{CL}) \leq k_{NOB}(D_{CL}) \leq (1-4.0\times10^{-5} \cdot D_{CL})$, the function: $k_{NARB}(D_{CL})$ that satisfies $(1-7.9\times10^{-5} \cdot D_{CL}) \leq k_{NARB}(D_{CL}) \leq (1-1.0\times10^{-5} \cdot D_{CL})$ and the function: $k_{NIRB}(D_{CL})$ that satisfies $(1-6.0\times10^{-5} \cdot D_{CL}) \leq k_{NIRB}(D_{CL}) \leq (1-5.0\times10^{-6} \cdot D_{CL})$ (wherein "$D_{CL}$" represents a chloride ion concentration (mg/L), and the functions $V_{AOB}=f_{AOB}(L_{AOB}) \cdot k_{AOB}(D_{CL})$, $V_{NOB}=f_{NOB}(L_{NOB}) \cdot k_{NOB}(D_{CL})$, $V_{NARB}=f_{NARB}(L_{NARB}) \cdot k_{NARB}(D_{CL})$, and $V_{NIRB}=f_{NIRB}(L_{NIRB}) \cdot k_{NIRB}(D_{CL})$ are set between the maximum rates of reaction, thereby being capable of improving the prediction accuracy of the quality of the treated water as compared with the case in the conventional simulation.

As described above, it is understood that according to the present invention, a simulation method of being capable of reducing the labor of calibration while suppressing a decrease in prediction accuracy and the like can be executed.

SEQUENCE LISTING

<110> Kobelco Eco-Solutions Co,.LTD.

<120> Simulation method, simulation equipment, biological treatment
      method and biological treatment apparatus

<130> P-1615

<160> 13

<210> 1
<211> 19
<212> DNA
<213> Artificial

<220>
<223> A sequence of primer CTO 189fA/B

<400> 1
ggagraaagc aggggatcg                                              19


<210> 2
<211> 19
<212> DNA
<213> Artificial

<220>
<223> A sequence for primer CTO 189fC

<400> 2
ggaggaaagt aggggatcg                                              19


<210> 3
<211> 22
<212> DNA
<213> Artificial

<220>
<223> A sequence for primer RT1r

<400> 3
cgtcctctca gaccarctac tg                                          22


<210> 4

<211>  28
<212>  DNA
<213>  Artificial

<220>
<223>  A sequence for probe TMP1

<400>  4
caactagcta atcagrcatc rgccgctc                                  28


<210>  5
<211>  20
<212>  DNA
<213>  Artificial

<220>
<223>  A sequence for primer NSR1113f

<400>  5
cctgctttca gttgctaccg                                          20


<210>  6
<211>  20
<212>  DNA
<213>  Artificial

<220>
<223>  A sequence for primer NSR1264r

<400>  6
gtttgcagcg ctttgtaccg                                          20


<210>  7
<211>  24
<212>  DNA
<213>  Artificial

<220>
<223>  A sequence for probe NSR1143Taq

<400>  7
agcactctga aaggactgcc cagg                                     24


<210>  8

```
<211>  17
<212>  DNA
<213>  Artificial sequence

<220>
<223>  A sequence for primer narH50F

<400>  8
aartgyatcg gytgcca                                           17


<210>  9
<211>  18
<212>  DNA
<213>  Artificial sequence

<220>
<223>  A sequence of primer narHr3B

<400>  9
tcccarkcct tgggrtag                                          18


<210>  10
<211>  16
<212>  DNA
<213>  Artificial

<220>
<223>  A sequence for primer nirK876

<400>  10
atyggcggva yggcga                                            16


<210>  11
<211>  20
<212>  DNA
<213>  Artificial

<220>
<223>  A sequence for primer nirK1040

<400>  11
gcctcgatca grttrtggtt                                        20


<210>  12
```

<211> 20
<212> DNA
<213> Artificial

<220>
<223> A sequence for primer cd3aF

<400> 12
gtsaacgtsa aggaracsgg                                                  20


<210> 13
<211> 19
<212> DNA
<213> Artificial

<220>
<223> A sequence for primer R3cd

<400> 13
gasttcggrt gsgtcttga                                                   19


## Claims

1. A simulation method of using the value of a maximum reaction rate of a substance to be treated with bacteria as a parameter in order to predict the quality of treated water after a biological treatment process of biologically treating water to be treated containing a substance to be treated with bacteria that decompose the substance to be treated, wherein the value of the maximum reaction rate is used as a parameter in a state where the value of the maximum reaction rate and the amount of the substance to be treated that is loaded per bacterial cell per unit time or the amount of the substance to be treated that has been treated per bacterial cell per unit time in the biological treatment process are in a functional relation, and the function is an increasing function of V with increasing L, wherein V represents the value of the maximum reaction rate and L represents the amount of the substance to be treated that is loaded per bacterial cell per unit time or the amount of the substance to be treated that has been treated per bacterial cell per unit time in the biological treatment process.

2. The simulation method according to claim 1, wherein the value of the maximum reaction rate is used as a parameter in a state where the functional relation of equation (1):

$$[\text{Eq. 1}]$$

$$V = f\ (L) \quad \cdots \quad (1)$$

is satisfied, wherein V ($fg \cdot copy^{-2} \cdot h^{-1}$) represents the value of the maximum reaction rate and L ($fg \cdot copy^{-1} \cdot day^{-1}$) represents the amount of the substance to be treated that is loaded per bacterial cell per unit time or the amount of the substance to be treated that has been treated per bacterial cell per unit time in the biological treatment process, and the function f(x) of a variable x (wherein x > 0) is a function between a function g(x) that increases y1 in equation (2):

[Eq. 2]

$$y_1 = g\ (x) \quad \cdots \quad (2)$$

with increasing x and a function h(x) that increases $y_2$ in equation (3):

[Eq. 3]

$$y_2 = h\ (x) \quad \cdots \quad (3)$$

with increasing x and satisfies $y_2 > y_1$.

3. The simulation method according to claim 2, wherein the substance to be treated is a nitrogen component, and the biological treatment process is either a nitrification process or denitrification process.

4. The simulation method according to claim 3, wherein the nitrogen component is ammonia nitrogen, $V_{AOB}$ and $L_{AOB}$ are related and used as a parameter in a function $f_{AOB}$ of equation (4):

[Eq. 4]

$$V_{AOB} = f_{AOB}\ (L_{AOB}) \quad \cdots \quad (4)$$

wherein $f_{AOB}$ is a function defined such that $\{4.0\times10^3\cdot L_{AOB}/(1.0\times10^4+L_{AOB})-2.5\times10^3\}\leq V_{AOB}\leq\{4.0\times10^3\cdot L_{AOB}/(1.0\times10^4+L_{AOB})+2.5\times10^3\}$ in the case where $1.0\times10^2\leq L_{AOB}\leq3.5\times10^4$, $V_{AOB}$ (fg·copy$^{-1}$·h$^{-1}$) represents the maximum reaction rate of the oxidation reaction by ammonia-oxidizing bacteria in the nitrification process, and $L_{AOB}$ (fg·copy-1·day$^{-1}$) represents the amount of the ammonia nitrogen that is loaded per ammonia-oxidizing bacterial cell per unit time, or the amount of the ammonia nitrogen that is treated per ammonia-oxidizing bacterial cell per unit time in the nitrification process.

5. The simulation method according to claim 3, wherein the nitrogen component is nitrite nitrogen, and $V_{NOB}$ and $L_{NOB}$ are related and used as a parameter in a function $f_{NOB}$ of equation (5):

[Eq. 5]

$$V_{NOB} = f_{NOB}\ (L_{NOB}) \quad \cdots \quad (5)$$

wherein $f_{NOB}$ is a function defined such that

$$\{2.5\times10^5\cdot L_{NOB}/(4.0\times10^5+L_{NOB})-1.0\times10^5\}\leq V_{NOB}\leq\{2.5\times10^5\cdot L_{NOB}/(4.0\times10^5+L_{NOB})+1.0\times10^5\}$$

in the case where $1.0\times10^3\leq L_{NOB}\leq1.2\times10^6$, $V_{NOB}$ (fg·copy$^{-1}$·h$^{-1}$) represents the maximum reaction rate of the oxidation reaction by nitrite-oxidizing bacteria in the nitrification process, and $L_{NOB}$(fg·copy$^{-1}$·day$^{-1}$) represents the amount of the nitrite nitrogen that is loaded per nitrite-oxidizing bacterial cell per unit time, or the amount of the nitrite nitrogen that is treated per nitrite-oxidizing bacterial cell per unit time in the nitrification process.

6. The simulation method according to claim 3, wherein the nitrogen component is nitrate nitrogen, and $V_{NARB}$ and $L_{NARB}$ are related and used as a parameter in a function $f_{NARB}$ of equation (6):

[Eq. 6]

$$V_{NARB} = f_{NARB} (L_{NARB}) \quad \cdots \quad (6)$$

wherein $f_{NARB}$ is a function defined such that $\{2.2\times10^2 \cdot L_{NARB}/(7.0\times10^2+L_{NARB})-1.7\times10^2\}\leq V_{NARB}\leq\{2.2\times10^2 \cdot L_{NARB}/(7.0\times10^2+L_{NARB})+70\}$ in the case where $5.0\leq L_{NARB}\leq2.5\times10^3$, $V_{NARB}$ (fg·copy$^{-1}$·h$^{-1}$) represents the maximum reaction rate of the reduction reaction by nitrate-reducing bacteria in the denitrification process, and $L_{NARB}$ (fg·copy-1·day$^{-1}$) represents the amount of the nitrate nitrogen that is loaded per nitrate-reducing bacterial cell per unit time, or the amount of the nitrate nitrogen that is treated per nitrate-reducing bacterial cell per unit time in the denitrification process.

7. The simulation method according to claim 3, wherein the nitrogen component is nitrite nitrogen, and $V_{NIRB}$ and $L_{NIRB}$ are related and used as a parameter in a function $f_{NIRB}$ of equation (7):

[Eq. 7]

$$V_{NIRB} = f_{NIRB} (L_{NIRB}) \quad \cdots \quad (7)$$

wherein $f_{NIRB}$ is a function defined such that $\{7.0\times10^2 \cdot L_{NIRB}/(2.5\times10^3+L_{NIRB})-2.5\times10^2\}\leq V_{NIRB}\leq\{7.0\times10^2 \cdot L_{NIRB}/(2.5\times10^3+L_{NIRB})+2.5\times10\,2\}$ in the case where $5.0\leq L_{NIRB}\leq3.5\times10^3$, $V_{NIRB}$ (fg·copy$^{-1}$·h$^{-1}$) represents the maximum reaction rate of the reduction reaction by nitrite-reducing bacteria in the denitrification process, and $L_{NIRB}$ (fg·copy$^{-1}$·day$^{-1}$) represents the amount of the nitrite nitrogen that is loaded per nitrite-reducing bacterial cell per unit time, or the amount of the nitrite nitrogen that is treated per nitrite-reducing bacterial cell per unit time in the denitrification process.

8. The simulation method according to any one of claims 4 to 7, wherein water to be treated includes a chloride ion together with the nitrogen component, the value of a maximum reaction rate obtained by calculating the quality of the treated water after the biological nitrification treatment or denitrification treatment of the water to be treated with bacteria on the basis of the concentration of the nitrogen component included in the water to be treated is used as a parameter and the value of the maximum reaction rate is calculated based on the calculation result of the function having as a variable a chlorine ion concentration in the water to be treated, whereby the value of the maximum reaction rate is used as a parameter for prediction in a state where the maximum reaction rate and the chlorine ion concentration are in a functional relation.

9. A simulation apparatus of using the value of a maximum reaction rate of a substance to be treated with bacteria as a parameter and executing a simulation in order to predict the quality of treated water after a biological treatment process of biologically treating water to be treated containing a substance to be treated with bacteria that decompose the substance to be treated,
wherein the value of the maximum reaction rate is used for a parameter in a state where the value of the maximum reaction rate and the amount of the substance to be treated that is loaded per bacterial cell per unit time or the amount of the substance to be treated that is treated per bacterial cell per unit time in the biological treatment process are in a functional relation, and the function is an increasing function of V with increasing L, wherein V represents the value of the maximum reaction rate and L represents the amount of the substance to be treated that is loaded per bacterial cell per unit time or the amount of the substance to be treated that is treated per bacterial cell per unit time in the biological treatment process.

10. The simulation apparatus according to claim 9, wherein the value of the maximum reaction rate is used as a parameter in a state where the functional relation of equation (1):

[Eq. 8]

$$V = f (L) \quad \cdots \quad (1)$$

is satisfied, wherein V (fg·copy$^{-1}$·h$^{-1}$) represents the value of the maximum reaction rate and L (fg·copy$^{-1}$·day$^{-1}$) represents the amount of the substance to be treated that is loaded per bacterial cell per unit time or the amount of the substance to be treated that has been treated per bacterial cell per unit time in the biological treatment process, and the function f(x) of a variable x (wherein x>0) is a function between a function g(x) that increases $y_1$ in equation (2):

[Eq. 9]

$$y_1 = g\ (x) \quad \cdots \ (2)$$

with increasing x and a function h(x) that increases $y_2$ in equation (3):

[Eq. 10]

$$y_2 = h\ (x) \quad \cdots \ (3)$$

with increasing x and satisfies $y_2 > y_1$.

**11.** A biological treatment method of performing a biological treatment process while predicting the quality of treated water after the biological treatment process of biologically treating water to be treated containing a substance to be treated with bacteria that decompose the substance to be treated, by means of a simulation that uses as a parameter the value of a maximum reaction rate of the substance to be treated with the bacteria,
wherein the value of the maximum reaction rate is used as a parameter in a state where the value of the maximum reaction rate and the amount of the substance to be treated that is loaded per bacterial cell per unit time or the amount of the substance to be treated that has been treated per bacterial cell per unit time in the biological treatment process are in a functional relation, and the function is an increasing function of V with increasing L, wherein V represents the value of the maximum reaction rate and L represents the amount of the substance to be treated that is loaded per bacterial cell per unit time or the amount of the substance to be treated that has been treated per bacterial cell per unit time in the biological treatment process.

**12.** The simulation method according to claim 11, wherein the value of the maximum reaction rate is used as a parameter in a state where the functional relation of equation (1):

[Eq. 11]

$$V = f\ (L) \quad \cdots \ (1)$$

is satisfied, wherein V (fg·copy$^{-1}$·h$^{-1}$) represents the value of the maximum reaction rate and L (fg·copy$^{-1}$·day$^{-1}$) represents the amount of the substance to be treated that is loaded per bacterial cell per unit time or the amount of the substance to be treated that has been treated per bacterial cell per unit time in the biological treatment process, and the function f(x) of a variable x (wherein x>0) is a function between function g(x) that increases the $y_1$ in equation (2):

[Eq. 12]

$$y_1 = g\ (x) \quad \cdots \ (2)$$

with increasing x and a function h(x) that increases $y_2$ in equation (3):

[Eq. 13]

$$y_2 = h\ (x)\quad \cdots\quad (3)$$

with increasing x and satisfies $y_2 > y_1$.

13. A biological treatment apparatus for performing a biological treatment process while predicting the quality of treated water after a biological treatment process of biologically treating water to be treated containing a substance to be treated with bacteria that decompose the substance to be treated, by means of a simulation that uses as a parameter the value of a maximum reaction rate of the substance to be treated with the bacteria,
wherein the value of the maximum reaction rate is used as the parameter in a state where the value of the maximum reaction rate and the amount of the substance to be treated that is loaded per bacterial cell per unit time or the amount of the substance to be treated that has been treated per one bacterial cell per unit time in the biological treatment process are in a functional relation, and the function is an increasing function of V with increasing L, wherein V represents the value of the maximum reaction rate and L represents the amount of the substance to be treated that is loaded per bacterial cell per unit time or the amount of the substance to be treated that has been treated per one bacterial cell per unit time in the biological treatment process.

14. The biological treatment apparatus according to claim 13, wherein the value of the maximum reaction rate is used as a parameter in a state where the functional relation of equation (1):

[Eq. 14]

$$V = f\ (L)\quad \cdots\quad (1)$$

is satisfied, wherein V (fg·copy$^{-1}$·h$^{-1}$) represents the value of the maximum reaction rate and L (fg·copy$^{-1}$·day$^{-1}$) represents the amount of the substance to be treated that is loaded per bacterial cell per unit time or the amount of the substance to be treated that has been treated per bacterial cell per unit time in the biological treatment process, the value of the maximum reaction rate is used as a parameter and the function f(x) of a variable x (wherein x>0) is a function between function g(x) that increases the $y_1$ in equation (2):

[Eq. 15]

$$y_1 = g\ (x)\quad \cdots\quad (2)$$

with increasing x and a function h(x) that increases $y_2$ in equation (3):

[Eq. 16]

$$y_2 = h\ (x)\quad \cdots\quad (3)$$

with increasing x and satisfies $y_2 > y_1$.

# Fig. 1

Discharge water → Nitrification tank (2) → Denitrification tank (4) → Reaeration tank (6) → Sedimentation tank (8) → Separated liquid

Return sluge

Excess sludge

# Fig. 2

Maximum reaction rate: $V_{AOB}$ (fg-N · copy$^{-1}$ · h$^{-1}$)

Load : $L_{AOB}$ (fg-N (NH$_4$-N) · copy$^{-1}$ · d$^{-1}$)

$g(x) = (7.0 \times 10^2) \, x / \{(5.5 \times 10^3) + x\} - 70$

$f(x) = (7.0 \times 10^2) \, x / \{(5.5 \times 10^3) + x\}$

$h(x) = (7.0 \times 10^2) \, x / \{(5.5 \times 10^3) + x\} + 80$

# Fig. 3

$g(x) = (2.5 \times 10^4) \; x \diagup \{ (9.5 \times 10^4) + x \} - 1.7 \times 10^3$

$f(x) = (2.5 \times 10^4) \; x \diagup \{ (9.5 \times 10^4) + x \}$

$h(x) = (2.5 \times 10^4) \; x \diagup \{ (9.5 \times 10^4) + x \} + 2.2 \times 10^3$

# Fig. 4

$g(x) = (1.0 \times 10^2) \; x \diagup \{ (8.5 \times 10^2) + x \} - 2.0$

$f(x) = (1.0 \times 10^2) \; x \diagup \{ (8.5 \times 10^2) + x \}$

$h(x) = (1.0 \times 10^2) \; x \diagup \{ (8.5 \times 10^2) + x \} + 1.8$

# Fig. 5

$$g(x) = (6.0 \times 10) \, x / \{ (3.5 \times 10^2) + x \} - 7.0$$
$$f(x) = (6.0 \times 10) \, x / \{ (3.5 \times 10^2) + x \}$$
$$h(x) = (6.0 \times 10) \, x / \{ (3.5 \times 10^2) + x \} + 3.0$$

# Fig. 6

# Fig. 7

# Fig. 8

# Fig. 9

# Fig. 10

# Fig. 11

# Fig. 12

$$V_{AOB} = 4.0 \times 10^3 \cdot L_{AOB} \diagup (1.0 \times 10^4 + L_{AOB})$$

# Fig. 13

$V_{NOB} = 2.5 \times 10^5 \cdot L_{NOB} / (4.0 \times 10^5 + L_{NOB})$

Maximum reaction rate : $V_{NOB}$ [fg-N·copy$^{-1}$·h$^{-1}$]

Load : $L_{NOB}$ [fg-N(NH$_4$-N+NO$_2$-N)·copy$^{-1}$·d$^{-1}$]

# Fig. 14

$V_{NARB} = 2.2 \times 10^2 \cdot L_{NARB} / (7.0 \times 10^2 + L_{NARB})$

Maximum reaction rate : $V_{NARB}$ [fg-N·copy$^{-1}$·h$^{-1}$]

Load : $L_{NARB}$ [fg-N(T-N)·copy$^{-1}$·d$^{-1}$]

# Fig. 15

$V_{NIRB}=7.0\times10^2\cdot L_{NIRB}/(2.5\times10^3+L_{NIRB})$

Maximum reaction rate : $V_{NIRB}$ [fg-N·copy⁻¹·h⁻¹]

Load : $L_{NIRB}$ [fg-N(T-N)·copy⁻¹·d⁻¹]

# Fig. 16

Maximum reaction rate : $V_{AOB}$ [fg-N·copy⁻¹·h⁻¹]

△ : Result of Experimental Examples 1
○ : Result of Experimental Examples 2
＊ : Result of Experimental Examples 3

Load : $L_{AOB}$ [fg-N(NH₄-N)·copy⁻¹·d⁻¹]

(1) $V_{AOB}=\{4.0\times10^3\cdot L_{AOB}/(1.0\times10^4+L_{AOB})+2.5\times10^3\}$
(2) $V_{AOB}=4.0\times10^3\cdot L_{AOB}/(1.0\times10^4+L_{AOB})$
(3) $V_{AOB}=\{4.0\times10^3\cdot L_{AOB}/(1.0\times10^4+L_{AOB})-2.5\times10^3\}$

# Fig. 17

Maximum reaction rate : $V_{NOB}$ [fg-N·copy$^{-1}$·h$^{-1}$]

△ : Result of Experimental Examples 1
○ : Result of Experimental Examples 2
* : Result of Experimental Examples 3

Load : $L_{NOB}$ [fg-N(NH$_4$-N+NO$_2$-N)·copy$^{-1}$·d$^{-1}$]

(1) $V_{NOB} = \{2.5 \times 10^5 \cdot L_{NOB} / (4.0 \times 10^5 + L_{NOB}) + 1.0 \times 10^5\}$
(2) $V_{NOB} = 2.5 \times 10^5 \cdot L_{NOB} / (4.0 \times 10^5 + L_{NOB})$
(3) $V_{NOB} = \{2.5 \times 10^5 \cdot L_{NOB} / (4.0 \times 10^5 + L_{NOB}) - 1.0 \times 10^5\}$

# Fig. 18

Maximum reaction rate : $V_{NARB}$ [fg-N·copy$^{-1}$·h$^{-1}$]

△ : Result of Experimental Examples 1
○ : Result of Experimental Examples 2
* : Result of Experimental Examples 3

Load : $L_{NARB}$ [fg-N(T-N)·copy$^{-1}$·d$^{-1}$]

(1) $V_{NARB} = \{2.2 \times 10^2 \cdot L_{NARB} / (7.0 \times 10^2 + L_{NARB}) + 70\}$
(2) $V_{NARB} = 2.2 \times 10^2 \cdot L_{NARB} / (7.0 \times 10^2 + L_{NARB})$
(3) $V_{NARB} = \{2.2 \times 10^2 \cdot L_{NARB} / (7.0 \times 10^2 + L_{NARB}) - 1.7 \times 10^2\}$

# Fig. 19

(1) $V_{NIRB} = \{7.0 \times 10^2 \cdot L_{NIRB} / (2.5 \times 10^3 + L_{NIRB}) + 2.5 \times 10^2\}$
(2) $V_{NIRB} = 7.0 \times 10^2 \cdot L_{NIRB} / (2.5 \times 10^3 + L_{NIRB})$
(3) $V_{NIRB} = \{7.0 \times 10^2 \cdot L_{NIRB} / (2.5 \times 10^3 + L_{NIRB}) - 2.5 \times 10^2\}$

# Fig. 20

| | | Conventional model | Novel model |
|---|---|---|---|
| Ammonia oxidation reaction | M (18,1) | $1 - 3.43/Y_{NH4}$ | $1 - 3.43 \cdot f_{AOB}/\mu_{NH4}$ |
| | M (18,5) | $-1/Y_{NH4} - i_{NBM}$ | $-f_{AOB}/\mu_{NH4} - i_{NBM}$ |
| | M (18,7) | $1/Y_{NH4}$ | $f_{AOB}/\mu_{NH4}$ |
| Nitrite oxidation reaction | M (19,1) | $1 - 1.14/Y_{NO2}$ | $1 - 1.14 \cdot f_{NOB}/\mu_{NO2}$ |
| | M (19,7) | $-1/Y_{NO2} - i_{NBM}$ | $-f_{NOB}/\mu_{NO2} - i_{NBM}$ |
| | M (19,8) | $1/Y_{NO2}$ | $f_{NOB}/\mu_{NO2}$ |
| Nitrate reduction reaction | M (7,7) | $(1 - YSTO\_anox)/1.71$ | $f_{NARB} \cdot X_{NARB}/(k_{STO} \cdot \eta_{NO3} \cdot X_H)$ |
| | M (7,8) | $-(1 - YSTO\_anox)/1.71$ | $-f_{NARB} \cdot X_{NARB}/(k_{STO} \cdot \eta_{NO3} \cdot X_H)$ |
| Nitrite reduction reaction | M (6,7) | $(1 - YSTO\_anox)/1.14$ | $f_{NIRB} \cdot X_{NIRB}/(k_{STO} \cdot \eta_{NO2} \cdot X_H)$ |
| | M (6,8) | $-(1 - YSTO\_anox)/1.14$ | $-f_{NIRB} \cdot X_{NIRB}/(k_{STO} \cdot \eta_{NO2} \cdot X_H)$ |

↑ "M(Process、Component)" is expressed.

# Fig. 21

Result immediately after load increase

# Fig. 22

Result 48 hours after load increase

# Fig. 23

$$(1) \quad V_{AOB}= \{4.0\times10^3 \cdot L_{AOB}/ (1.0\times10^4 + L_{AOB}) +2.5\times10^3\}$$
$$(2) \quad V_{AOB}= 4.0\times10^3 \cdot L_{AOB}/ (1.0\times10^4 + L_{AOB})$$
$$(3) \quad V_{AOB}= \{4.0\times10^3 \cdot L_{AOB}/ (1.0\times10^4 + L_{AOB}) -2.5\times10^3\}$$

# Fig. 24

$$(1) \quad V_{NOB}= \{2.5\times10^5 \cdot L_{NOB}/ (4.0\times10^5 + L_{NOB}) +1.0\times10^5\}$$
$$(2) \quad V_{NOB}= 2.5\times10^5 \cdot L_{NOB}/ (4.0\times10^5 + L_{NOB})$$
$$(3) \quad V_{NOB}= \{2.5\times10^5 \cdot L_{NOB}/ (4.0\times10^5 + L_{NOB}) -1.0\times10^5\}$$

# Fig. 25

Maximum reaction rate : $V_{NARB}$ [fg-N·copy$^{-1}$·h$^{-1}$]

△ : Data of Experimental Examples 1
○ : Data of Experimental Examples 2
* : Data of Experimental Examples 3

Amount of treatment : $L_{NARB}$ [fg-N(T-N)·copy$^{-1}$·d$^{-1}$]

(1) $V_{NARB}= \{2.2 \times 10^2 \cdot L_{NARB} / (7.0 \times 10^2 + L_{NARB}) + 70\}$
(2) $V_{NARB}= 2.2 \times 10^2 \cdot L_{NARB} / (7.0 \times 10^2 + L_{NARB})$
(3) $V_{NARB}= \{2.2 \times 10^2 \cdot L_{NARB} / (7.0 \times 10^2 + L_{NARB}) - 1.7 \times 10^2\}$

# Fig. 26

Maximum reaction rate : $V_{NIRB}$ [fg-N·copy$^{-1}$·h$^{-1}$]

△ : Data of Experimental Examples 1
○ : Data of Experimental Examples 2
* : Data of Experimental Examples 3

Amount of treatment : $L_{NIRB}$ [fg-N(T-N)·copy$^{-1}$·d$^{-1}$]

(1) $V_{NIRB}= \{7.0 \times 10^2 \cdot L_{NIRB} / (2.5 \times 10^3 + L_{NIRB}) + 2.5 \times 10^2\}$
(2) $V_{NIRB}= 7.0 \times 10^2 \cdot L_{NIRB} / (2.5 \times 10^3 + L_{NIRB})$
(3) $V_{NIRB}= \{7.0 \times 10^2 \cdot L_{NIRB} / (2.5 \times 10^3 + L_{NIRB}) - 2.5 \times 10^2\}$

# Fig. 27

$A_{AOB}(D_{Cl}) = 1 0 0 + (4. 4 \times 1 0^{-3}) \cdot D_{Cl}$

$B_{AOB}(D_{Cl}) = 1 0 0 + (1. 6 4 \times 1 0^{-2}) \cdot D_{Cl}$

# Fig. 28

$A_{NOB}(D_{Cl}) = 1 0 0 - (8. 7 \times 1 0^{-3}) \cdot D_{Cl}$

$B_{NOB}(D_{Cl}) = 1 0 0 - (4. 0 \times 1 0^{-3}) \cdot D_{Cl}$

# Fig. 29

$A_{NARB}(D_{CL}) = 1 0 0 - (7. 9 \times 1 0^{-3}) \cdot D_{CL}$

$B_{NARB}(D_{CL}) = 1 0 0 - (1. 0 \times 1 0^{-3}) \cdot D_{CL}$

# Fig. 30

$A_{NIRB}(D_{CL}) = 1 0 0 - (6. 0 \times 1 0^{-3}) \cdot D_{CL}$

$B_{NIRB}(D_{CL}) = 1 0 0 - (5. 0 \times 1 0^{-4}) \cdot D_{CL}$

## INTERNATIONAL SEARCH REPORT

| | International application No. |
|---|---|
| | PCT/JP2008/061264 |

**A. CLASSIFICATION OF SUBJECT MATTER**
*C02F3/34*(2006.01)i, *C02F3/12*(2006.01)i, *G01N33/18*(2006.01)i

According to International Patent Classification (IPC) or to both national classification and IPC

**B. FIELDS SEARCHED**

Minimum documentation searched (classification system followed by classification symbols)
C02F3/34, C02F3/12, G01N33/18

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
JSTPlus(JDreamII)

**C. DOCUMENTS CONSIDERED TO BE RELEVANT**

| Category* | Citation of document, with indication, where appropriate, of the relevant passages | Relevant to claim No. |
|---|---|---|
| A | JP 08-323393 A (Meidensha Corp.), 10 December, 1996 (10.12.96), Par. Nos. [0018] to [0032] (Family: none) | 1-14 |
| A | JP 2001-334253 A (Toshiba Corp.), 04 December, 2001 (04.12.01), Par. Nos. [0002] to [0007], [0049] to [0054] (Family: none) | 1-14 |
| A | Mariko NAKAMURA, Koji TSUJI, Naohiro TAKEDA, Akifumi FUJITA, Akira AKASHI, "Shoka Saikingun no Kassei o Koryo shita Hanno Sokudo Shiki no Kento", Dai 43 Kai Proceedings of Forum on Environmental Engineering Research, 17 November, 2006 (17.11.06), pages 38 to 40 | 1-14 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 September, 2008 (10.09.08) | 22 September, 2008 (22.09.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2008/061264

C (Continuation).   DOCUMENTS CONSIDERED TO BE RELEVANT

| Category* | Citation of document, with indication, where appropriate, of the relevant passages | Relevant to claim No. |
|---|---|---|
| P,X | Tetsuo YAMASHITA, Naohiro TAKEDA, Koji TSUJI, Akifumi FUJITA, Takashi MINO, Akira AKASHI, "Shoka Saikin no Saidai Kassei no Hendo o Koryo shita Kassei Odei Model no Kento", Kankyo System Keisoku Seigyo Gakkaishi, 15 October, 2007 (15.10.07), Vol.12, No.2/3, pages 76 to 77 | 1-14 |
| P,X | Akifumi FUJITA, Koji TSUJI, Emi KAWAI, Tetsuo YAMASHITA, Akira AKASHI, "Ryuiki Shumatsu Gesui Shori Shisetsu ni Okeru Shoka Saikingun no Saibosu to Saidai Shoka Sokudo no Hendo", Dai 44 Kai Proceedings of Sewage Research Conference, 29 June, 2007 (29.06.07), pages 1 to 3 | 1-14 |
| P,X | Akifumi FUJITA, "Bunshi Seibutsugakuteki Shuho o Riyo shita Shoka Hanno Model no Kaihatsu", Journal of Japan Society on Water Environment, 10 January, 2008 (10.01.08), Vol.31, No.1, pages 17 to 20 | 1-14 |

Form PCT/ISA/210 (continuation of second sheet) (April 2007)

## REFERENCES CITED IN THE DESCRIPTION

**Patent documents cited in the description**

- JP 8323393 A
- JP 2000107796 A
- JP 7106357 A
- JP 9047785 A

**Non-patent literature cited in the description**

- Sewage Test Method. JAPAN SEWAGE WORKS ASSOCIATION, 1997, vol. 1
- *Appl. Environ. Microbiol.,* 2001, vol. 67, 972-976
- *Environ. Sci. Technol.,* 2003, vol. 37, 343-351
- *System. Appl. Microbiol.,* 2000, vol. 23, 47-57
- *FEMS Microbiology Ecology,* 2004, vol. 49, 401-417
- *J. Microbiol. Methods.,* 2004, vol. 59, 327-335
- *Appl. Environ. Microbiol,* 2002, vol. 68, 245-253